# FASCICULE DE BREVET EUROPEEN

(11) **EP 0 970 687 B1**
(45) Date de publication et mention de la délivrance du brevet: **08.06.2005**
(21) Numéro de dépôt: 99401580.8
(22) Date de dépôt: 24.06.1999
(51) Int. Cl.: A61K 7/13

(54) **Composition de teinture pour fibres kératiniques avec un colorant direct cationique et un polymère épaississant**
Färbemittel für Keratinfasern, das ein Kationischer Farbstoffe und eine Verdickendes Polymer enthält
Composition for dyeing keratinous fibres containing a cationic direct dye and a thickening polymer

(30) Priorité: 09.07.1998 FR 9808833
(43) Date de publication de la demande: 12.01.2000
(73) Titulaire: L'OREAL, 75008 Paris (FR)
(72) Inventeur: Rondeau, Christine, 78500 Versailles (FR)
(74) Mandataire: Miszputen, Laurent

(56) Documents cités:
- EP-A- 0 801 942
- EP-A- 0 850 636
- EP-A- 0 850 637
- EP-A- 0 850 638
- WO-A-95/01772
- WO-A-95/15144
- WO-A-99/20234
- WO-A-99/20235
- FR-A- 2 140 205
- FR-A- 2 189 006
- FR-A- 2 282 860
- FR-A- 2 285 851
- GB-A- 2 142 348
- US-A- 5 474 578

## Description

L'invention concerne une composition de teinture pour fibres kératiniques, en particulier pour fibres kératiniques humaines telles que les cheveux, comprenant, dans un milieu approprié pour la teinture, au moins un colorant direct cationique de formule donnée, et au moins un polymère épaississant comportant au moins un motif sucre particulier.

L'invention a également pour objets les procédés et dispositifs de teinture mettant en oeuvre ladite composition.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.
Le deuxième est la coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
Pour varier les nuances obtenues avec lesdits colorants d'oxydation, ou les enrichir de reflets, Il arrive qu'on leur ajoute des colorants directs.

Parmi les colorants directs cationiques disponibles dans le domaine de la teinture des fibres kératiniques notamment humaines, on connaît déjà les composés dont la structure est développée dans le texte qui va suivre; néanmoins, ces colorants conduisent à des colorations qui présentent des caractéristiques encore insuffisantes sur le plan de la puissance, de l'homogénéité de la couleur répartie le long de la fibre, on dit alors que la coloration est trop sélective, et sur le plan de la tenacité, en terme de résistance aux diverses agressions que peuvent subir les cheveux (lumière, intempéries,shampooings).

Or, après d'importantes recherches menées sur la question, la demanderesse vient maintenant de découvrir qu'il est possible d'obtenir de nouvelles compositions pour la teinture des fibres kératiniques capables de conduire à des colorations plus puissantes et néanmoins peu sélectives et résistant bien aux diverses agressions que peuvent subir les cheveux, en associant au moins un polymère épaississant comportant au moins un motif sucre particulier, à au moins un colorant direct cationique connu de l'art antérieur et de formules respectivement définies ci-après.

Cette découverte est à la base de la présente invention.

La présente invention a donc pour premier objet une composition pour la teinture des fibres kératiniques et en particulier des fibres kératiniques humaines telles que les cheveux, renfermant dans un milieu approprié pour la teinture, **(i)**au moins un colorant direct cationique dont la structure répond aux formules (I) à (III) définies ci-après, caractérisée par le fait qu'elle contient en outre **(ii)**au moins un polymère épaississant comportant au moins un motif sucre choisi dans le groupe comprenant :
- les gommes de guar non ioniques;
- les gommes de biopolysaccharides d'origine microbienne telles que les gommes de Scléroglucane ou de Xanthane;
- les gommes issues d'extrudats végétaux telles que les gommes Arabique, Ghatti, Karaya, Tragacanthe, Carrageenane, Agar et Caroube;
- les pectines;
- les alginates;
- les amidons;
- les hydroxyalkylcelluloses et carboxyalkylcelluloses.

**(i)** Le colorant direct cationique utilisable selon la présente invention est un composé choisi parmi ceux de formules (I), (II), (III), (III') suivantes :
   **a) les composés de formule (I) suivante :** 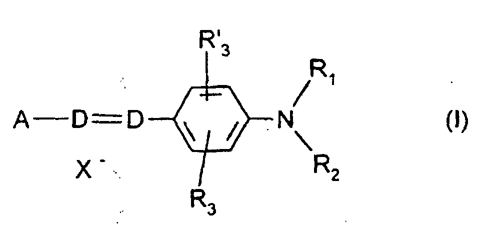 dans laquelle:
      D représente un atome d'azote ou le groupement -CH,
      R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
      R₃ et R'₃, identiques ou différents, représentent un atome d'hydrogène ou d'halogène choisi parmi le chlore, le brome, l'iode et le fluor, un radical cyano, alkyl en C₁-C₄ , alcoxy en C₁-C₄ ou acétyloxy,
      X ⁻ représente un anion de préférence choisi parmi le chlorure, le méthyl sulfate et l'acétate,
      A représente un groupement choisi par les structures A1 à A18 suivantes : 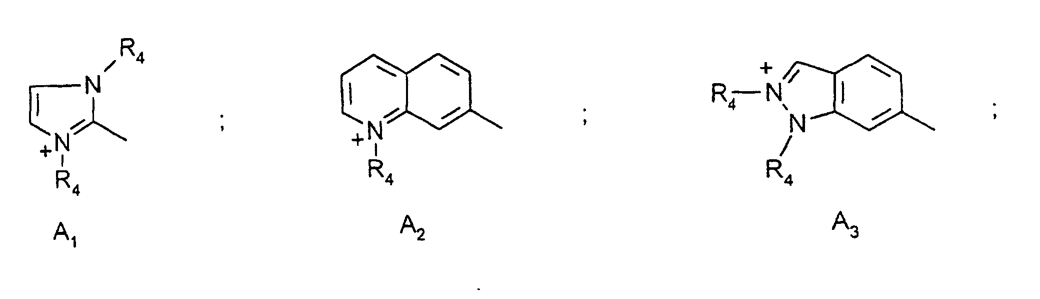 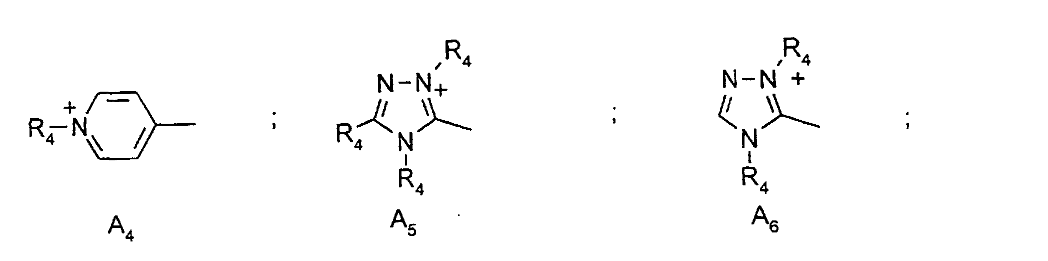 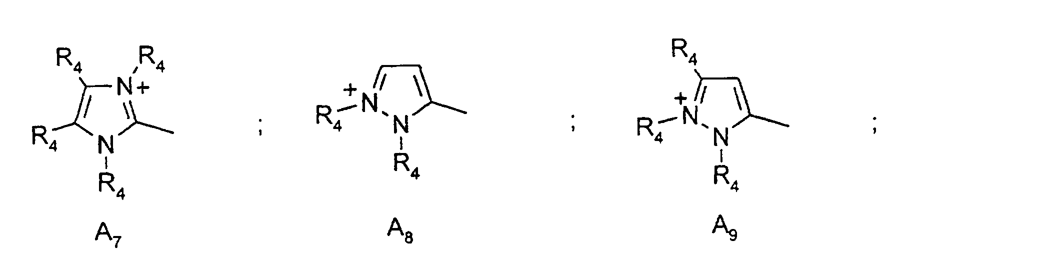 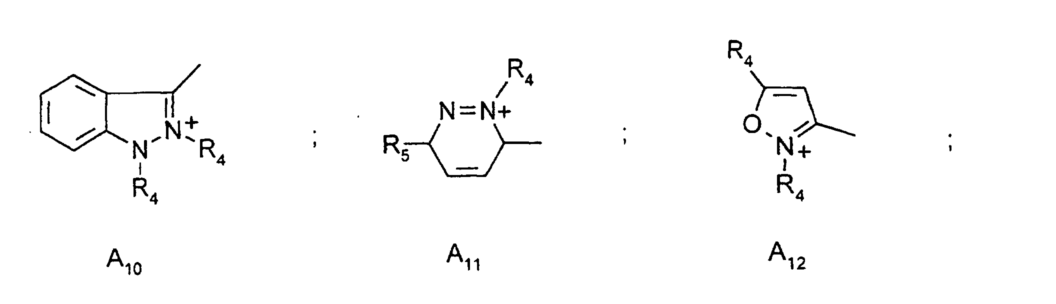 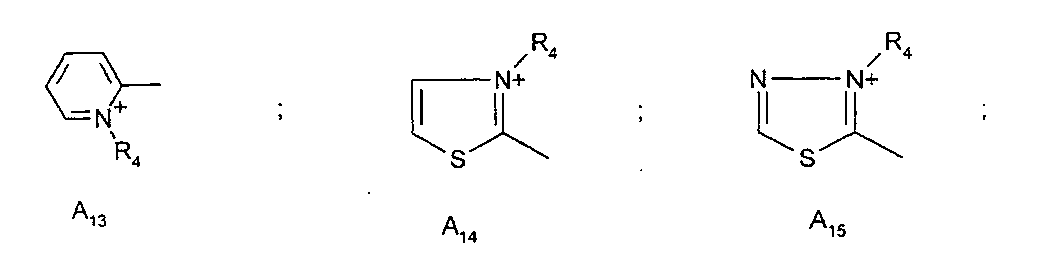 et 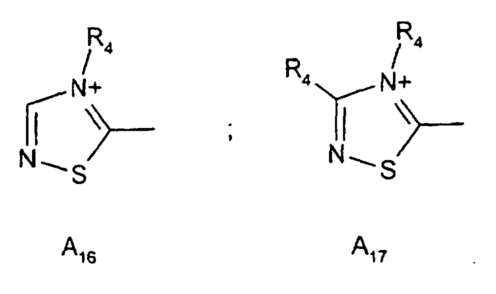
      et 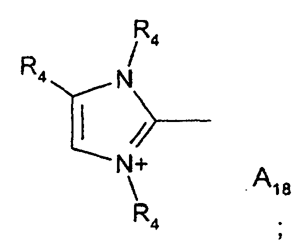 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
   **b) les composés de formule (II) suivante :** 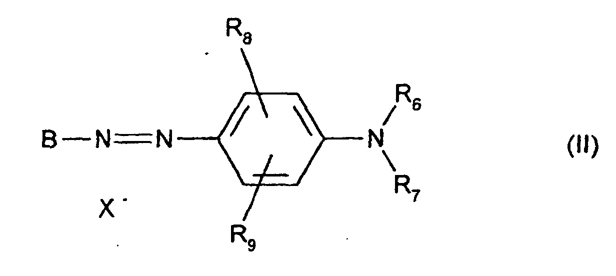 dans laquelle :
      R₆ représente un atome d'hydrogène ou un radical alkyle en C₁-C₄,
      R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
      R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
      X ⁻ représente un anion de préférence choisi parmi le chlorure, le méthyl sulfate et l'acétate,
      B représente un groupement choisi par les structures B1 à B6 suivantes : 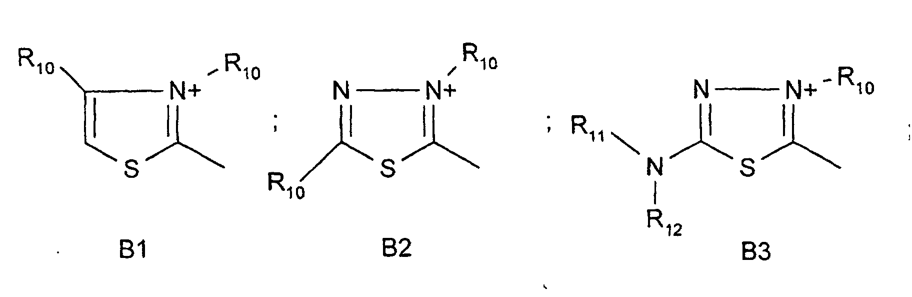 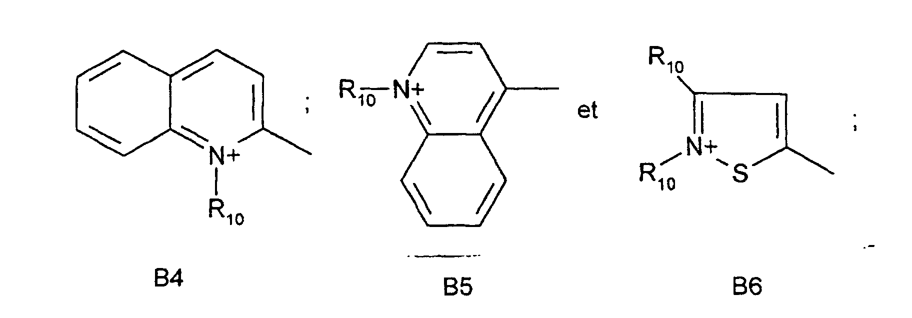
      dans lesquelles R₁₀ représente un radical alkyle en C₁-C₄, R₁₁ et R₁₂, identiques ou différents, représentent un atome d'hydrogène ou un radical alkyle en C₁-C₄ ;
   **c) les composés de formules (III) et (III') suivantes :** 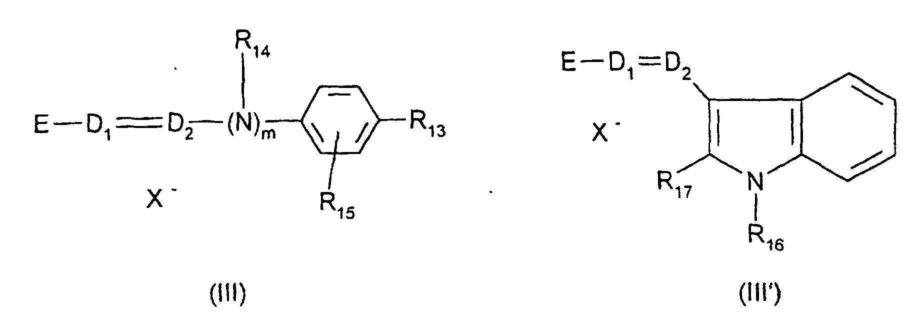 dans lesquelles :
      R₁₃ représente un atome d'hydrogène, un radical alcoxy en C₁-C₄, un atome d'halogène tel que le brome, le chlore, l'iode ou le fluor ou un radical amino,
      R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
      R₁₅ représente un atome d'hydrogène ou d'halogène tel que le brome, le chlore, l'iode ou le fluor,
      R₁₆ et R₁₇, identiques ou différents, représentent un atome d'hydrogène ou un radical alkyle en C₁-C₄,
      D₁ et D₂, identiques ou différents, représentent un atome d'azote ou le groupement -CH,
      m = 0 ou 1,
      étant entendu que lorsque R₁₃ représente un groupement amino non substitué, alors D₁ et D₂ représentent simultanément un groupement -CH et m = 0,
      X ⁻ représente un anion de préférence choisi parmi le chlorure, le méthyl sulfate et l'acétate,
      E représente un groupement choisi par les structures E1 à E8 suivantes : 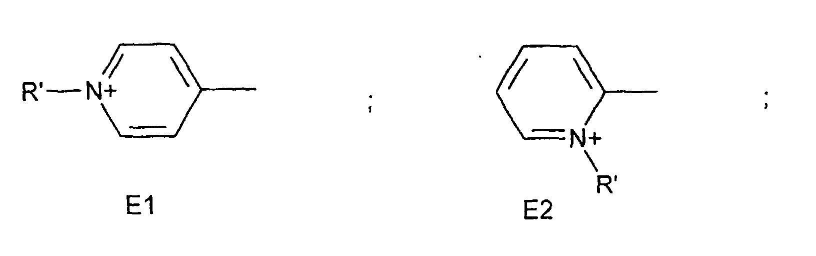 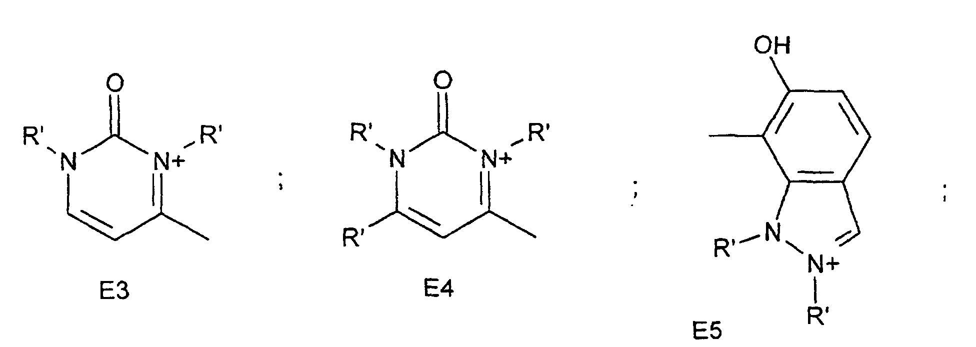 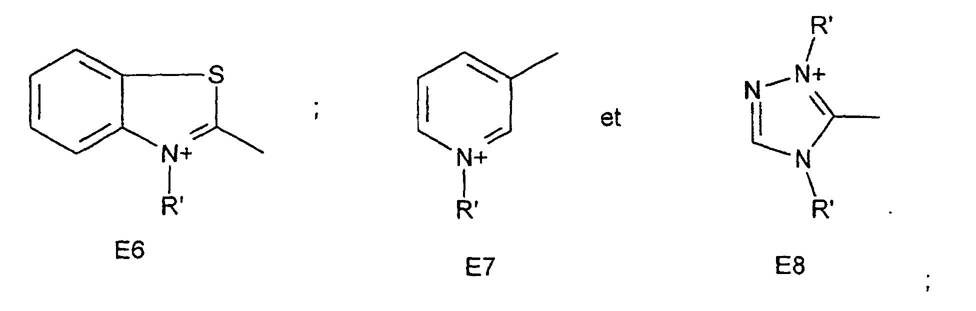 dans lesquelles R' représente un radical alkyle en C₁-C₄ ;
   lorsque m = 0 et que D₁ représente un atome d'azote, alors E peut également désigner un groupement de structure E9 suivante : 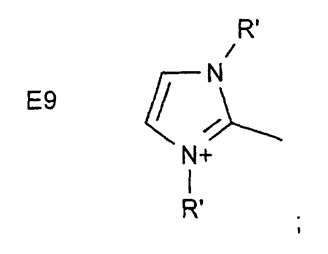 dans laquelle R' représente un radical alkyle en C₁-C₄.
   Dans les structures (I) à (III) et (III') définies ci-dessus le groupement alkyle ou alcoxy en C₁-C₄ désigne de préférence méthyle, éthyle, butyle, méthoxy, éthoxy.
   Les colorants directs cationiques de formules (I), (II), (III) et (III') utilisables dans les compositions tinctoriales conformes à l'invention, sont des composés connus et sont décrits par exemple dans les demandes de brevets WO 95/01772, WO 95/15144 et EP-A-0 714 954. Ceux de formule (IV) utilisables dans les compositions tinctoriales conformes à l'invention, sont des composés connus et sont décrits par exemple dans les demandes de brevets FR-2189006, FR-2285851 et FR-2140205 et ses certificats d'addition.
   Des compositions de colorants directs cationiques avec des polymères épaissisants comportant au moins un motif sucre, notamment de la cellulose quaternisée ou de l'hydroxyéthyl propylcellulose, sont également connues et décrites dans la demande de brevet FR 2282860.
   Parmi les colorants directs cationiques de formule (I) utilisables dans les compositions tinctoriales conformes à l'invention, on peut plus particulièrement citer les composés répondant aux structures (I1) à (I54) suivantes : 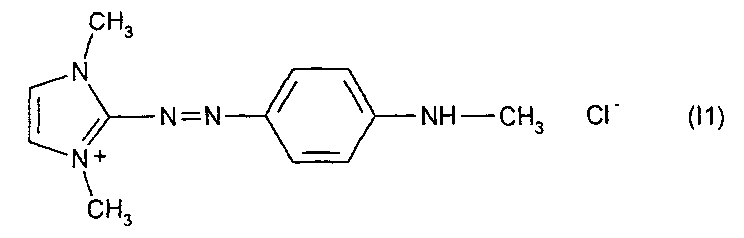 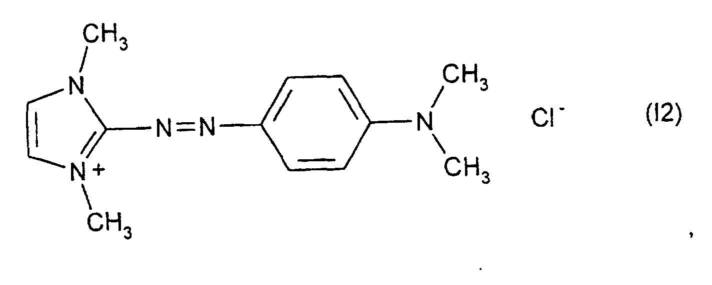 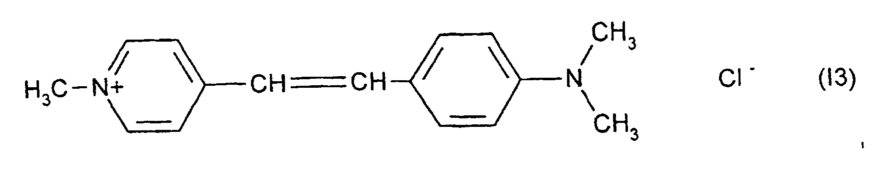 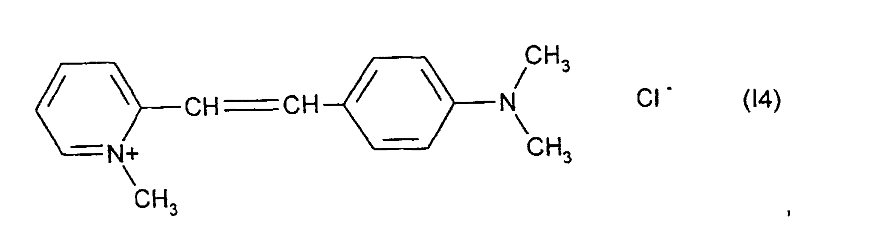 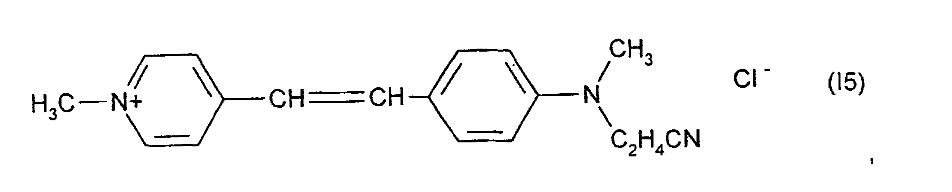 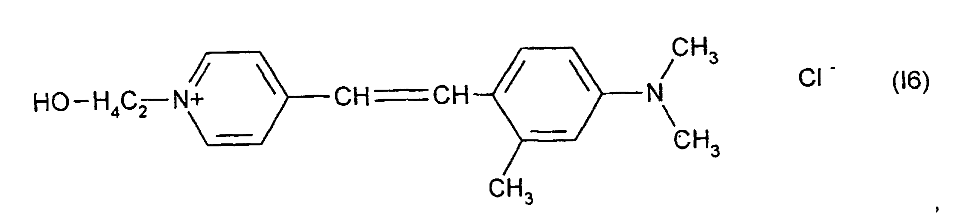 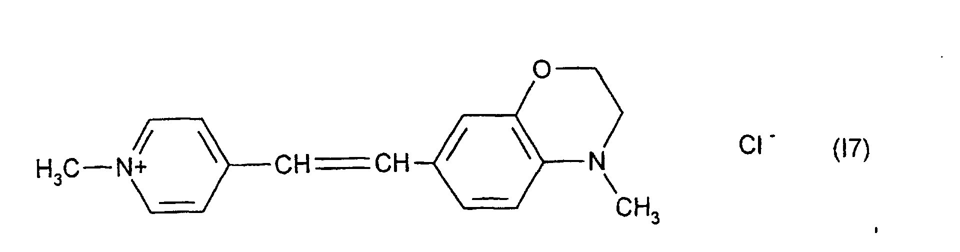 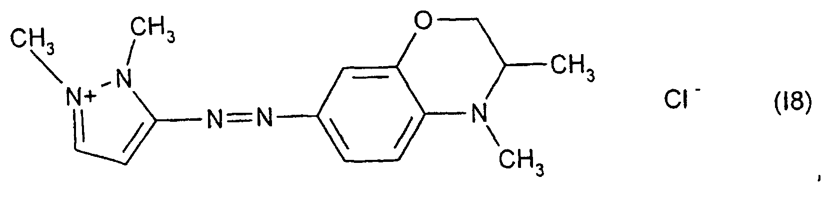 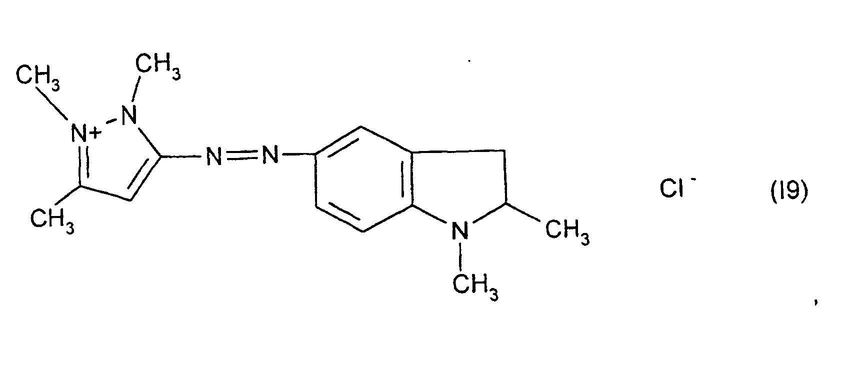 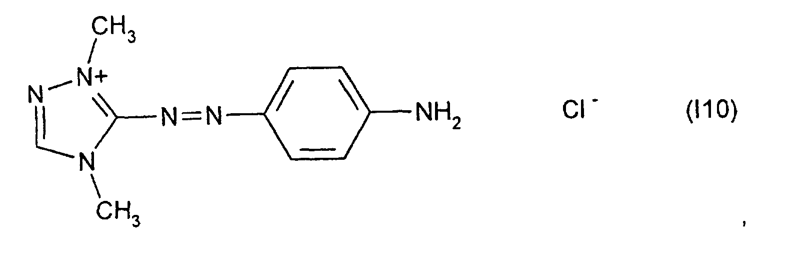 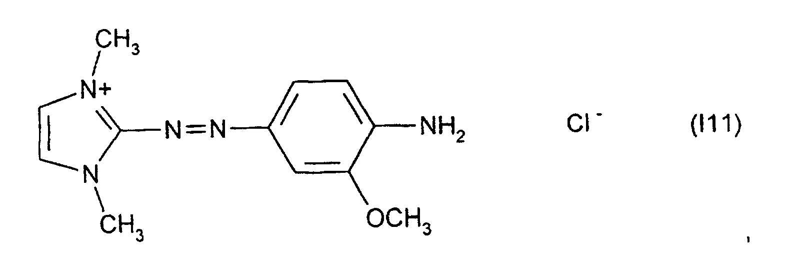 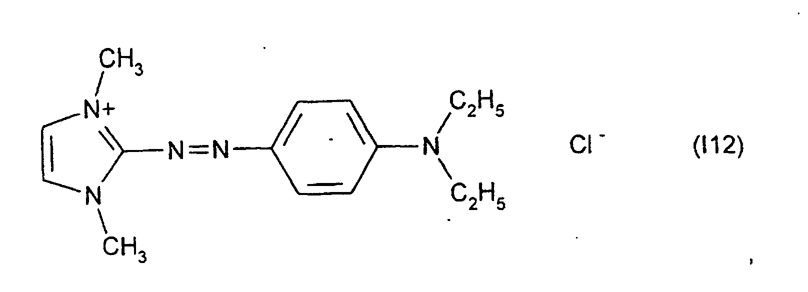 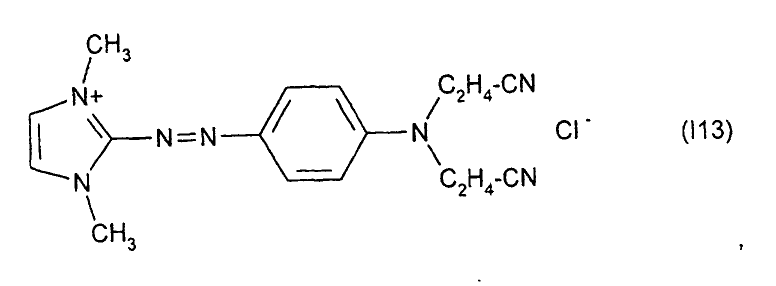 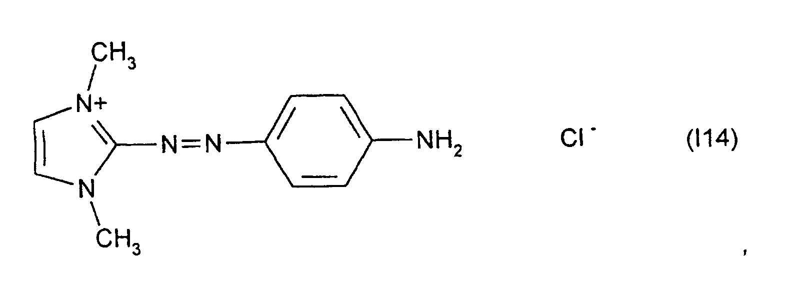 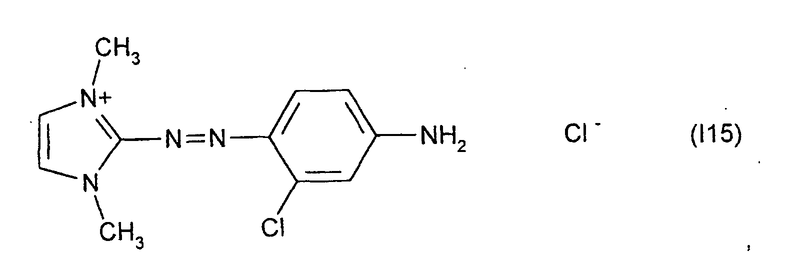 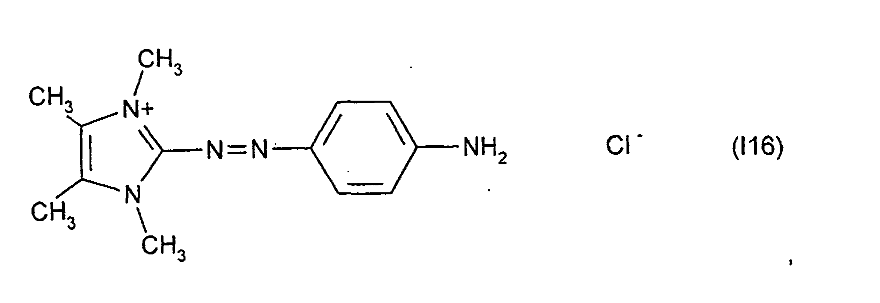 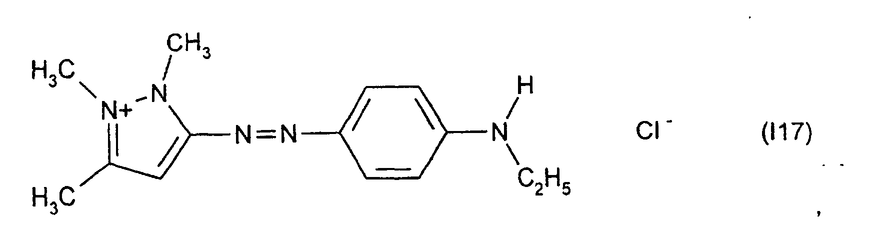 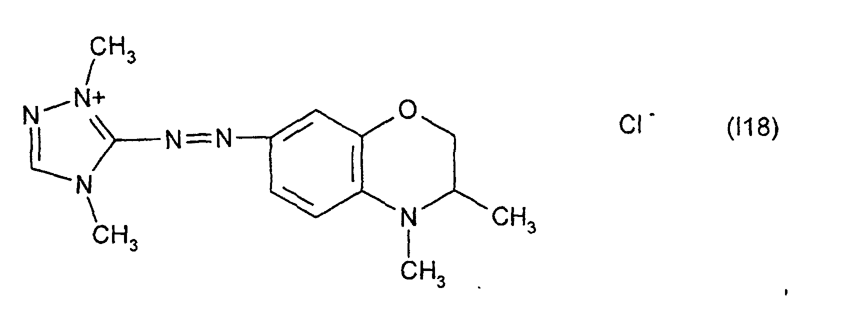 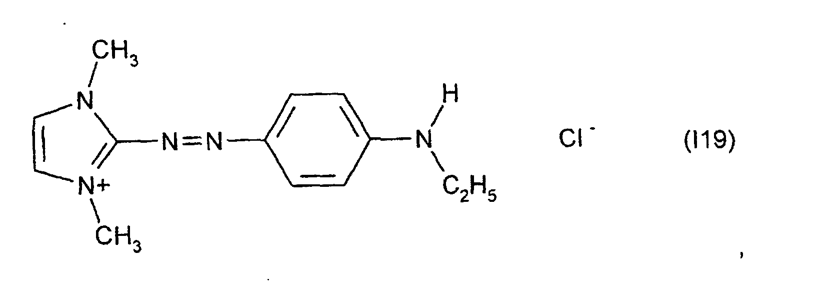 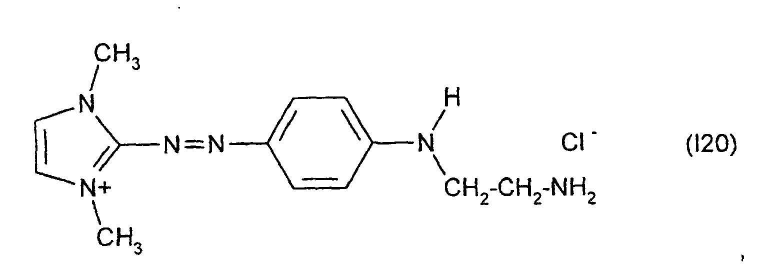 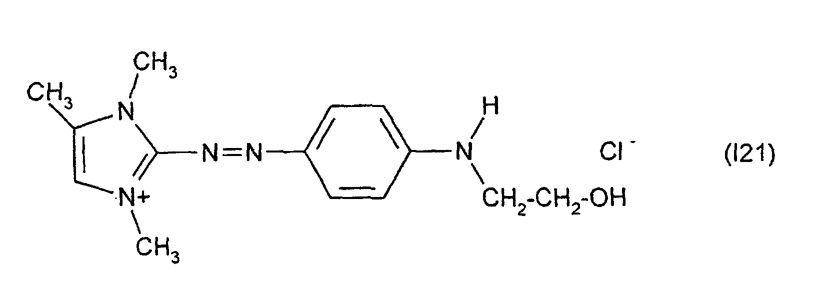 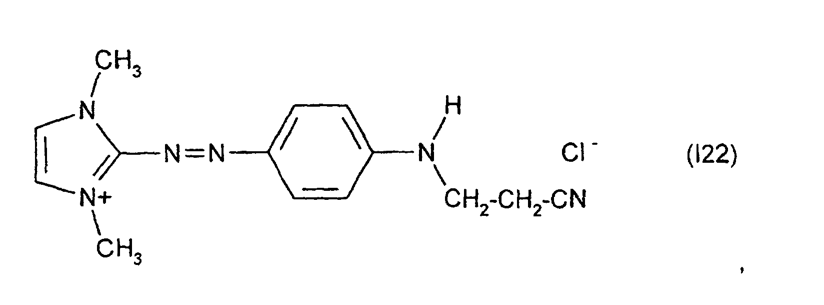 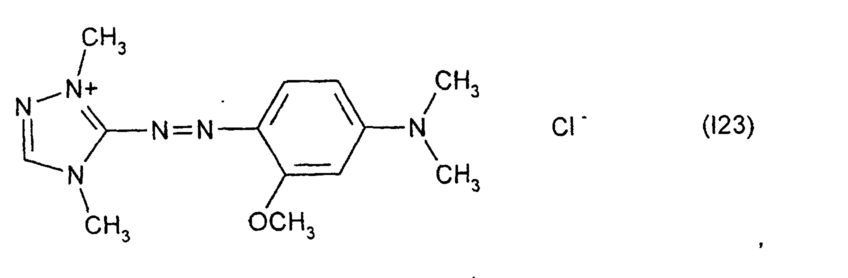 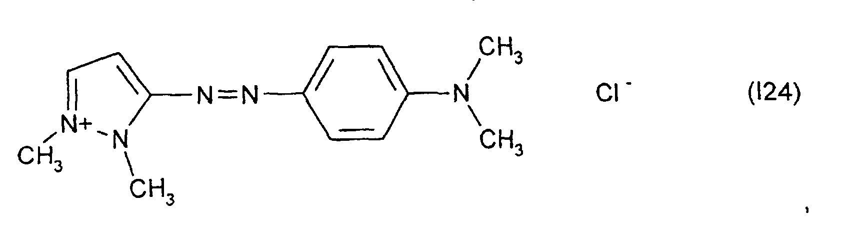 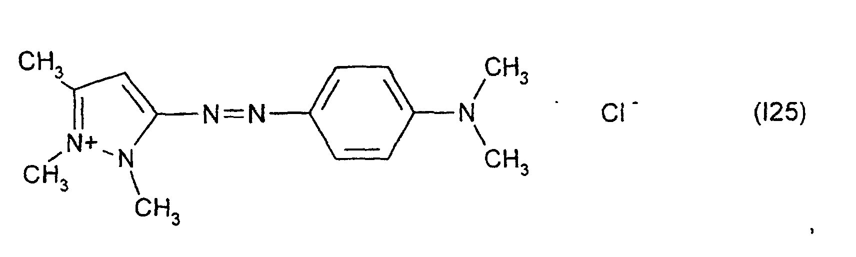 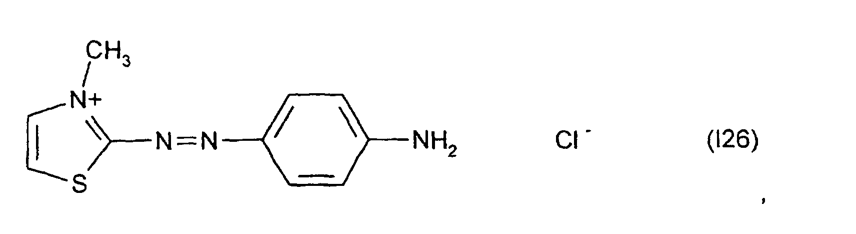 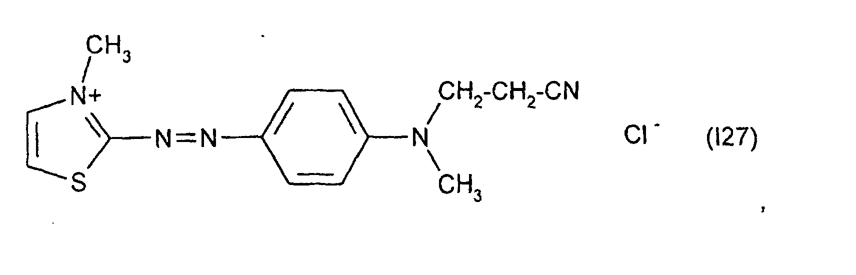 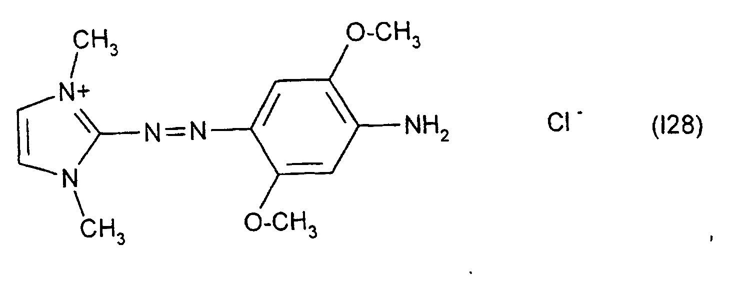 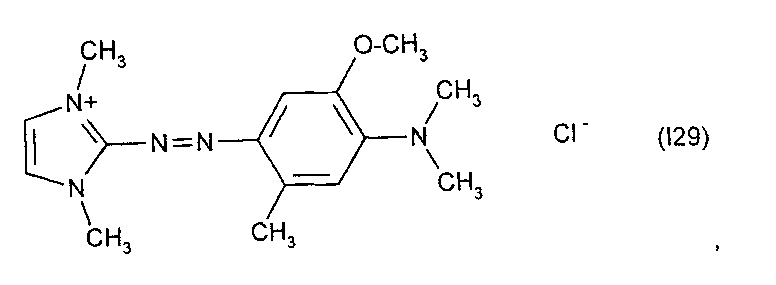 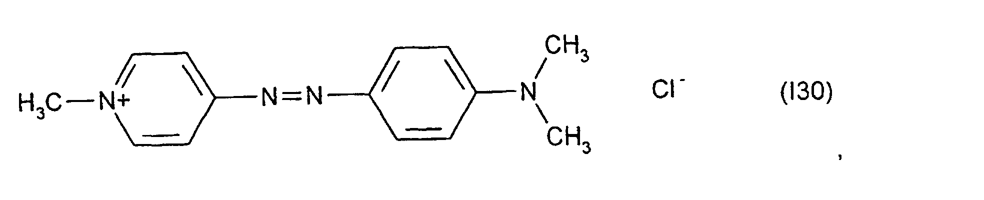 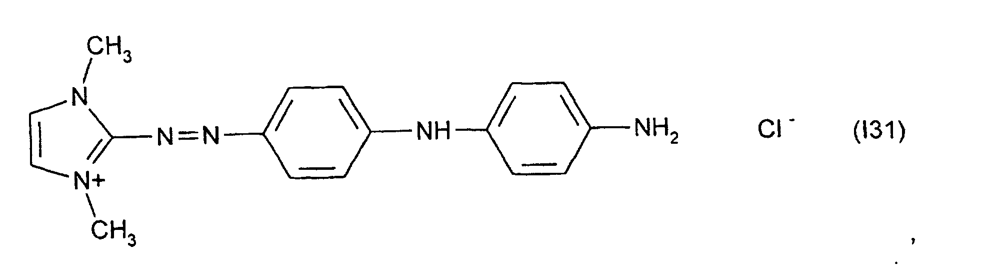 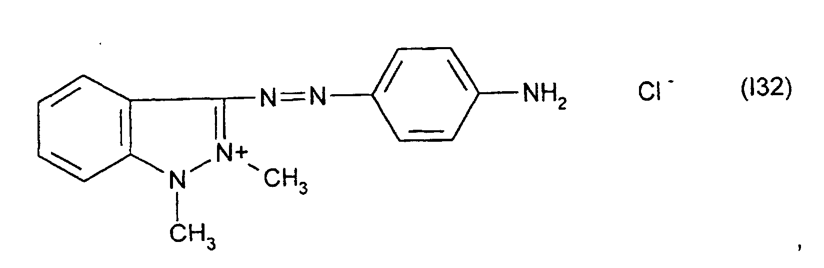 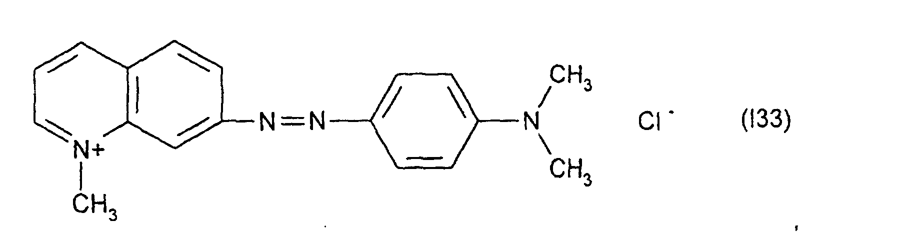 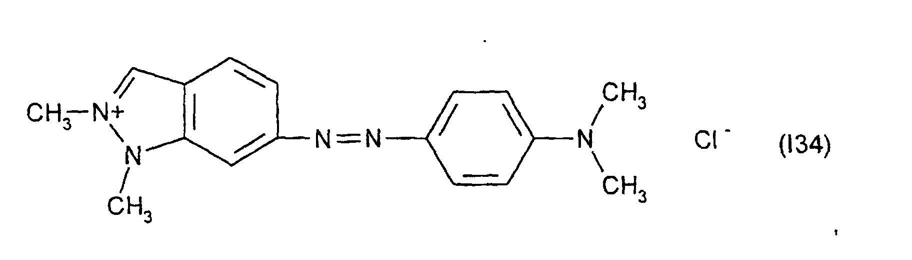 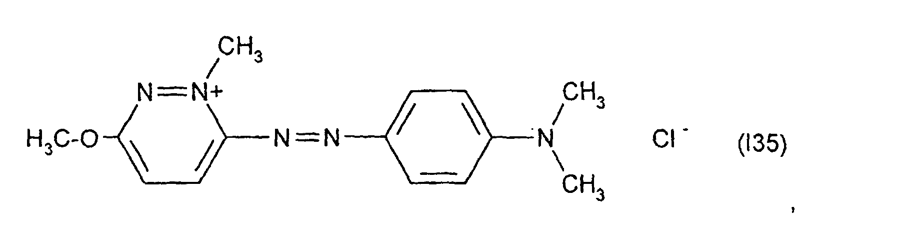 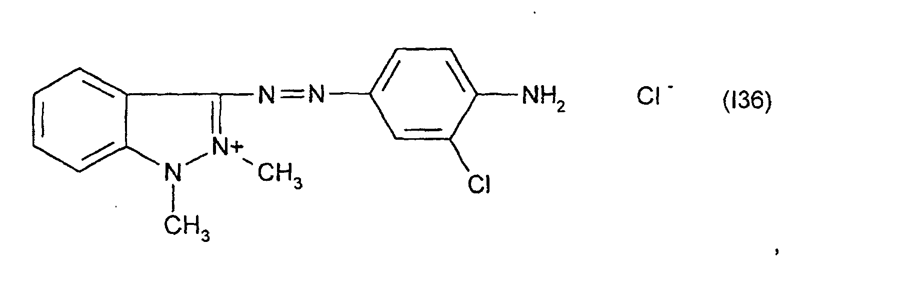 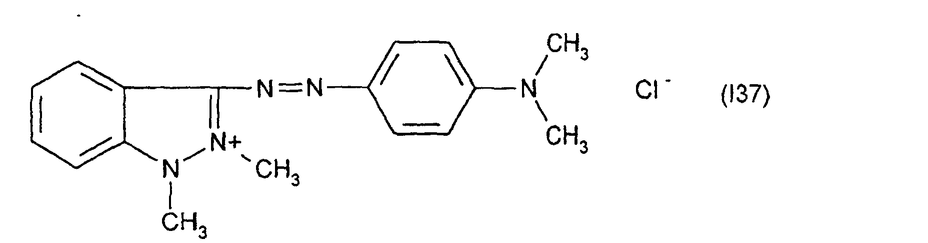 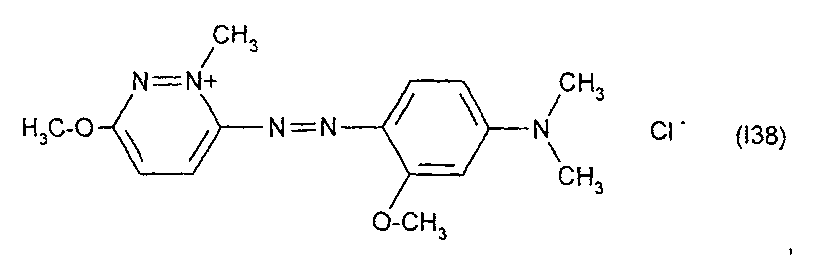 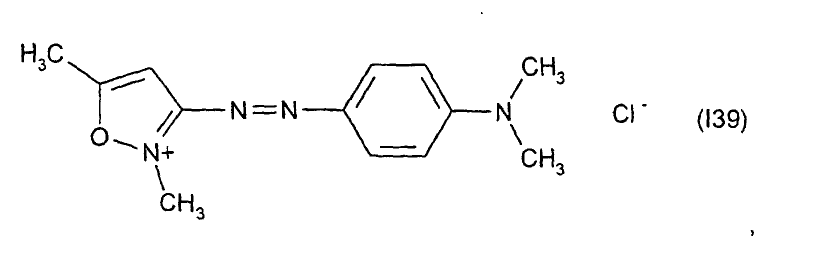 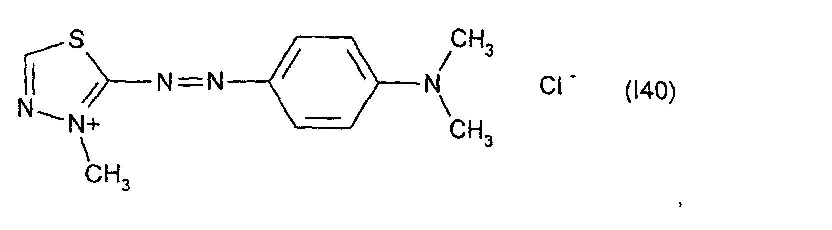 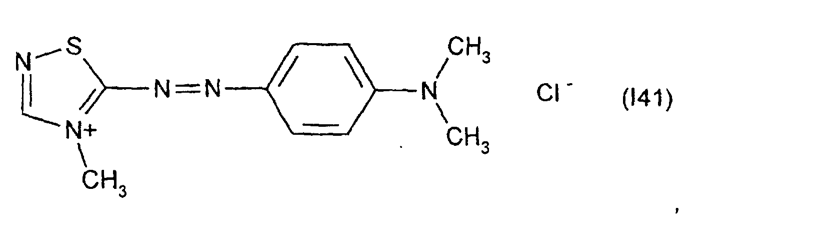 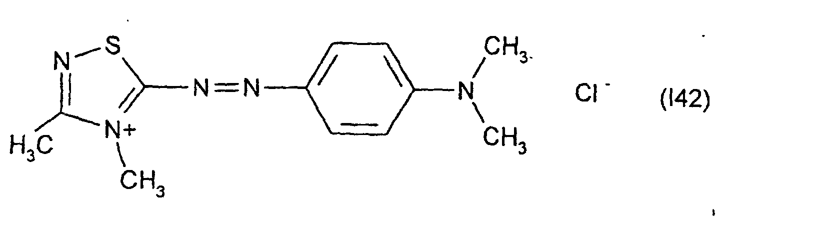 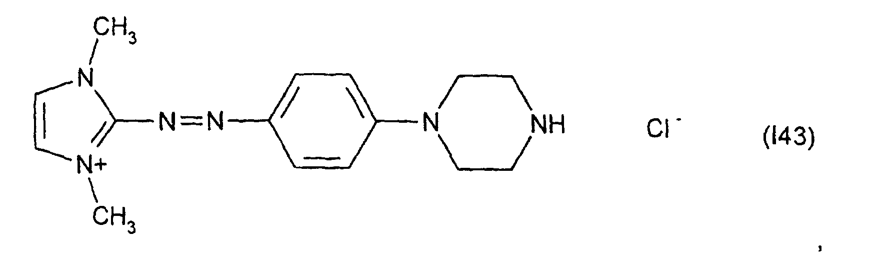 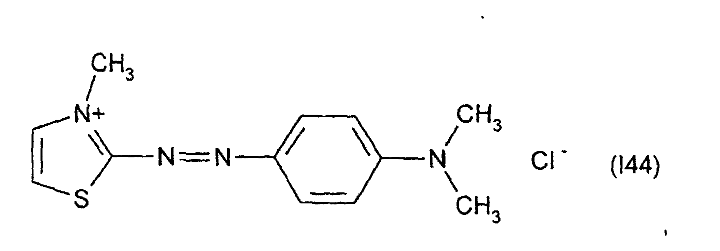 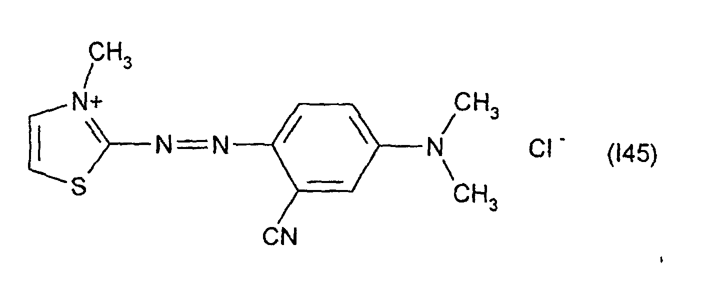 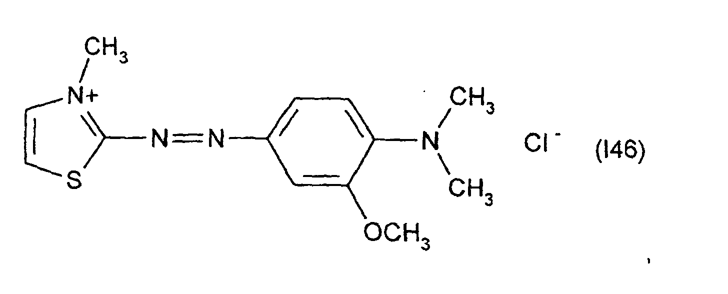 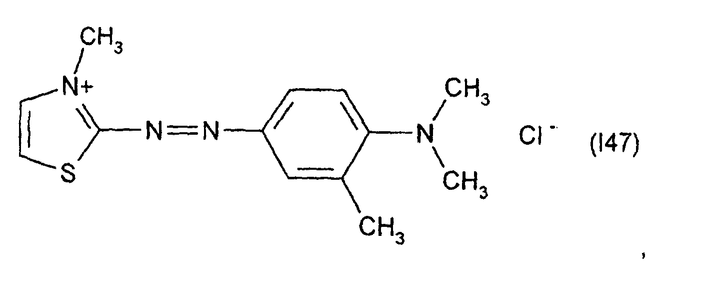 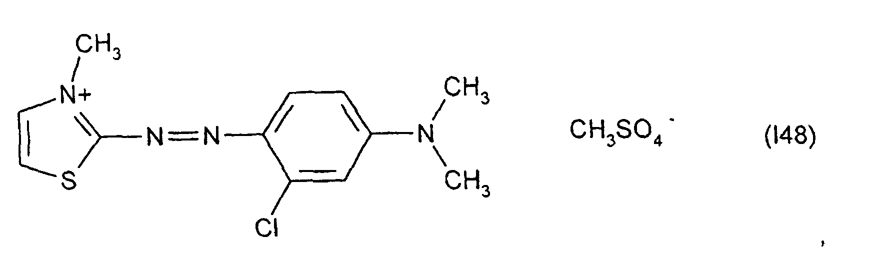 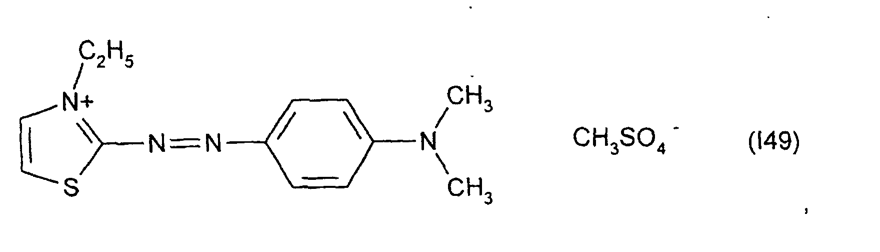 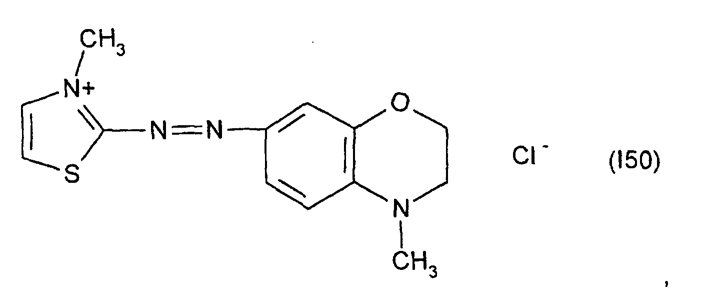 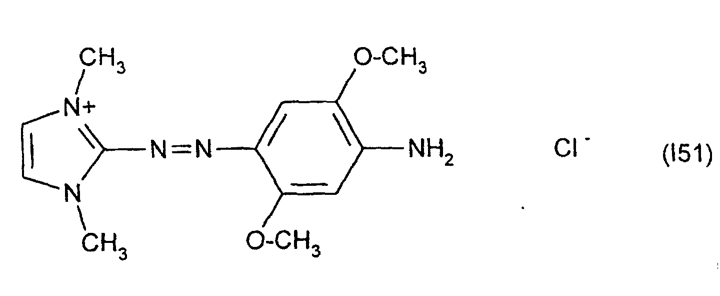 et 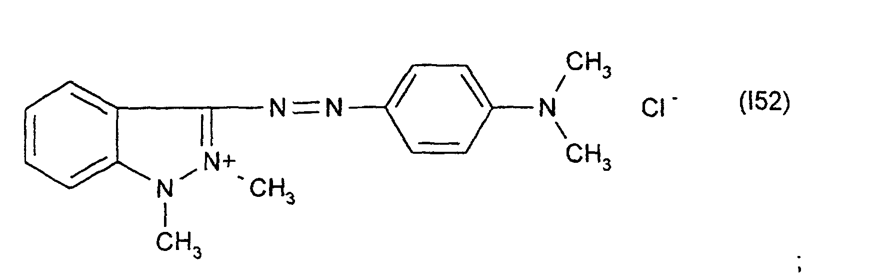 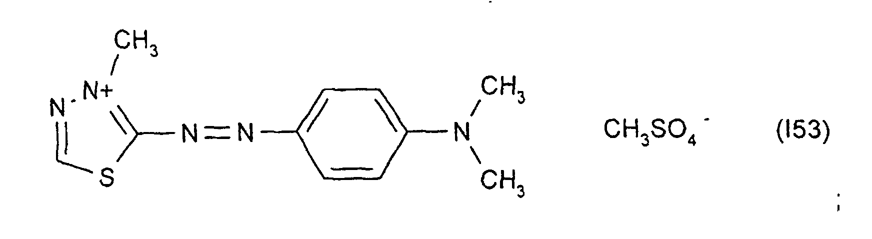 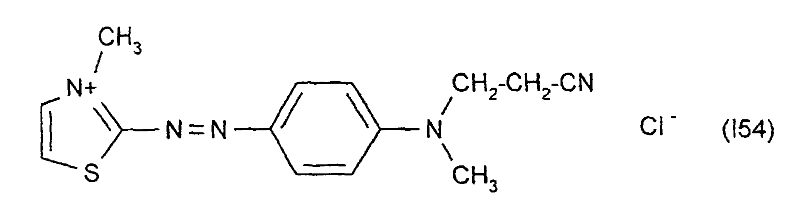
   Parmi les composés de structures (I1) à (I54) décrits ci-dessus, on préfère tout particulièrement les composés répondant aux structures (I1), (I2), (I14) et (I31).
   Parmi les colorants directs cationiques de formule (II) utilisables dans les compositions tinctoriales conformes à l'invention, on peut plus particulièrement citer les composés répondant aux structures (II1) à (II9) suivantes : 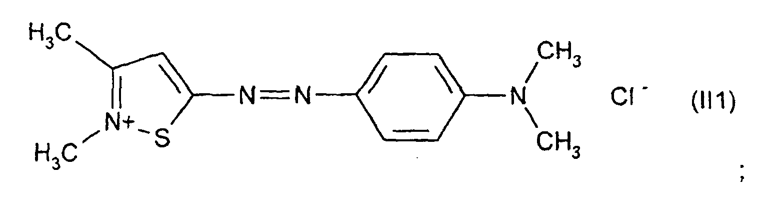 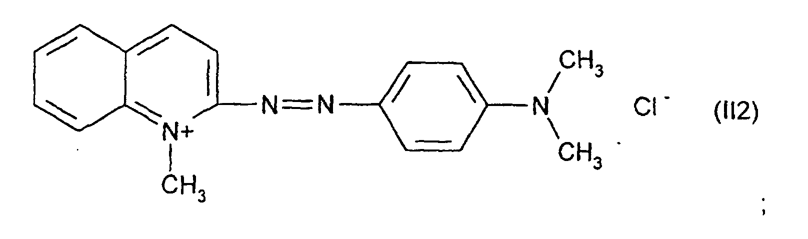 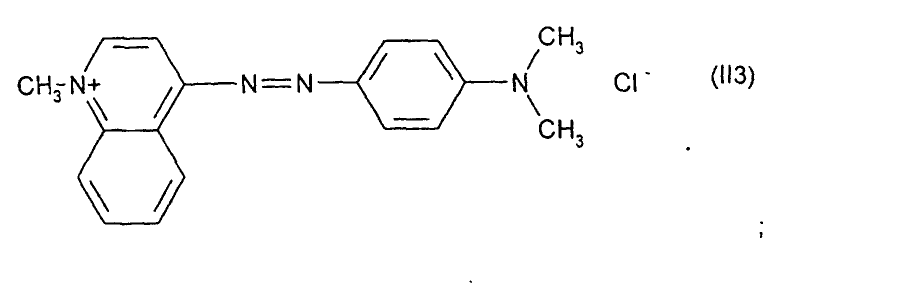 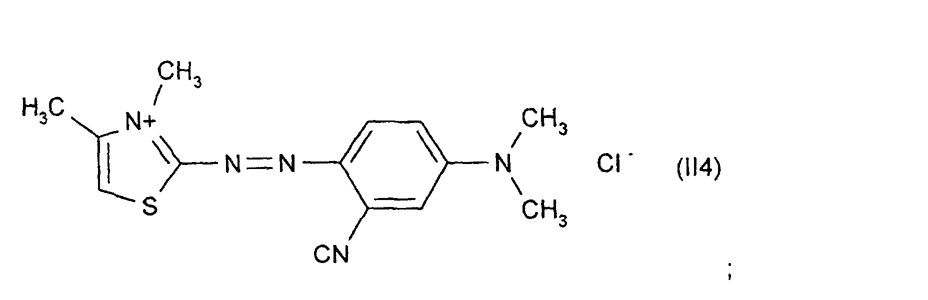 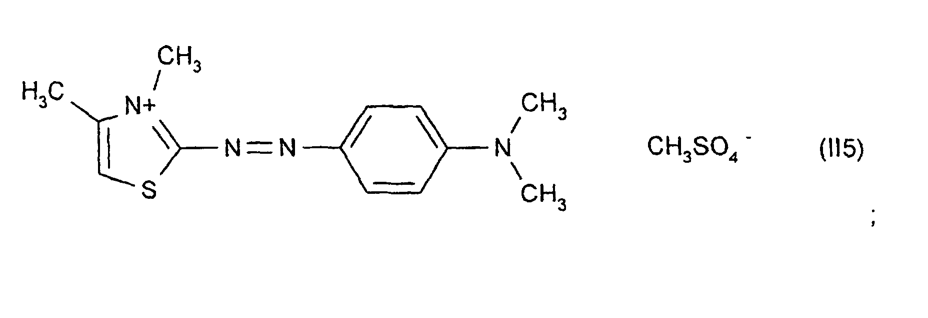 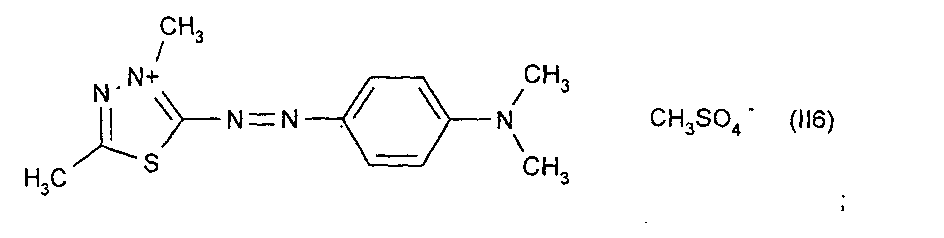 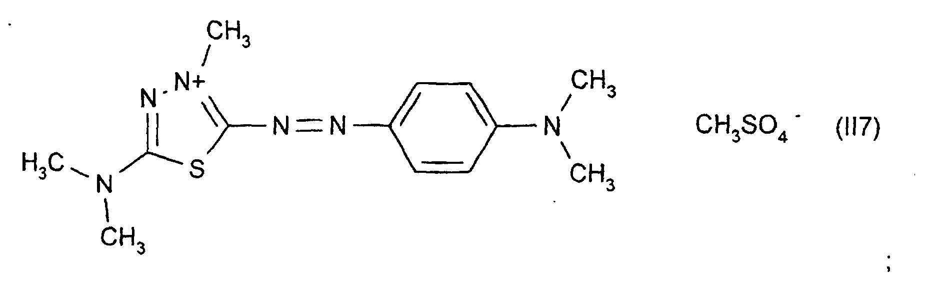 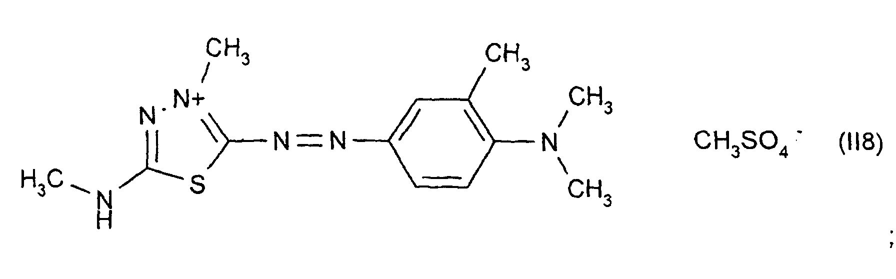 et 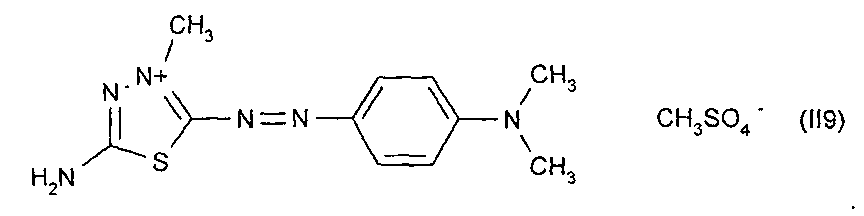
   Parmi les colorants directs cationiques de formule (III), utilisables dans les compositions tinctoriales conformes à l'invention, on peut plus particulièrement citer les composés répondant aux structures (III1) à (III18) suivantes : 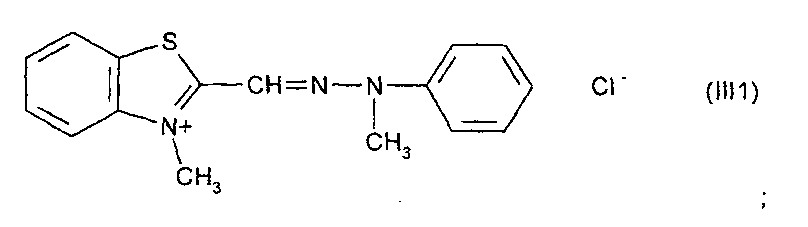 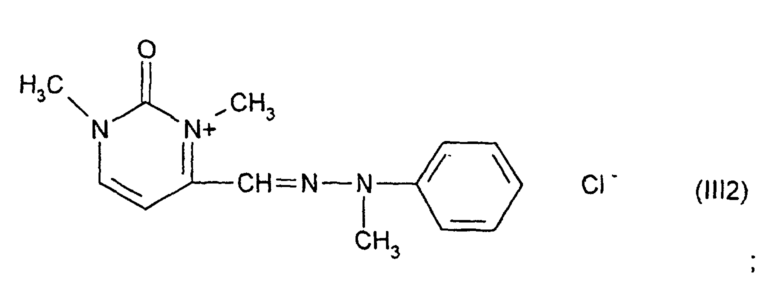 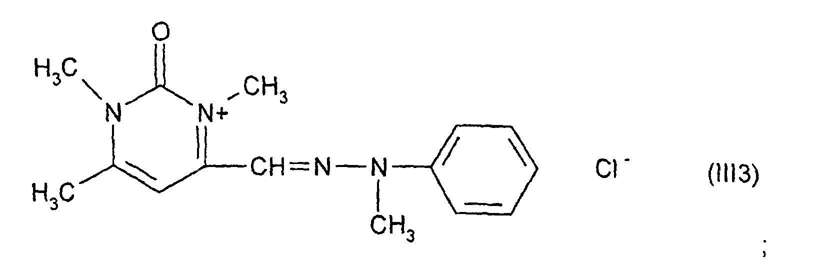 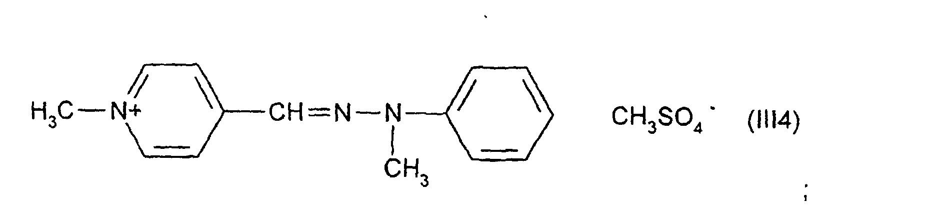 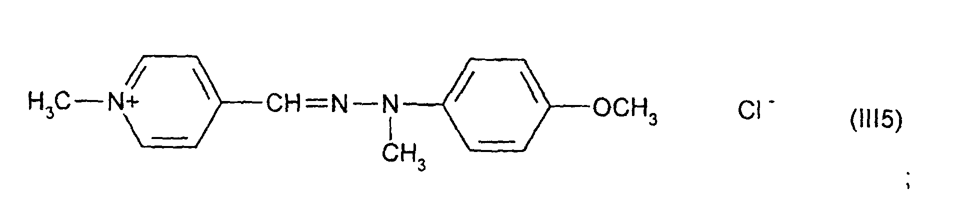 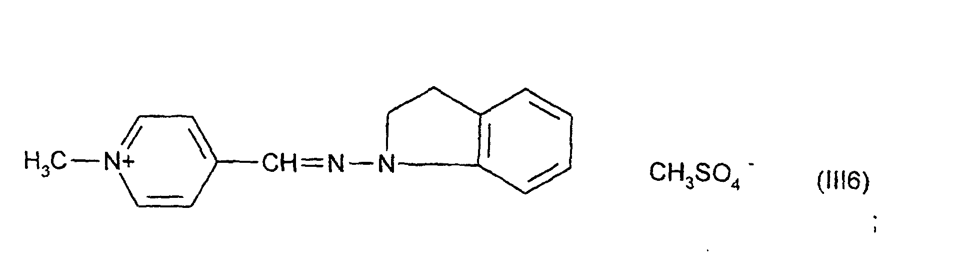 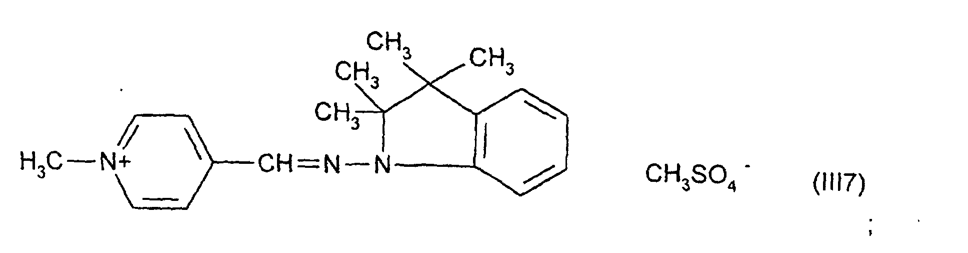 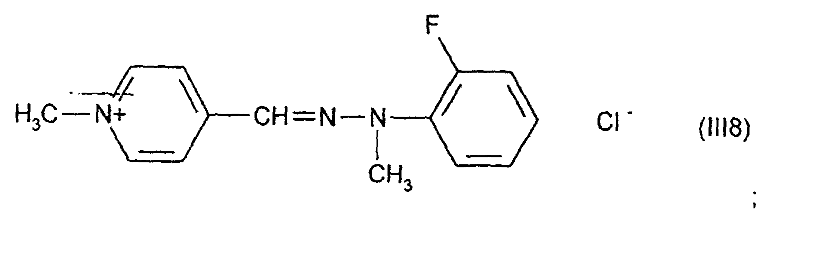 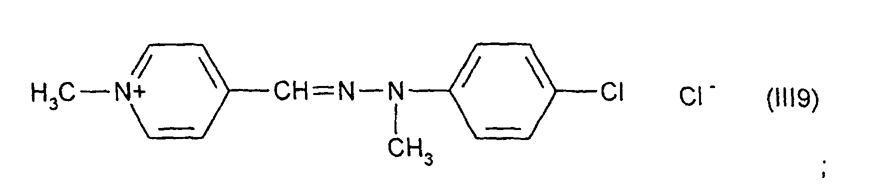 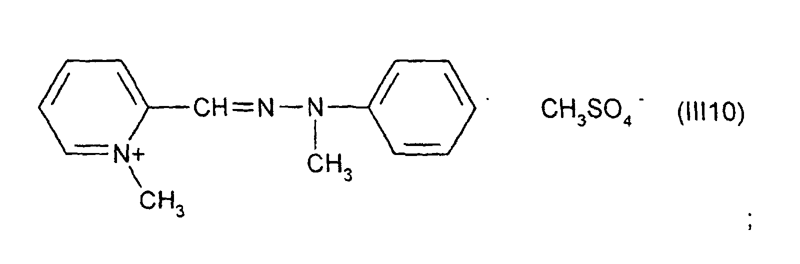 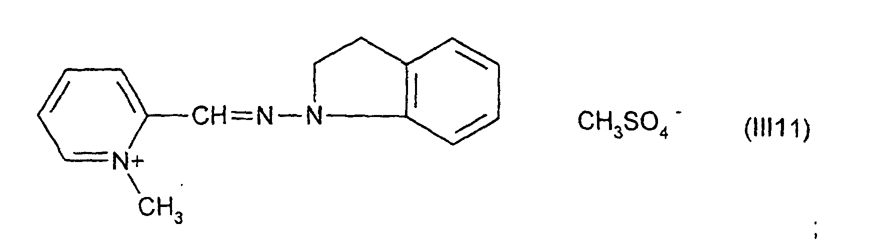 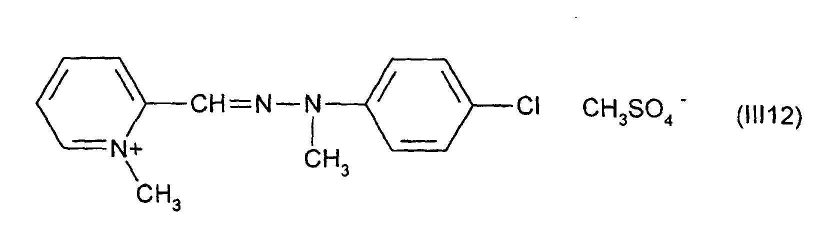 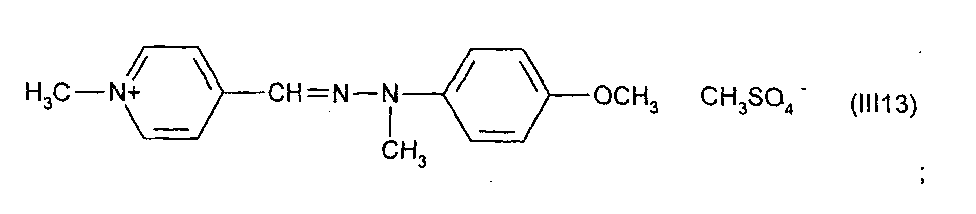 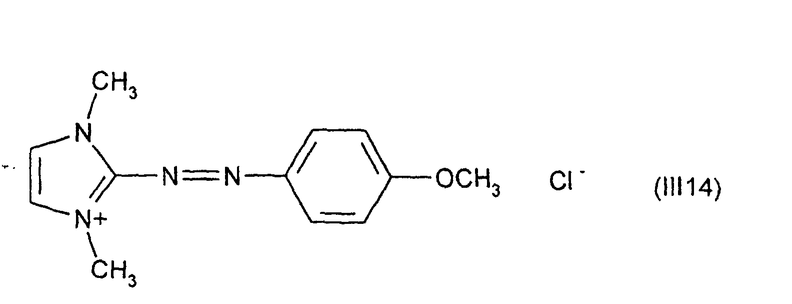 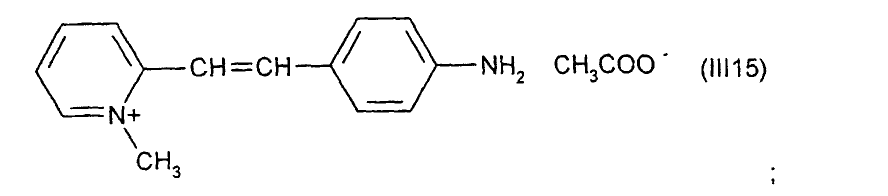 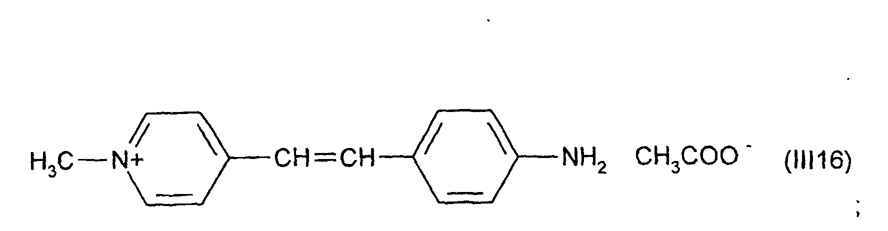 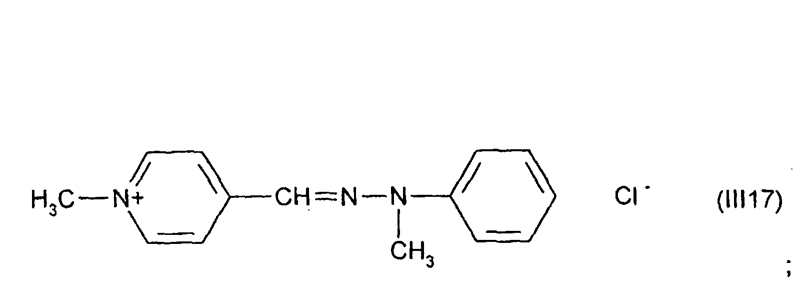 et 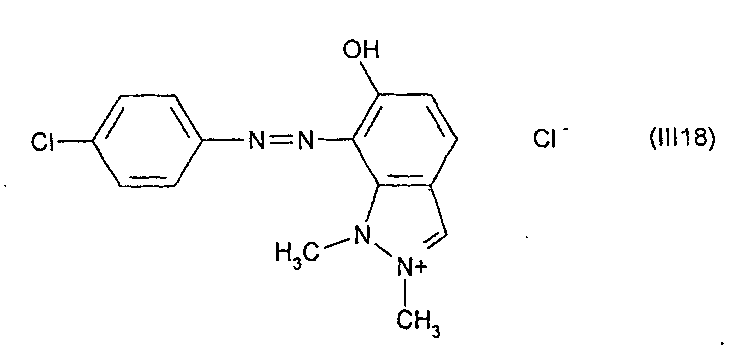
   Parmi les composés particuliers de structures (III1) à (III18) décrits ci-dessus, on préfère tout particulièrement les composés répondant aux structures (III4), (III5) et (III13).
   Parmi les colorants directs cationiques de formule (III'), utilisables dans les compositions tinctoriales conformes à l'invention, on peut plus particulièrement citer les composés répondant aux structures (III'1) à (III'3) suivantes : 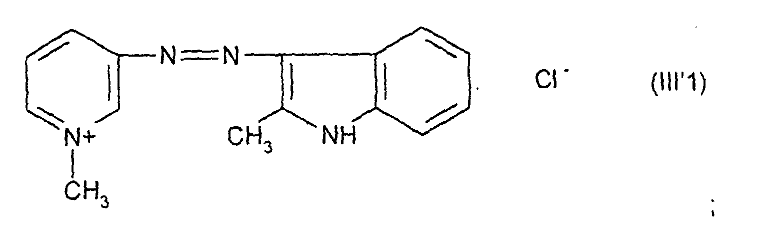 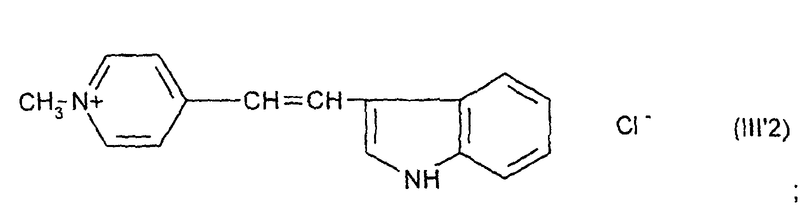 et 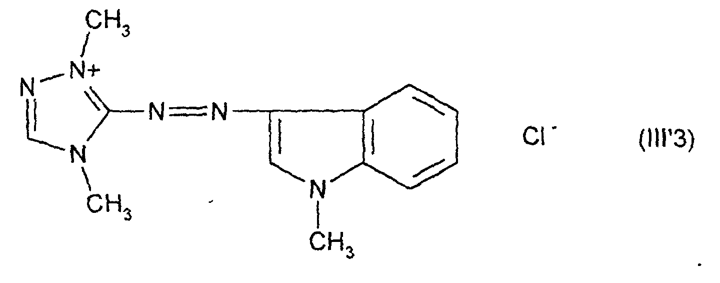
   Le ou les colorants directs cationiques utilisés selon l'invention, représentent de préférence de 0,001 à 10 % en poids environ du poids total de la composition tinctoriale et encore plus préférentiellement de 0,005 à 5 % en poids environ de ce poids.
**(ii)** Le polymère épaississant comportant au moins un motif sucre et utilisable selon la présente invention est choisi dans le groupe constitué par:
   **(ii)**_{**1**} - les gommes de guar non-ioniques;
   **(ii)**_{**2**} - les gommes de biopolysaccharides d'origine microbienne telles que les gommes de Scléroglucane ou de Xanthane;
   **(ii)**_{**3**} - les gommes issues d'exudats végétaux telles que les gommes Arabique, Ghatti, Karaya, Tragacanthe, Carrageenane, Agar et Caroube;
   **(ii)**_{**4**} - les pectines;
   **(ii)**_{**5**} - les alginates;
   **(ii)**_{**6**} - les amidons;
   **(ii)**_{**7**} - les hydroxyalkylcelluloses et carboxyalkylcelluloses.

Par "motif sucre", on désigne au sens de la présente invention, une portion monosaccharidique (i.e. monosaccharide ou oside ou sucre simple) ou une portion oligosaccharidique (chaînes courtes formées de l'enchaînement d'unités monosaccharidiques, éventuellement différentes) ou une portion polysaccharidique [longues chaînes constituées d'unités monosaccharidiques, éventuellement différentes, i.e. polyholosides ou polyosides (homopolyosides ou hétéropolyosides)]. Les unités saccharidiques peuvent être en outre substituées par des groupements alkyl, ou hydroxyalkyl, ou alcoxy, ou acyloxy, ou carboxyle.

Les gommes de guar non ioniques peuvent être modifiées ou non modifiées.
Les gommes de guar non modifiées sont par exemple les produits vendus sous la dénomination VIDOGUM GH 175 par la société UNIPECTINE et sous la dénomination JAGUAR C par la société MEYHALL.

Selon la présente invention, on préfère utiliser les gommes de guar non-ioniques modifiées par des groupements hydroxyalkyle en C₁-C₆.
Parmi les groupements hydroxyalkyle, on peut mentionner à titre d'exemple, les groupements hydroxyméthyle, hydroxyéthyle, hydroxypropyle et hydroxybutyle.
Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.
Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHONE POULENC (MEYHALL) ou sous la dénomination GALACTASOL 4H4FD2 par la société AQUALON.

Les gommes de biopolysaccharides d'origine microbienne telles que les gommes de scléroglucane ou de xanthane, les gommes issues d'exudats végétaux telles que les gommes Aabique, gomme Ghatti, gomme Karaya, Tragacanthe, Carrageenane, Agar et Caroube, les hydroxyalkylcelluloses et les carboxyméthylcelluloses, les pectines, les alginates et les amidons sont bien connus de l'homme de l'art et décrits notamment dans l'ouvrage de Robert L. DAVIDSON intitulé "Handbook of Water soluble gums and resins" édité chez Mc Graw Hill Book Company (1980).

Parmi ces gommes, les scléroglucanes plus particulièrement utilisés selon la présente invention, sont représentés par les produits vendus sous la dénomination ACTIGUM CS par la société SANOFI BIO INDUSTRIES et en particulier ACTIGUM CS 11 et sous la dénomination AMIGEL par la société ALBAN MULLER INTERNATIONAL. D'autres scléroglucanes tels que celui traité au glyoxal dans la demande de brevet français N°2633940 peuvent également être utilisés.

Les gommes de Xanthane plus particulièrement utilisés selon la présente invention, sont représentés par les produits vendus sous les dénominations KELTROL, KELTROL T, KELTROL TF KELTROL BT, KELTROL RD, KELTROL CG par la société NUTRASWEET KELCO, ou sous les dénominations RHODICARE S, RHODICARE H par la société RHODIA CHIMIE.

Les hydroxyalkylcelluloses sont plus particulièrement des hydroxyéthylcelluloses telles que celles vendues sous les dénominations CELLOSIZE QP3L, CELLOSIZE QP4400H, CELLOSIZE QP30000H, CELLOSIZE HEC30000A, CELLOSIZE POLYMER PCG10, par la société AMERCHOL, ou NATROSOL 250HHR, NATROSOL 250MR, NATROSOL 250M, NATROSOL 250HHXR, NATROSOL 250HHX, NATROSOL 250HR, NATROSOL HX, par la société HERCULES, ou encore TYLOSE H1000 par la société HOECHST.
Les hydroxyalkylcelluloses sont également plus particulièrement des hydroxypropylcelluloses comme les produits vendus sous les dénominations KLUCEL EF, KLUCEL H, KLUCEL LHF, KLUCEL MF, KLUCEL G, par la société AQUALON.

Parmi les carboxyalkylcelluloses, on utilise de préférence la carboxyméthylcellulose dont on peut citer les produits vendus sous les dénominations BLANOSE 7M8/SF, BLANOSE RAFFINEE 7M, BLANOSE 7LF, BLANOSE 7MF, BLANOSE 9M31F, BLANOSE 12M31XP, BLANOSE 12M31P, BLANOSE 9M31XF, BLANOSE 7H, BLANOSE 7M31, BLANOSE 7H3SXF, par la société AQUALON, ou encore AQUASORB A500 et AMBERGUM 1221, par le société HERCULES, ou encore CELLOGEN HP810A et CELLOGEN HP6HS9, par la société MONTELLO, ou encore PRIMELLOSE par la société AVEBE.

Les polymères épaississants (ii) utilisés dans les compositions de la présente invention sont présents de préférence à raison de 0,01 à 10% environ en poids, en particulier à raison de 0,1 à 5% environ en poids par rapport au poids total de la composition de teinture appliquée sur les fibres kératiniques.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les alcools aromatiques comme l'alcool benzylique, ainsi que les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 2 et 11 environ, et de préférence entre 5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VIII) suivante : 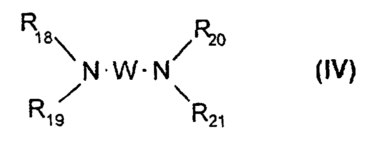 dans laquelle W est un reste propylène éventuellement substitué par un groupement hydroxyle ou un radical alkyle en C₁-C₆ ; R₁₈ R₁₉ R₂₀ R₂₁ identiques ou différents, représentent un atome d'hydrogène, un radical alkyle en C₁-C₆ ou hydroxyalkyle en C₁-C₆.

La composition tinctoriale conforme à l'invention peut, en plus du ou des colorants directs cationiques (i) définis précédemment, contenir un ou plusieurs colorants directs additionnels qui peuvent par exemple être choisis parmi les colorants benzéniques nitrés, les colorants anthraquinoniques, les colorants naphtoquinoniques, les colorants triarylméthaniques, les colorants xanthéniques, les colorants azoïques non cationiques.

Lorsqu'elle est destinée à la teinture d'oxydation, la composition tinctoriale conforme à l'invention contient, en plus du ou des colorants directs cationiques (i) une ou plusieurs bases d'oxydation, choisie parmi les bases d'oxydation classiquement utilisées pour la teinture d'oxydation et parmi lesquelles on peut notamment citer les paraphénylènediamines, les bis-phénylalkylènediamines, les para-aminophénols, les ortho-aminophénols et les bases hétérocycliques.
Lorsqu'elles sont utilisées, la ou les bases d'oxydation représentent de préférence de 0,0005 à 12 % en poids environ du poids total de la composition tinctoriale, et encore plus préférentiellement de 0,005 à 6 % en poids environ de ce poids.

Lorsqu'elle est destinée à la teinture d'oxydation, la composition tinctoriale conforme à l'invention peut également renfermer, en plus du colorant direct cationique (i) et du polymère épaississant (ii) ainsi que des bases d'oxydation, un ou plusieurs coupleurs de façon à modifier ou à enrichir en reflets les nuances obtenues en mettant en oeuvre le ou les colorants direct(s) cationique(s) (i) et la ou les bases d'oxydation.

Les coupleurs utilisables dans la composition tinctoriale conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.
Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des agents tensioactifs, des agents filmogènes, des céramides, des agents conservateurs, des agents filtrants, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale selon l'invention peut se présenter sous des formes diverses, telles que sous forme de liquides, de shampooings, de crèmes, de gels, ou sous toute autre forme appropriée pour réaliser une teinture des fibres kératiniques, et notamment des cheveux humains. Elle peut être obtenue par mélange extemporané d'une composition, éventuellement pulvérulente, contenant le ou les colorants directs cationiques avec une composition contenant le polymère épaississant (ii) selon l'invention.

Lorsque l'association du colorant direct cationique (i) et du polymère épaississant (ii) selon l'invention est utilisée dans une composition destinée à la teinture d'oxydation (une ou plusieurs bases d'oxydation sont alors utilisées, éventuellement en présence d'un ou plusieurs coupleurs) ou lorsqu'elle est utilisée dans une composition destinée à la teinture directe éclaircissante, alors la composition tinctoriale conforme à l'invention renferme en outre au moins un agent oxydant, choisi par exemple parmi le peroxyde d'hydrogène, le peroxyde d'urée, les bromates de métaux alcalins, les persels tels que les perborates et persulfates, et les enzymes telles que les peroxydases, les laccases et les oxydoréductases à deux électrons. L'utilisation du peroxyde d'hydrogène ou des enzymes est particulièrement préférée.

Un autre objet de l'invention est un procédé de teinture des fibres kératiniques et en particulier des fibres kératiniques humaines telles que les cheveux mettant en oeuvre la composition tinctoriale telle que définie précédemment.

Selon une première variante de ce procédé de teinture conforme à l'invention, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une deuxième variante de ce procédé de teinture conforme à l'invention, on applique sur les fibres au moins une composition tinctoriale telle que définie précédemment, pendant un temps suffisant pour développer la coloration désirée, sans rinçage final.

Selon une forme de réalisation particulière de ce procédé de teinture, et lorsque la composition tinctoriale conforme à l'invention renferme au moins une base d'oxydation et au moins un agent oxydant, le procédé de teinture comporte une étape préliminaire consistant à stocker sous forme séparée, d'une part, une composition (A1) comprenant, dans un milieu approprié pour la teinture, au moins un colorant direct cationique (i) tel que défini précédemment et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant le polymère épaississant (ii) tel que défini précédemment.

Selon une autre forme de réalisation particulière de ce procédé de teinture, et lorsque la composition tinctoriale conforme à l'invention renferme au moins un agent oxydant, le procédé de teinture comporte une étape préliminaire consistant à stocker sous forme séparée, d'une part, une composition (A2) comprenant, dans un milieu approprié pour la teinture, au moins un colorant direct cationique (i) tel que défini précédemment et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant le polymère épaississant tel que défini précédemment.

Un autre objet de l'invention est un dispositif à plusieurs compartiments ou "kit" de teinture ou tout autre système de conditionnement à plusieurs compartiments dont un premier compartiment renferme la composition (A1) ou (A2) telle que définie ci-dessus et un second compartiment renferme la composition (B1) ou (B2) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 à 4:

On a préparé les quatre compositions de teinture directe réunies dans le tableau suivant :
(*toutes teneurs exprimées en grammes*)

| | **Exemple 1** | **Exemple 2** | **Exemple 3** | **Exemple 4** |
|---|---|---|---|---|
| Colorant direct cationique de formule (I1) | 0,2 | | | |
| Colorant direct cationique de formule (I2) | | 0,2 | | |
| Colorant direct cationique de formule | | | | |
| (I14) | | | 0,2 | |
| Colorant direct cationique de formule (I31) | | | | 0,15 |
| Hydroxyéthylcellulose vendue sous la dénomination NATROSOL 250 HHR par la société AQUALON | 1,0 MA * | | | |
| Carboxyméthylcellulose vendue sous la dénomination BLANOSE 7M par la société AQUALON | | 1,0 MA * | | |
| Gomme de Guar vendue sous la dénomination VIDOGUM GH175 par la société UNIPECTINE | | | 1,0 MA * | |
| Gomme de Scléroglucane vendue sous la dénomination AMIGEL par la société ALBAN MULLER INTERNATIONAL | | | | 1,0 MA * |
| Ethanol | 10 | 10 | 10 | 10 |
| 2-amino-2-méthyl-1-propanol qs | pH 9 | pH 9 | pH 9 | pH 9 |
| Eau déminéralisée qsp | 100 | 100 | 100 | 100 |

| | | | | |
|---|---|---|---|---|
| MA* désigne Matière Active | | | | |

Les compositions ci-dessus ont été appliquées chacune pendant 30 minutes sur des mèches de cheveux gris naturels à 90 % de blancs. Les mèches de cheveux ont ensuite été rincées, lavées avec un shampooing standard puis séchées.

Les mèches ont été teintes dans les nuances suivantes :

| **Exemples** | **Nuances obtenues** |
|---|---|
| 1 | Rouge puissant |
| 2 | Rouge puissant |
| 3 | Orangé puissant |
| 4 | Violet puissant |

## Revendications

1. Composition pour la teinture des fibres kératiniques et en particulier des fibres kératiniques humaines telles que les cheveux, renfermant dans un milieu approprié pour la teinture, (i)au moins composé choisi parmi ceux de formules (I), (II), (III), (III') suivantes:
**a) les composés de formule (I) suivante :** 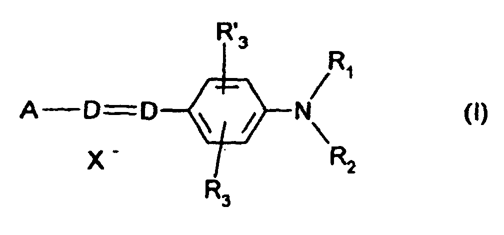 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄ , alcoxy en C₁-C₄ ou acétyloxy,
X ⁻ représente un anion de préférence choisi parmi le chlorure, le méthyl sulfate et l'acétate,
A représente un groupement choisi par les structures A1 à A18 suivantes : 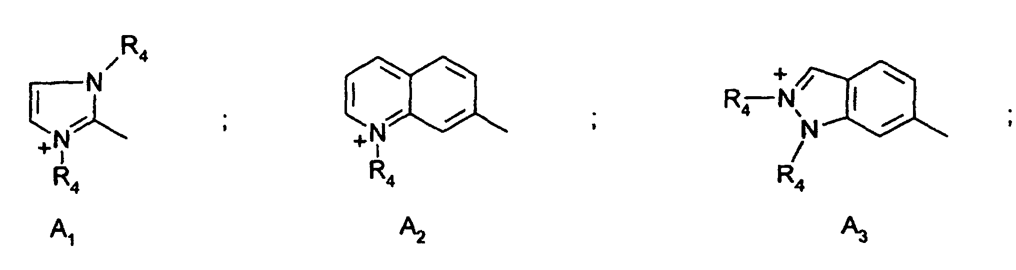 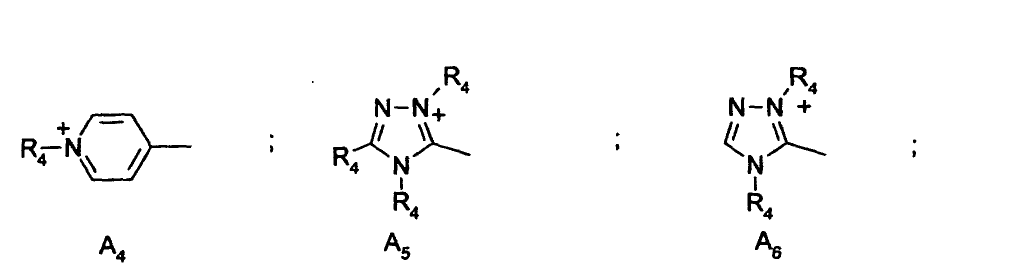 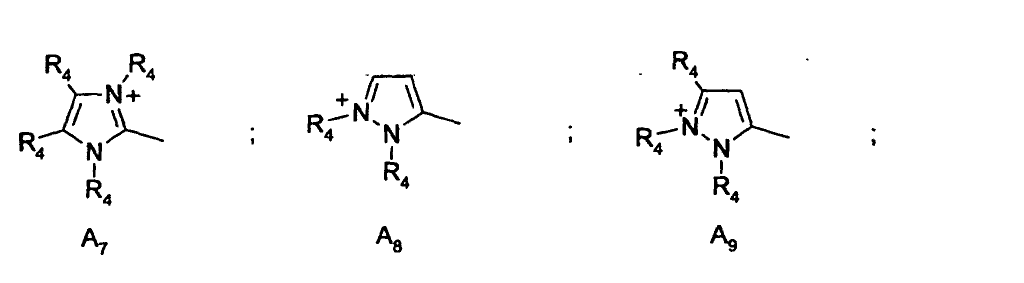 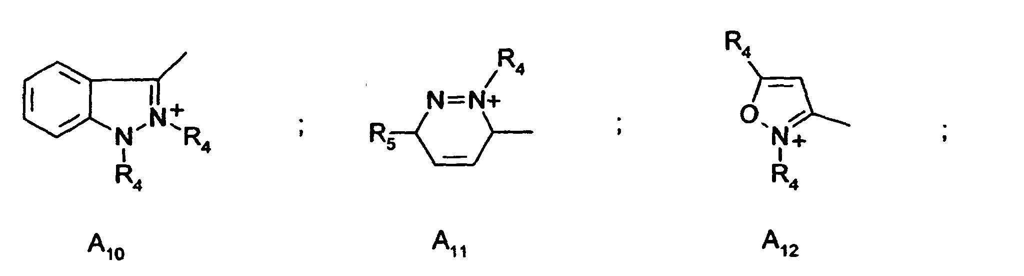 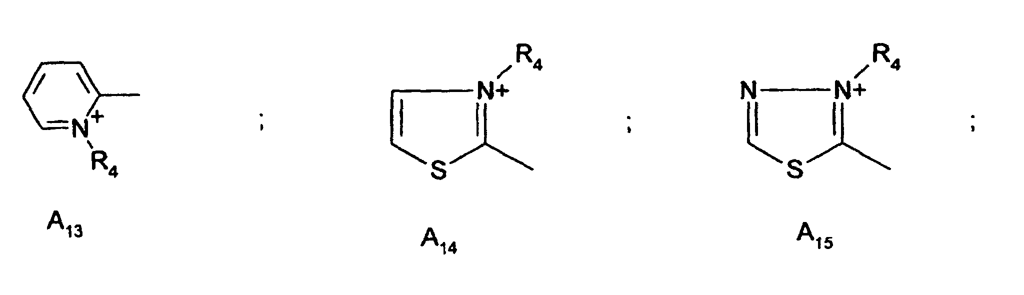 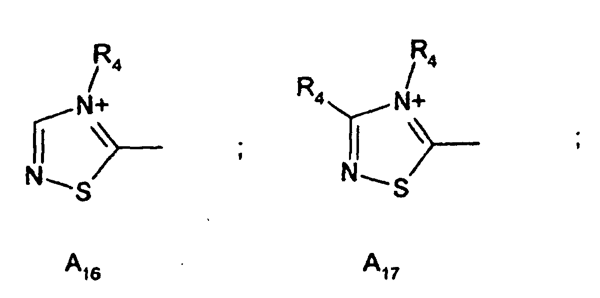 et 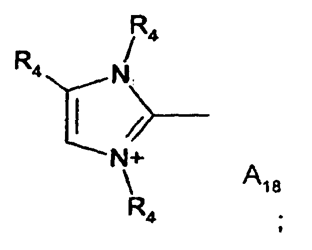
dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**b) les composés de formule (II) suivante :** 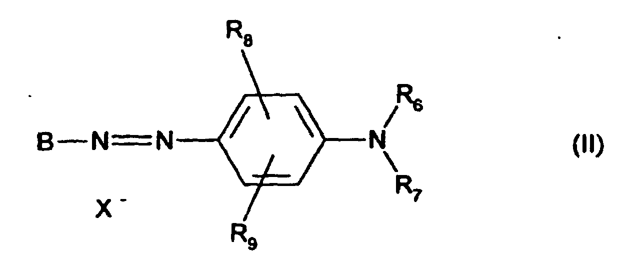 dans laquelle :
R₆ représente un atome d'hydrogène ou un radical alkyle en C₁-C₄,
R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X ⁻ représente un anion de préférence choisi parmi le chlorure, le méthyl sulfate et l'acétate,
B représente un groupement choisi par les structures B1 à B6 suivantes : 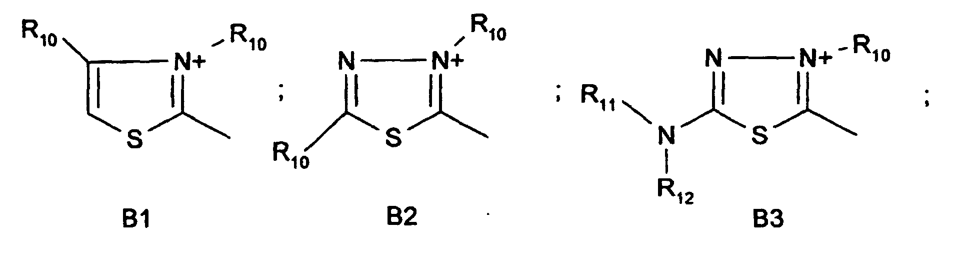 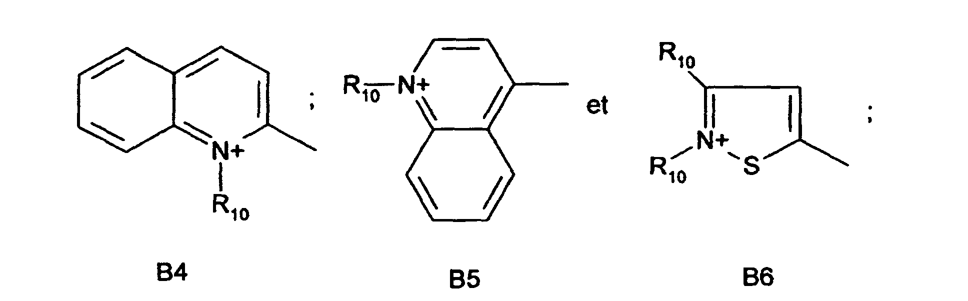 dans lesquelles R₁₀ représente un radical alkyle en C₁-C₄, R₁₁ et R₁₂, identiques ou différents, représentent un atome d'hydrogène ou un radical alkyle en C₁-C₄ ;
**c) les composés de formules (III) et (III') suivantes :** 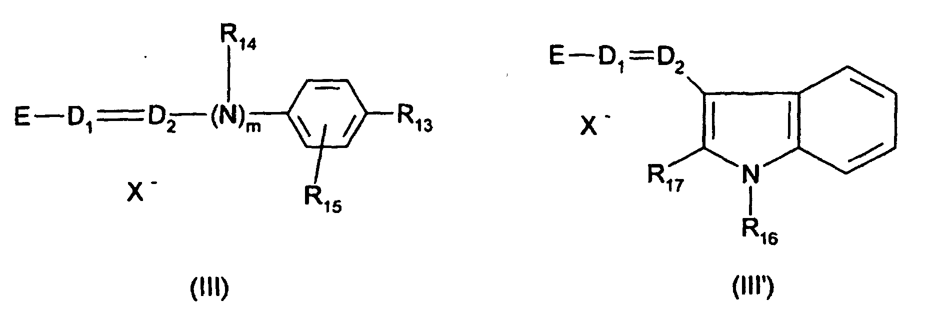 dans lesquelles :
R₁₃ représente un atome d'hydrogène, un radical alcoxy en C₁-C₄, un atome d'halogène tel que le brome, le chlore, l'iode ou le fluor ou un radical amino,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
R₁₆ et R₁₇, identiques ou différents, représentent un atome d'hydrogène ou un radical alkyle en C₁-C₄,
D₁ et D₂, identiques ou différents, représentent un atome d'azote ou le groupement -CH,
m = 0 ou 1,
étant entendu que lorsque R₁₃ représente un groupement amino non substitué, alors D₁ et D₂ représentent simultanément un groupement -CH et m = 0,
X ⁻ représente un anion de préférence choisi parmi le chlorure, le méthyl sulfate et l'acétate,
E représente un groupement choisi par les structures E1 à E8 suivantes : 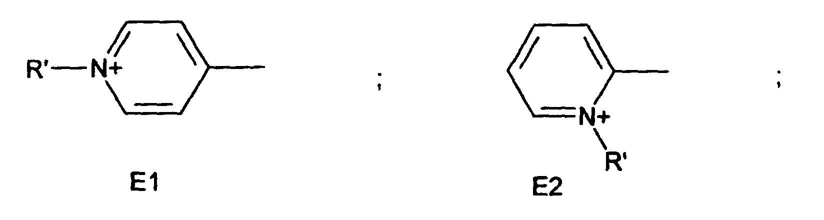 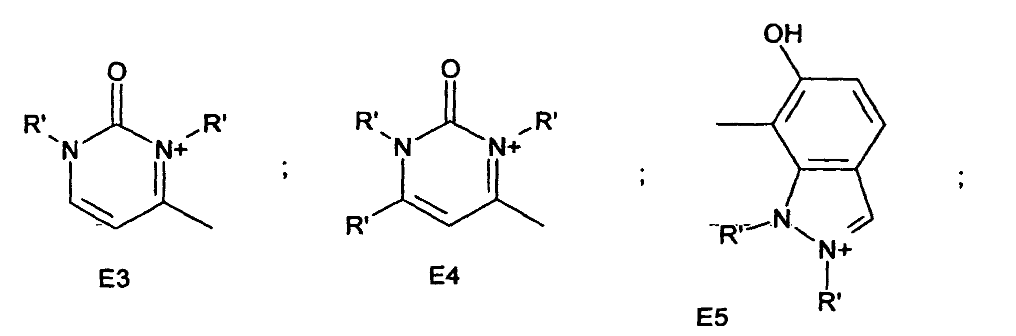 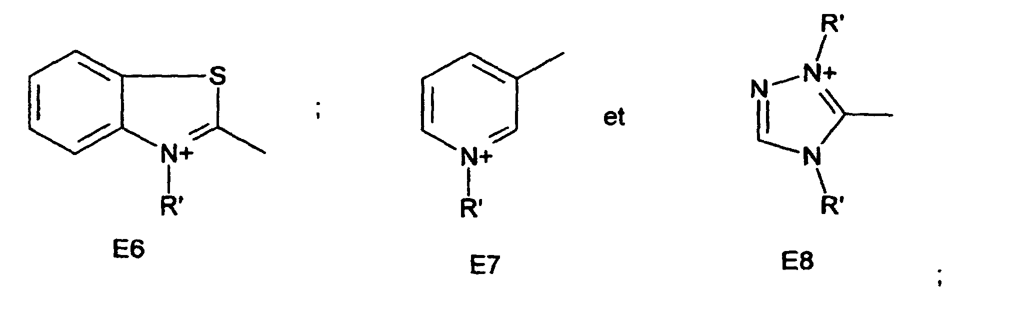 dans lesquelles R' représente un radical alkyle en C₁-C₄ ;
lorsque m = 0 et que D₁ représente un atome d'azote, alors E peut également désigner un groupement de structure E9 suivante : 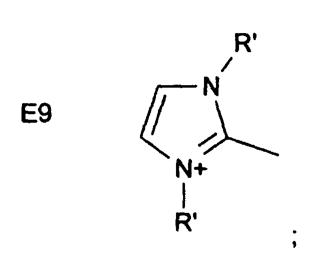 dans laquelle R' représente un radical alkyle en C₁-C₄;
ladite composition étant **caractérisée par le fait qu'**elle contient en outre (ii) au moins un polymère épaississant comportant au moins un motif sucre choisi dans le groupe comprenant :
**(ii)**_{**1**} - les gommes de guar non-ioniques;
**(ii)**_{**2**} - les gommes de biopolysaccharides d'origine microbienne telles que les gommes de Scléroglucane ou de Xanthane;
**(ii)**_{**3**} - les gommes issues d'exudats végétaux telles que les gommes Arabique, Ghatti, Karaya, Tragacanthe, Carrageenane, Agar et Caroube;
**(ii)**_{**4**} - les pectines;
**(ii)**_{**5**} - les alginates;
**(ii)**_{**6**} - les amidons;
**(ii)**_{**7**} - les hydroxyalkylcelluloses et carboxyalkylcelluloses.

2. Composition selon la revendication 1, **caractérisée par le fait que** les colorants directs cationiques de formule (I) sont choisis parmi les composés répondant aux structures (I1) à (I54) suivantes :

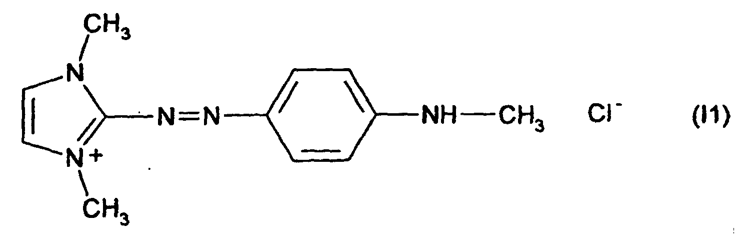

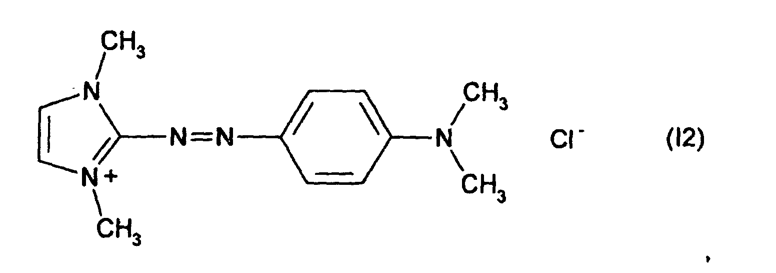

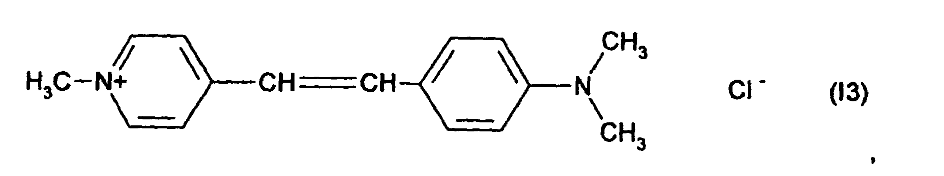

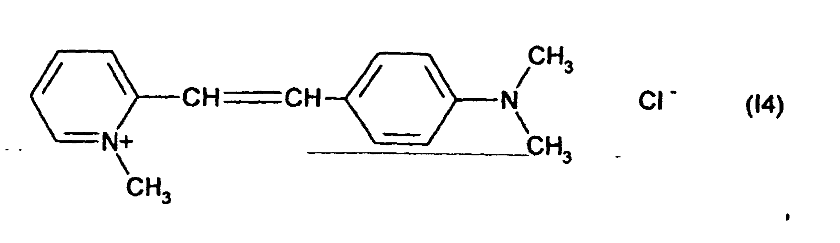

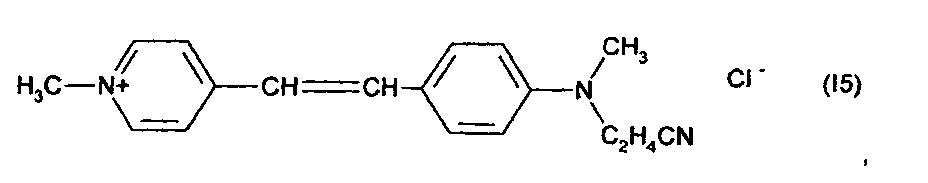

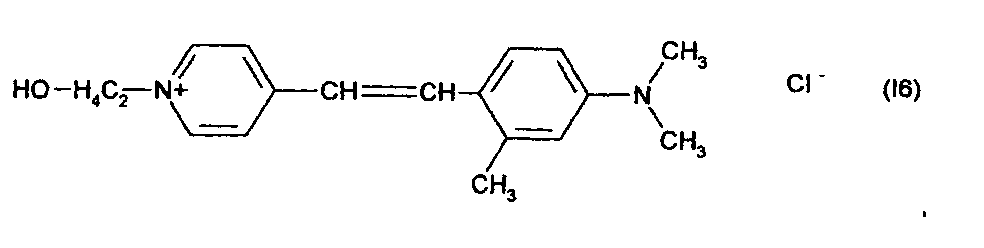

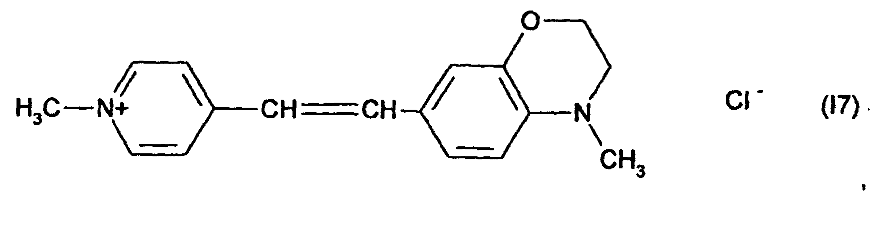

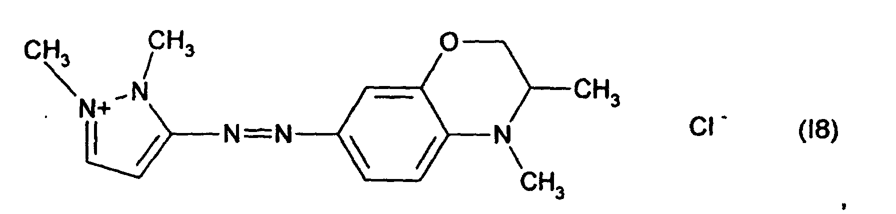

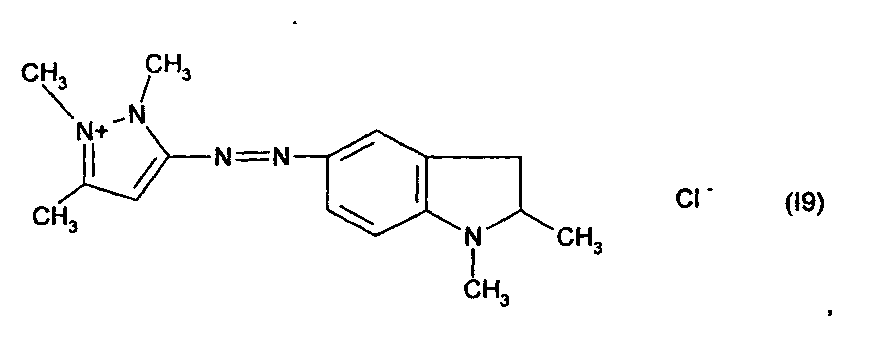

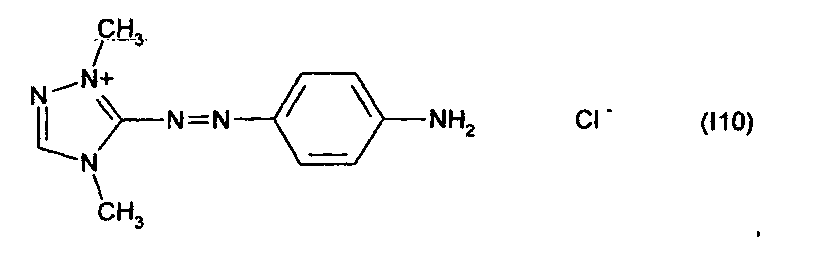

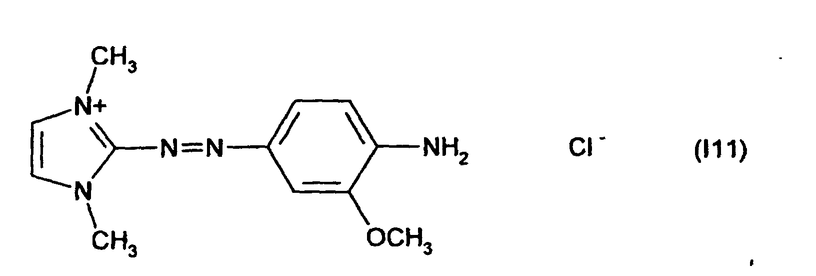

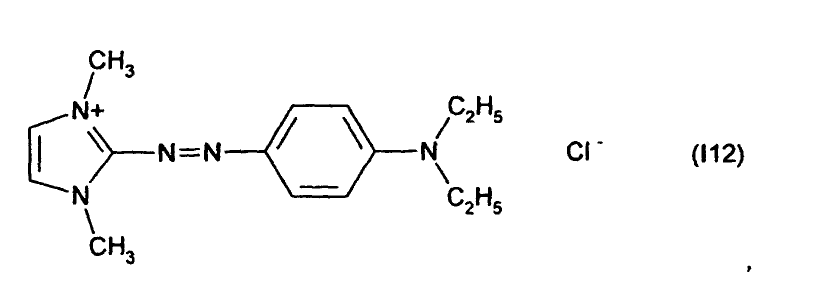

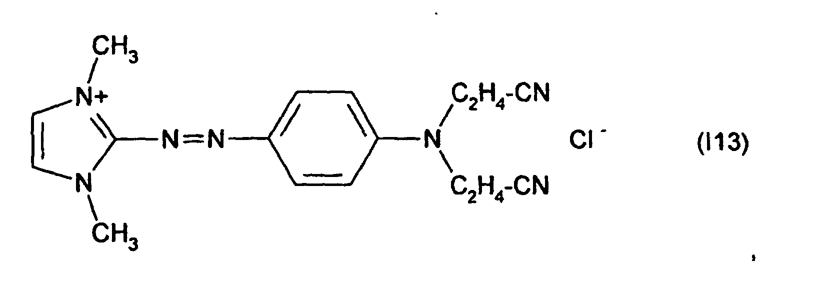

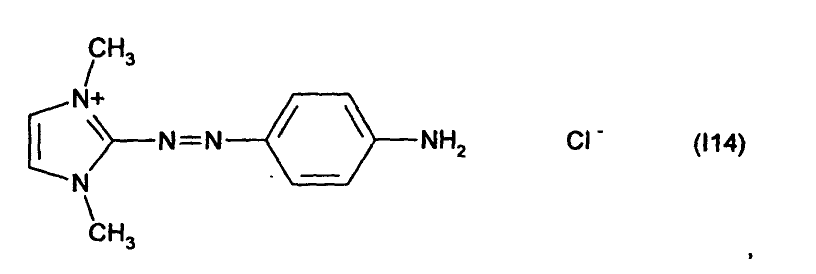

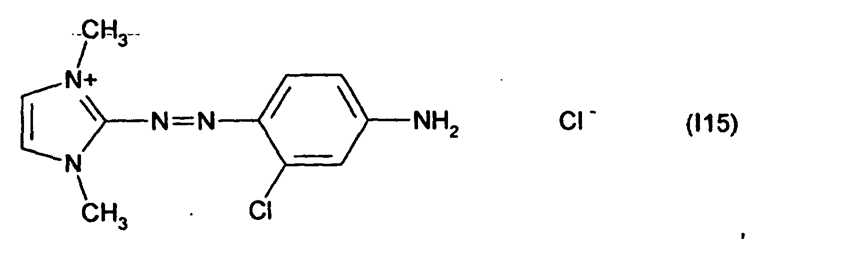

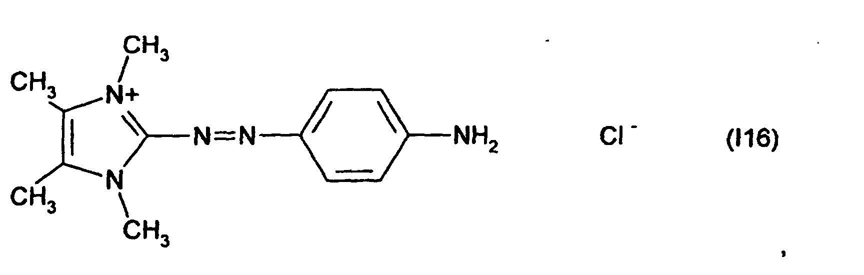

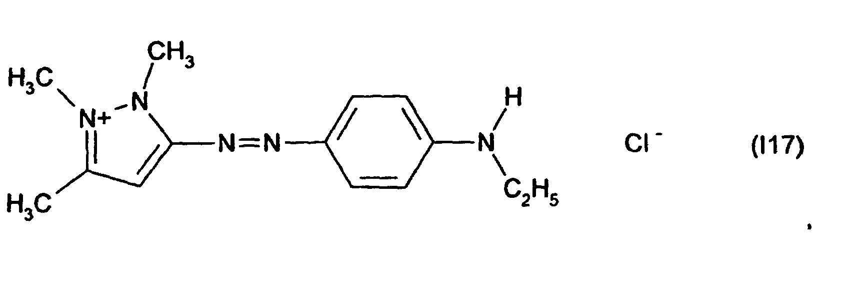

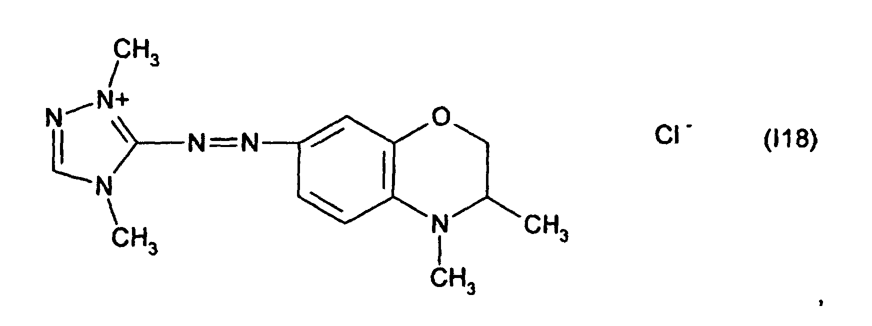

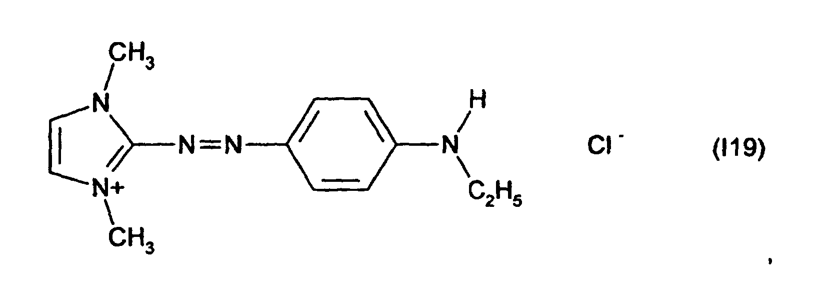

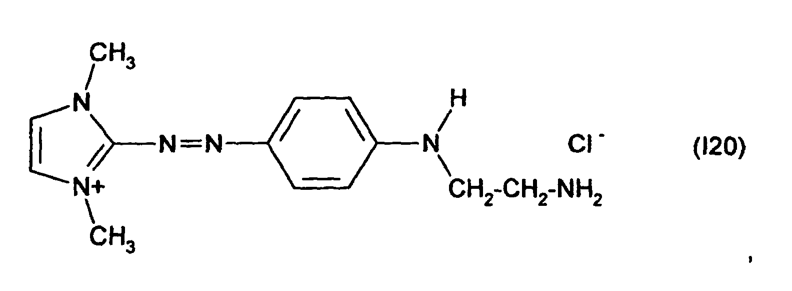

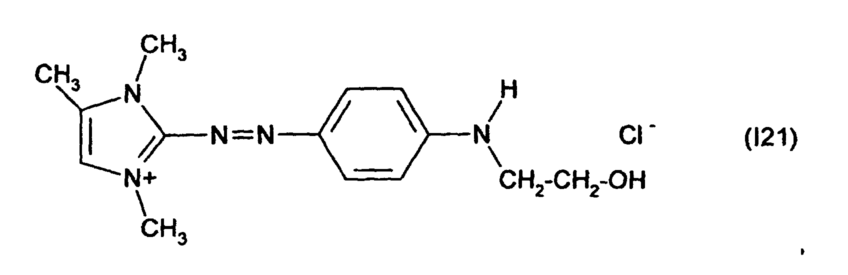

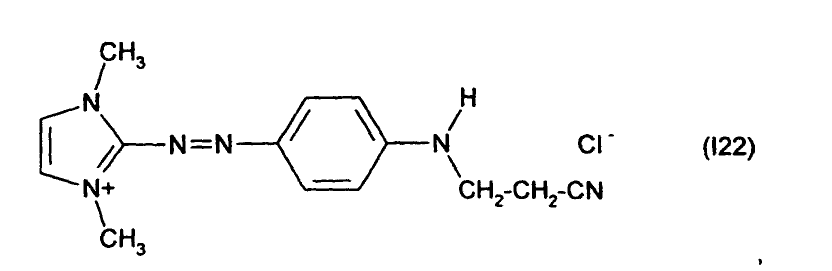

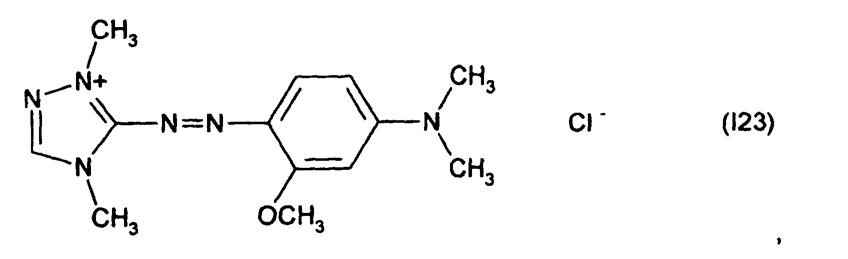

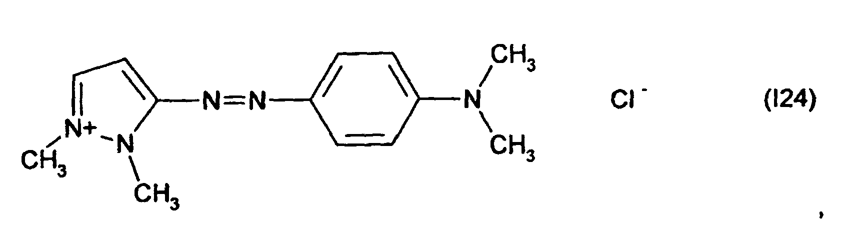

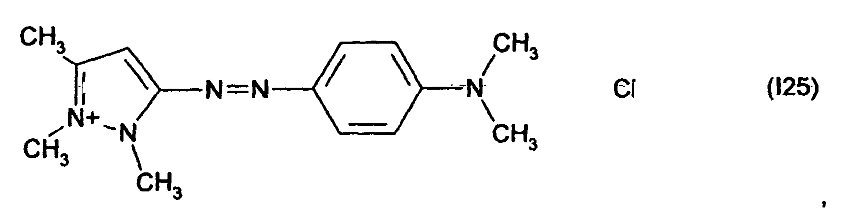

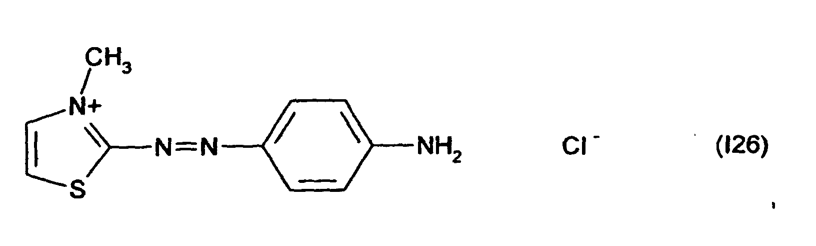

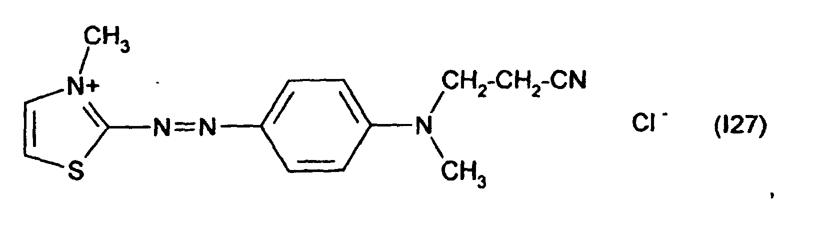

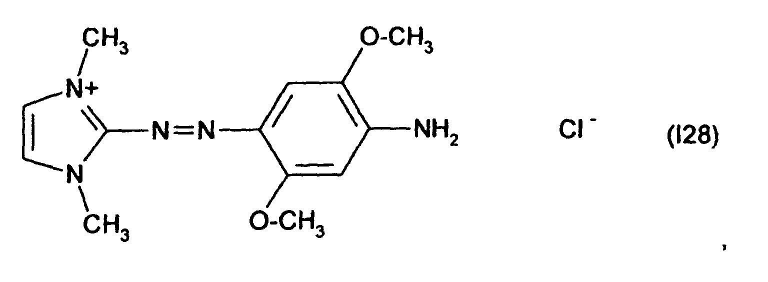

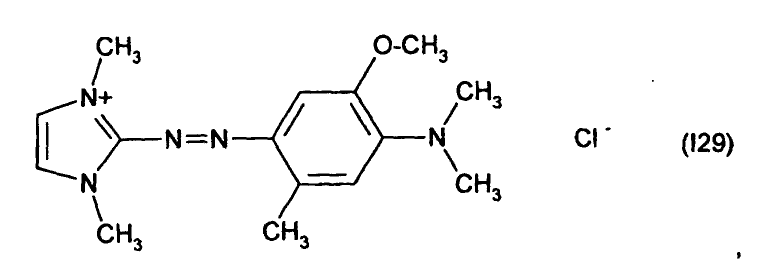

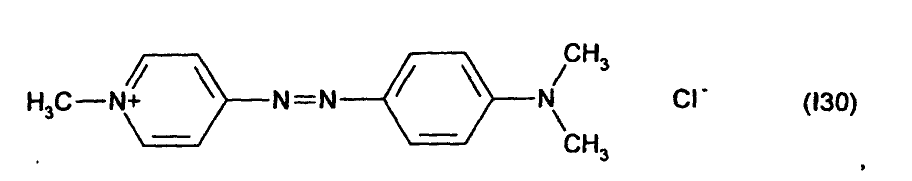

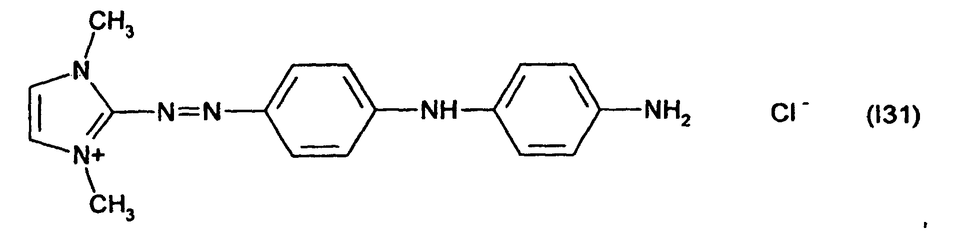

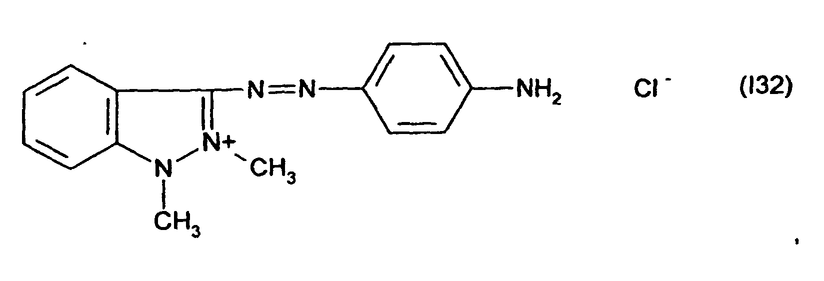

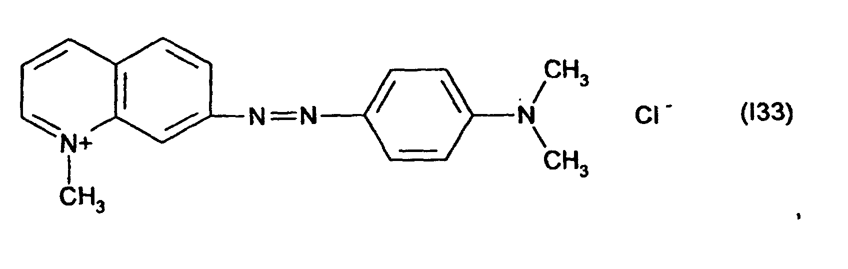

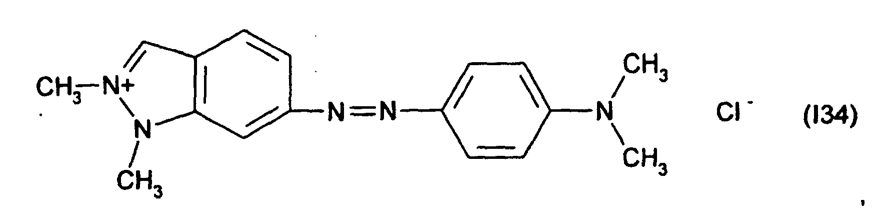

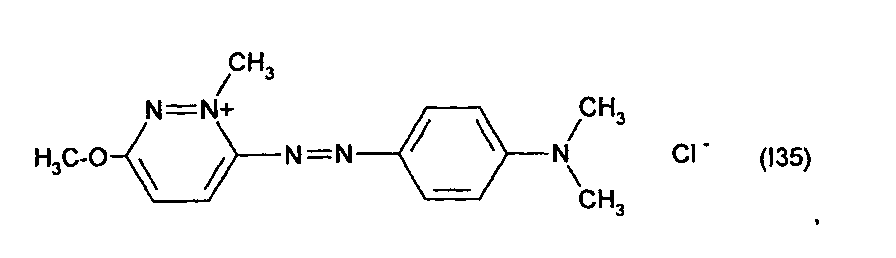

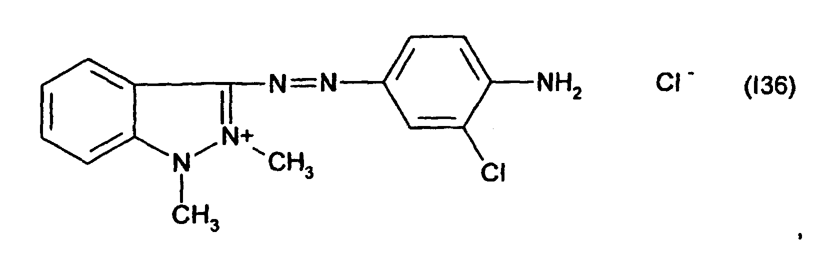

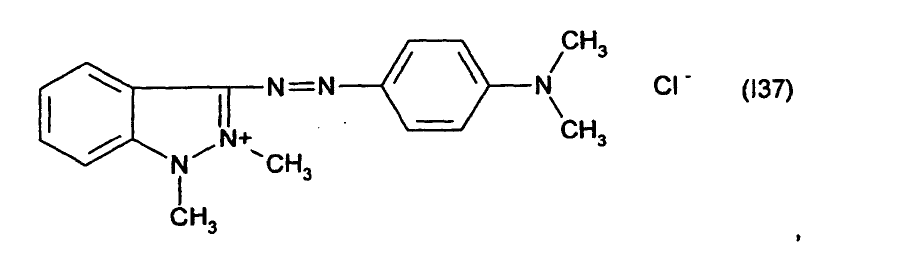

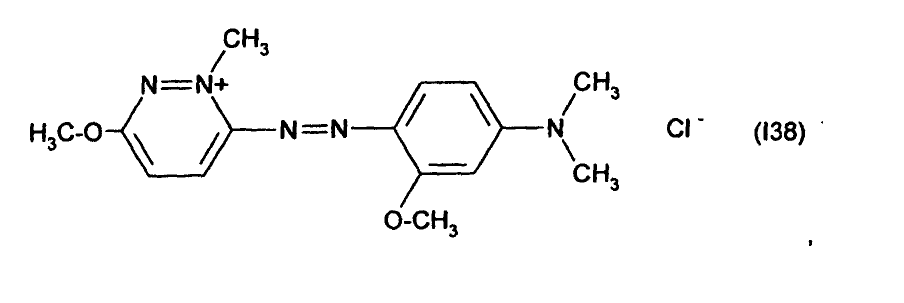

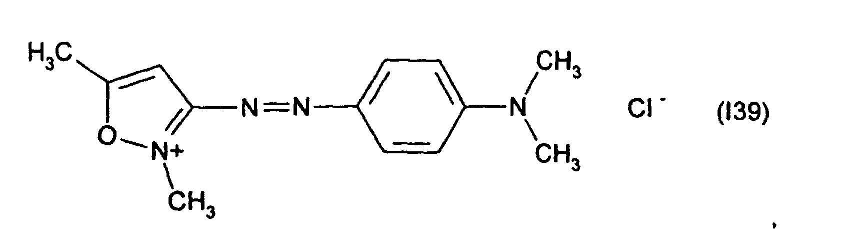

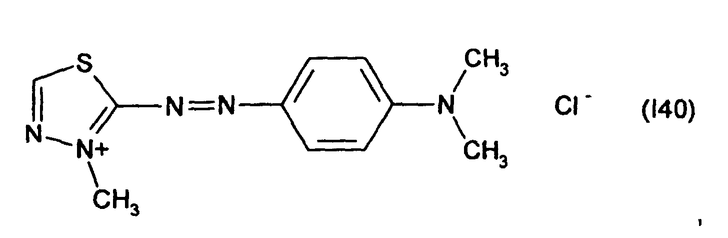

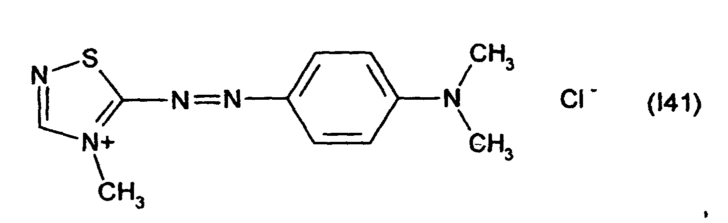

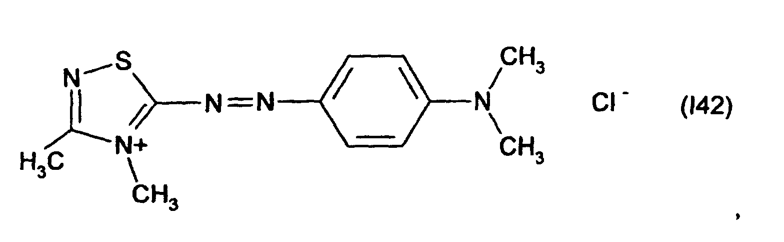

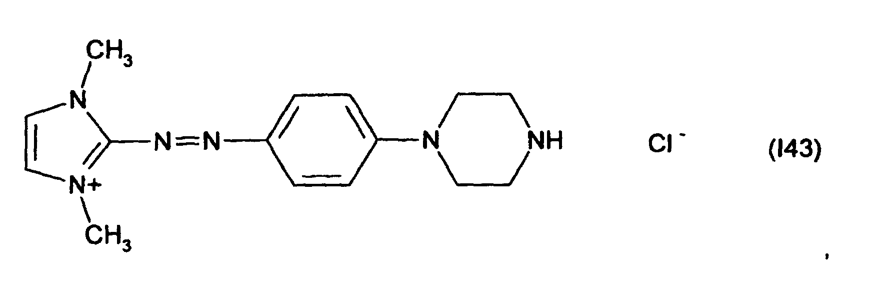

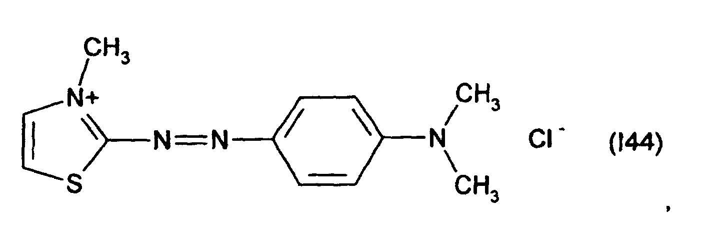

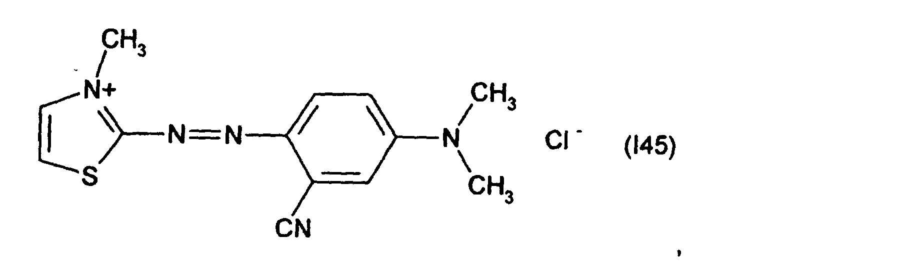

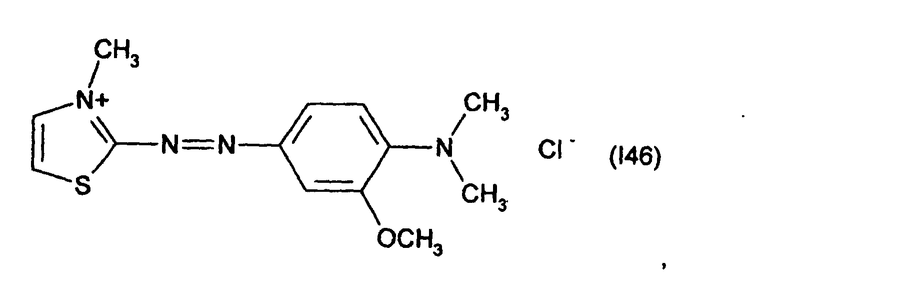

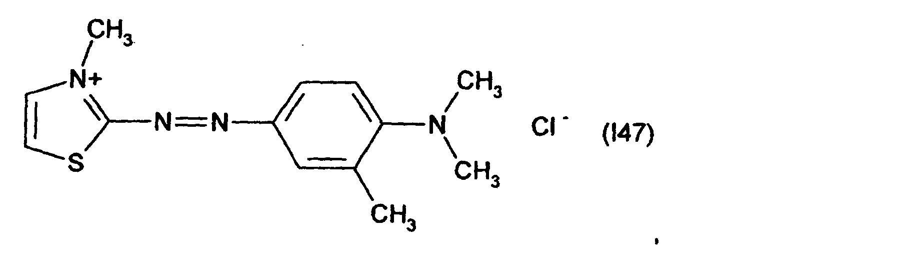

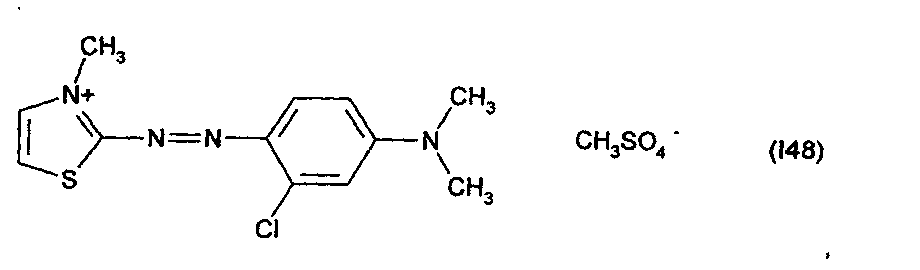

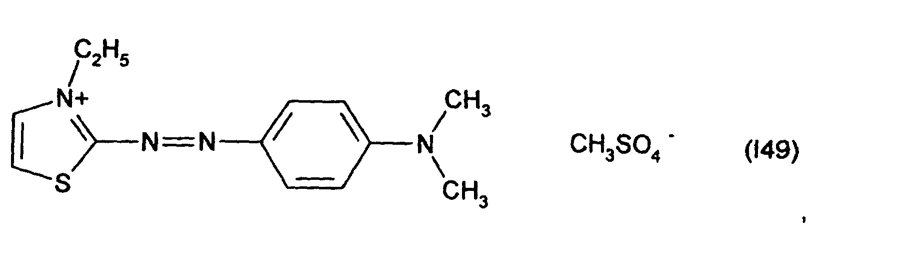

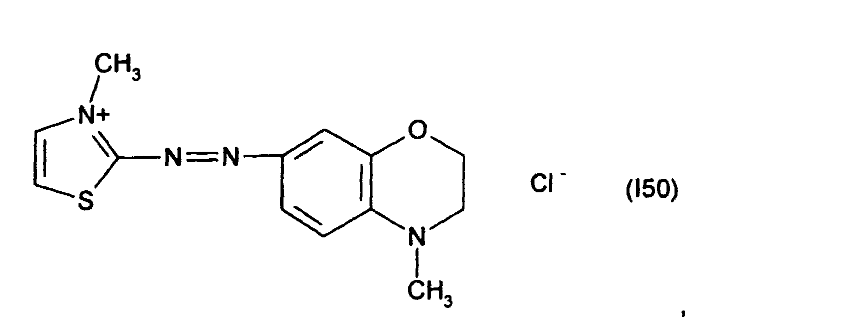

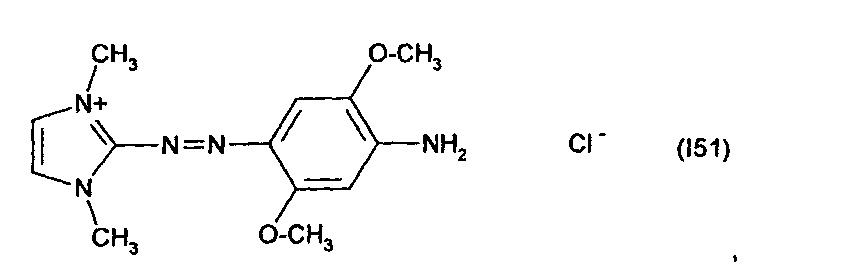

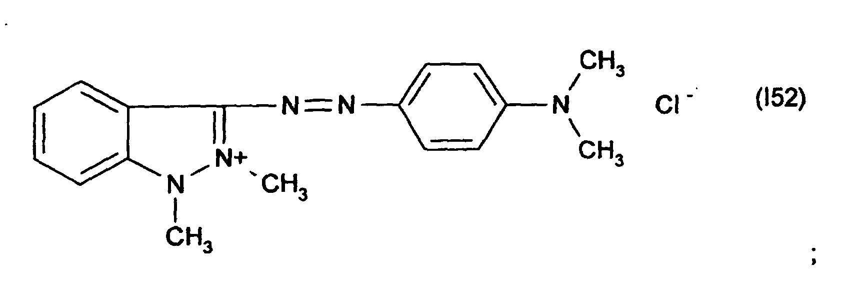

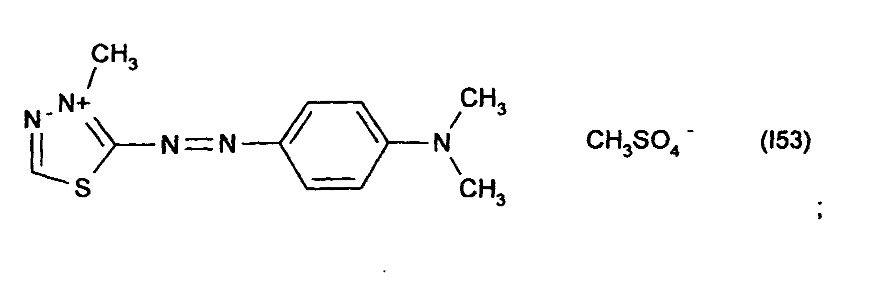

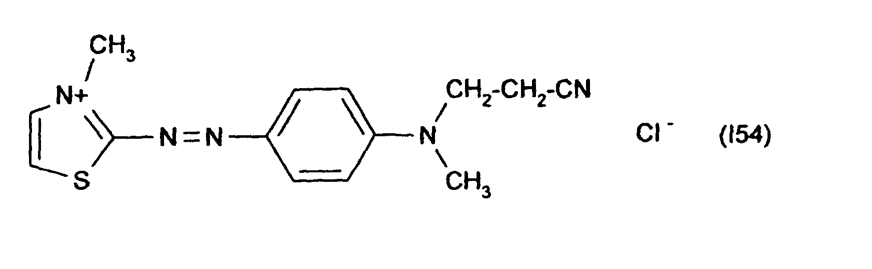

3. Composition selon la revendication 2, **caractérisée par le fait que** les colorants directs cationiques répondent aux structures (I1), (I2), (I14), et (I31).

4. Composition selon les revendications 1 ou 2, **caractérisée par le fait que** les colorants directs cationiques de formule (II) sont choisis parmi les composés répondant aux structures (II1) à (II9) suivantes : 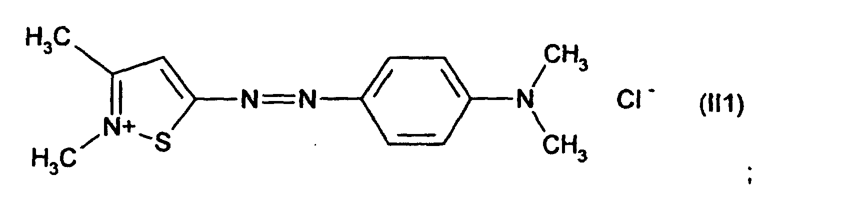 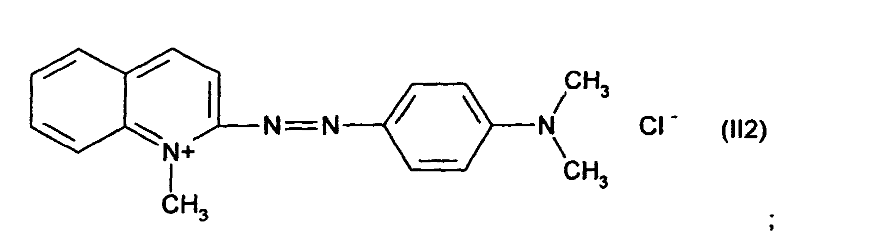 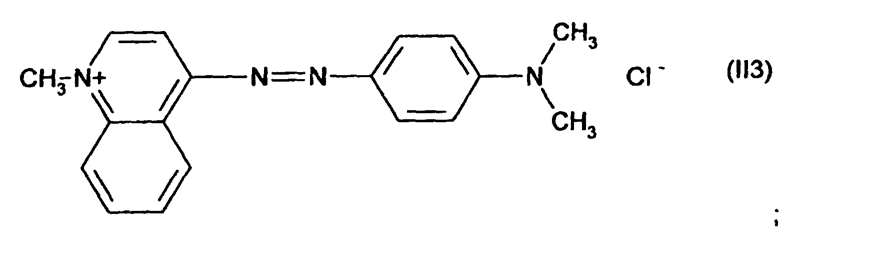 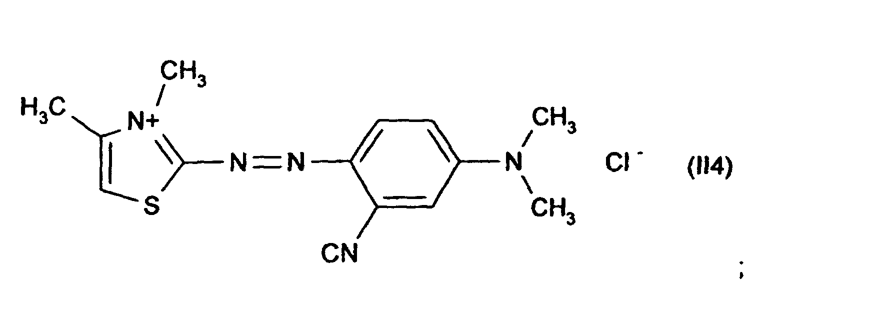 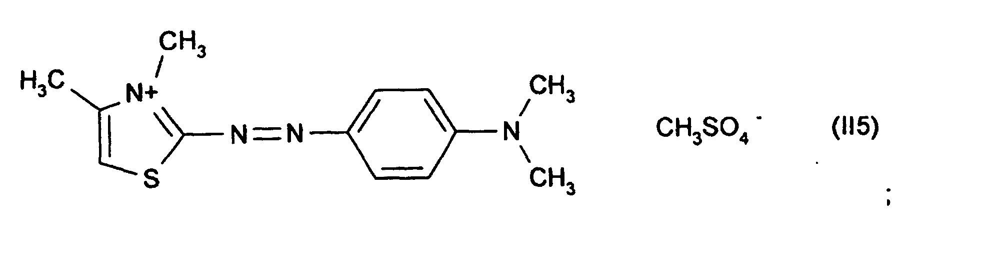 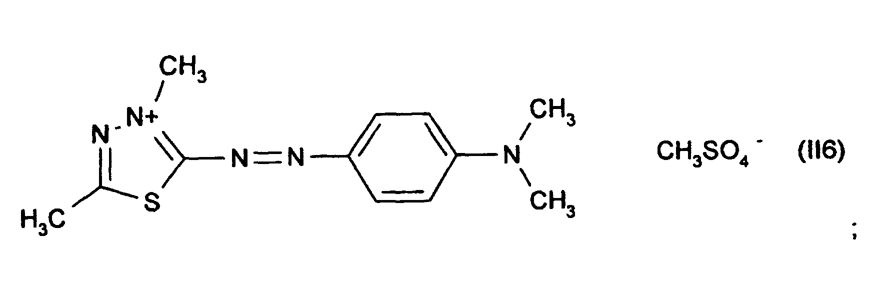 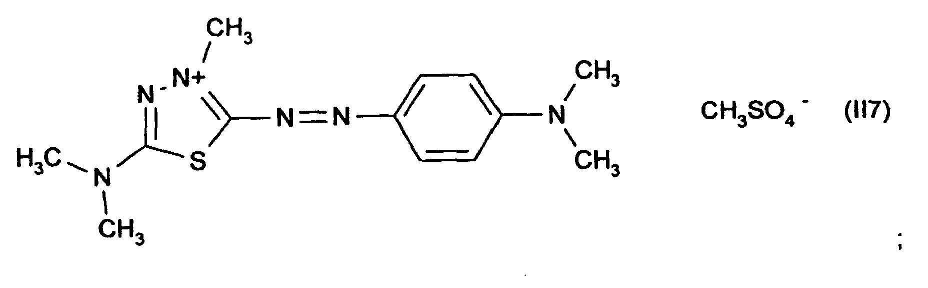 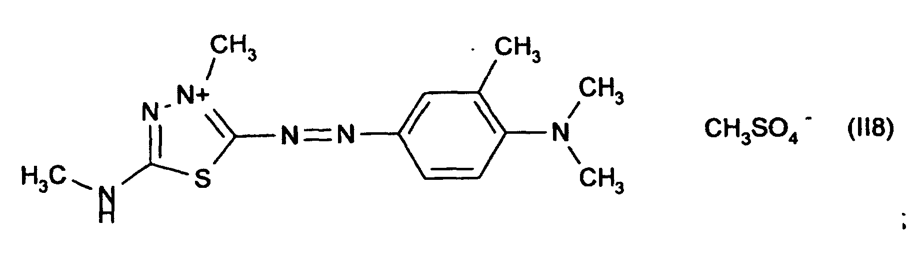 et

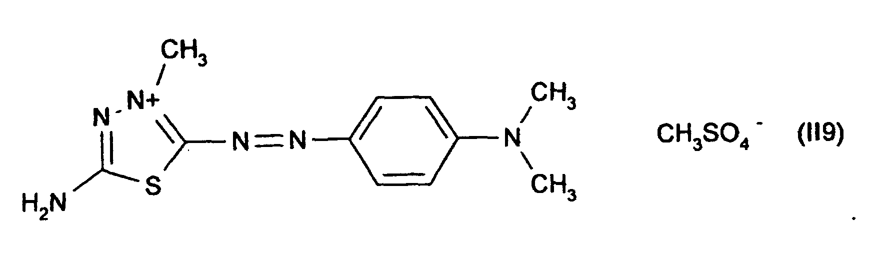

5. Composition selon les revendications 1 ou 2, **caractérisée par le fait que** les colorants directs cationiques de formule (III) sont choisis parmi les composés répondant aux structures (III1) à (III18) suivantes : 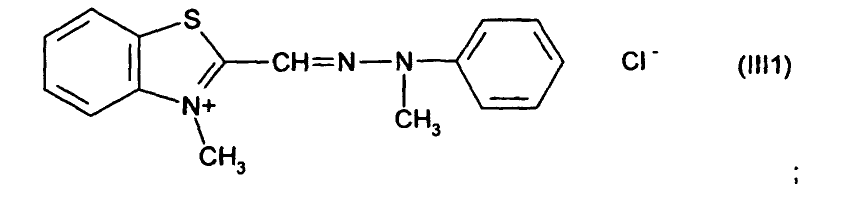 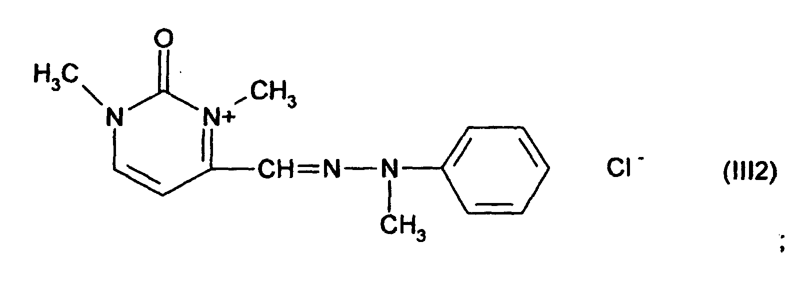 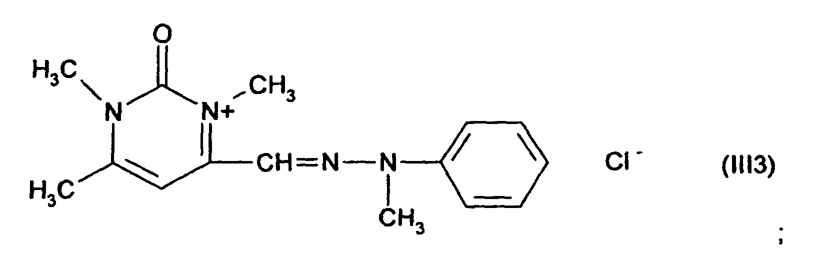 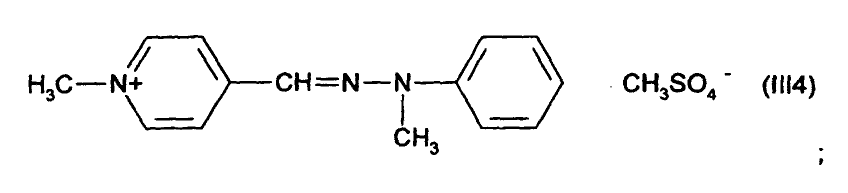 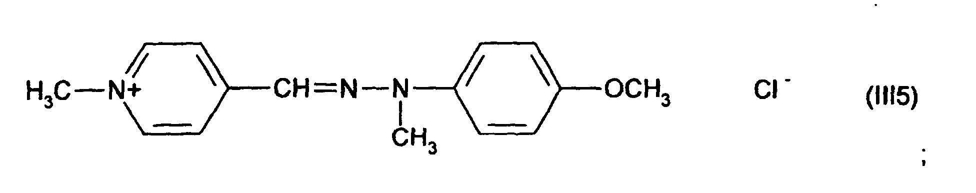 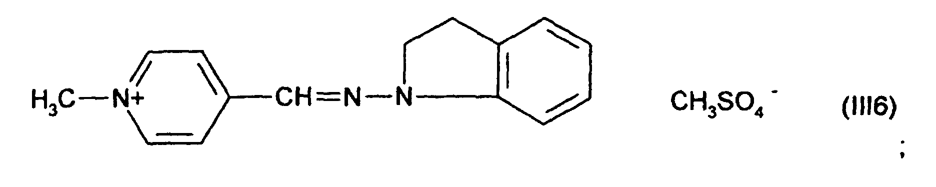 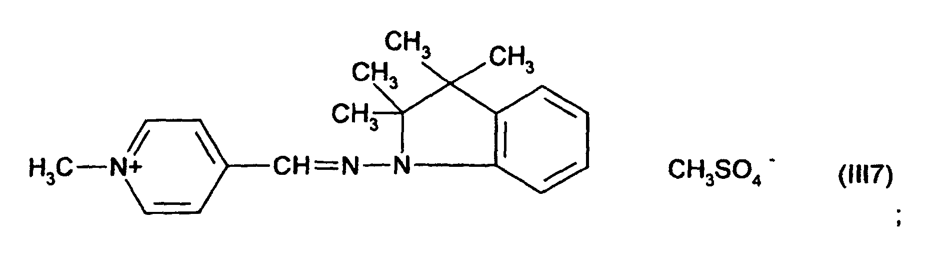 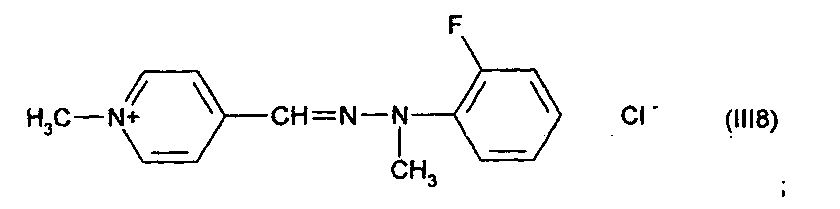 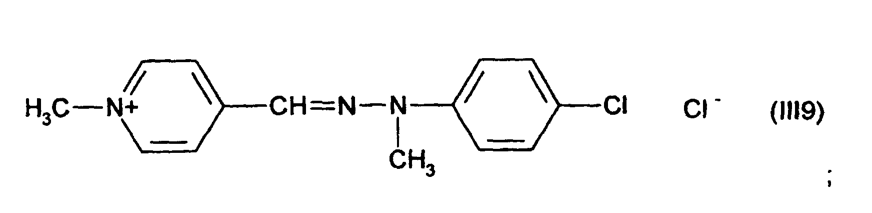 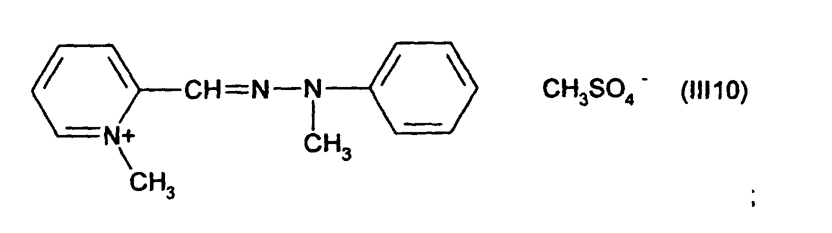 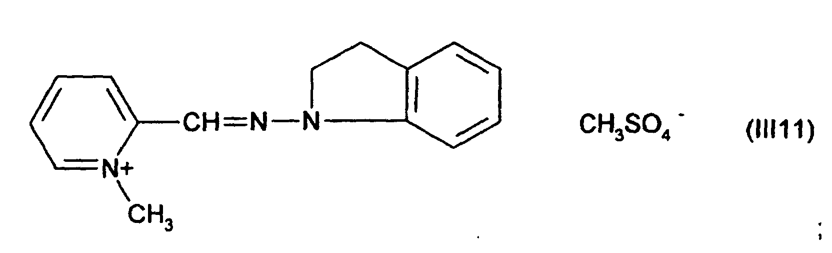 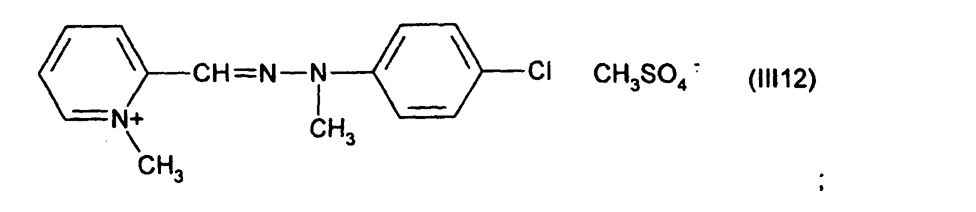 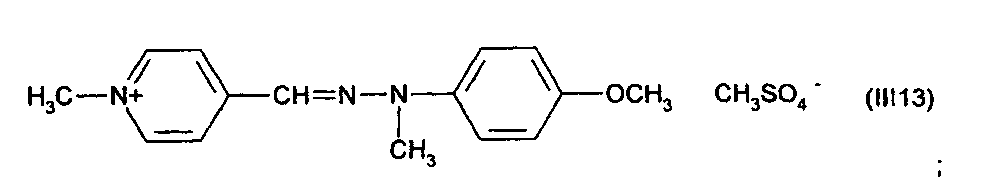 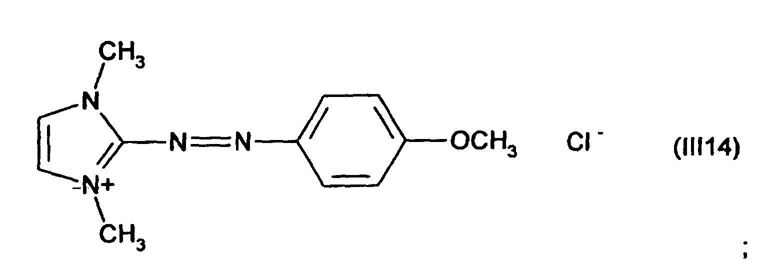 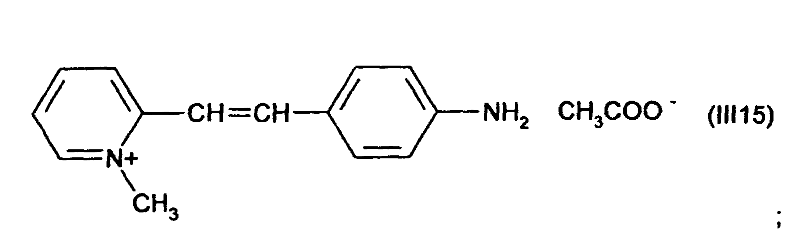 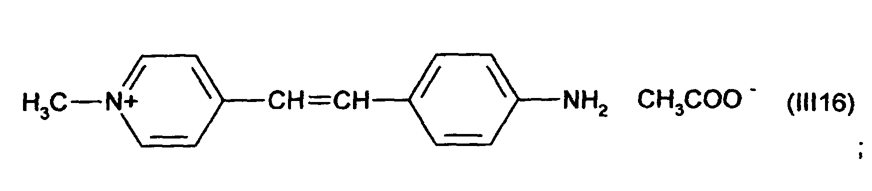 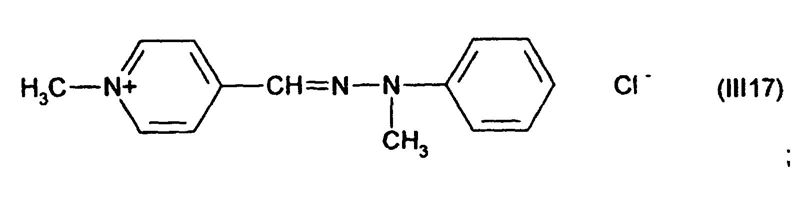 et

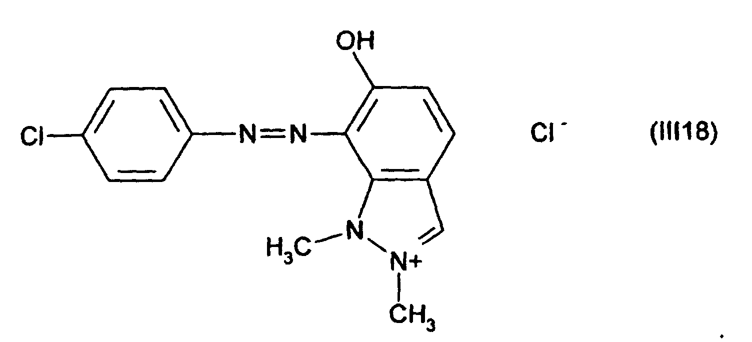

6. Composition selon la revendication 5, **caractérisée par le fait que** les colorants directs cationiques de formule (III) sont choisis parmi les composés répondant aux structures (III4), (III5) et (III13).

7. Composition selon les revendications 1 ou 2, **caractérisée par le fait que** les colorants directs cationiques de formule (III') sont choisis parmi les composés répondant aux structures (III'1) à (III'3) suivantes : 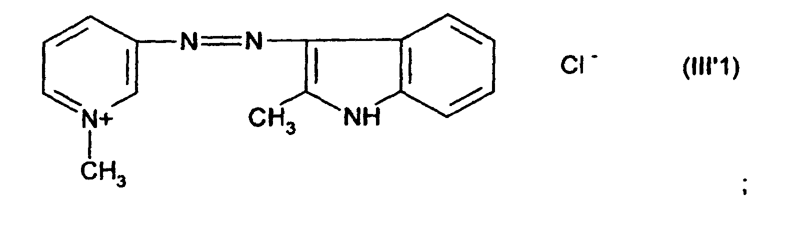 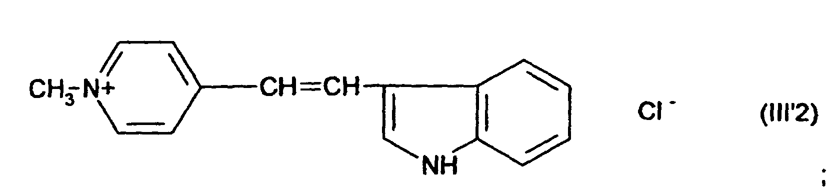 et

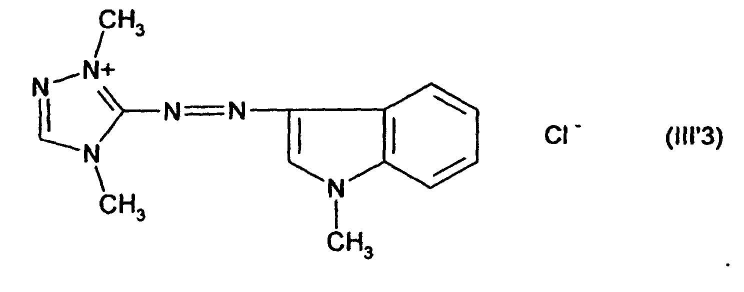

8. Composition selon l'une quelconque des revendications précédentes, **caractérisée par le fait que** le ou les colorants directs cationiques de formules (I), (II), (III), ou (III') représentent de 0,001 à 10 % en poids du poids total de la composition.

9. Composition selon la revendication 8, **caractérisée par le fait que** le ou les colorants directs cationiques de formules (I), (II), (III) ou (III') représentent de 0,005 à 5 % en poids du poids total de la composition.

10. Composition selon l'une quelconque des revendications précédentes, **caractérisée par le fait que** le polymère épaississant de type hydroxyalkylcellulose est une hydroxyéthylcellulose.

11. Composition selon l'une quelconque des revendications 1 à 9, **caractérisée par le fait que** le polymère épaississant de type hydroxyalkylcellulose est une hydroxypropylcellulose.

12. Composition selon l'une quelconque des revendications 1 à 9, **caractérisée par le fait que** le polymère épaississant est une gomme de guar non-ionique modifiée par des groupements hydroxyalkyle en C₁-C₆.

13. Composition selon la revendication 12, **caractérisée par le fait que** la gomme non-ionique présente un taux d'hydroxyalkylation variant entre 0,4 et 1,2.

14. Composition selon l'une quelconque des revendications 1 à 9, **caractérisée par le fait que** le polymère épaississant de type carboxyalkylcellulose est une carboxyéthylcellulose.

15. Composition selon l'une quelconque des revendications précédentes, **caractérisée par le fait que** le polymère épaississant (ii) représente de 0,01 à 10 % en poids du poids total de la composition.

16. Composition selon la revendication 15, **caractérisée par le fait que** le polymère épaississant (ii) représente de 0,1 à 5% en poids du poids total de la composition.

17. Composition selon l'une quelconque des revendications précédentes, **caractérisée par le fait que** le milieu approprié pour la teinture (ou support) est constitué par de l'eau ou par un mélange d'eau et d'au moins un solvant organique.

18. Composition selon l'une quelconque des revendications précédentes, **caractérisée par le fait qu'**elle présente un pH compris entre 2 et 11, et de préférence entre 5 et 10.

19. Composition selon l'une quelconque des revendications précédentes, **caractérisée par le fait qu'**elle est destinée à la teinture d'oxydation et qu'elle contient une ou plusieurs bases d'oxydation choisie parmi les paraphénylènediamines, les bis-phénylalkylènediamines, les para-aminophénols, les ortho-aminophénols et les bases hétérocycliques.

20. Composition selon la revendication 19, **caractérisée par le fait que** la ou les bases d'oxydation représentent 0,0005 à 12 % en poids du poids total de la composition tinctoriale.

21. Composition selon la revendication 20, **caractérisée par le fait que** la ou les bases d'oxydation représentent 0,005 à 6 % en poids du poids total de la composition tinctoriale.

22. Composition selon l'une quelconque des revendications 19 à 21, **caractérisée par le fait qu'**elle renferme un ou plusieurs coupleurs choisis parmi les métaphénylènediamines, les méta-aminophénols, les métadiphénols et les coupleurs hétérocycliques.

23. Composition selon la revendication 22, **caractérisée par le fait que** le ou les coupleurs représentent de 0,0001 à 10 % en poids du poids total de la composition tinctoriale.

24. Composition selon la revendication 23, **caractérisée par le fait que** le ou les coupleurs représentent de 0,005 à 5 % en poids du poids total de la composition tinctoriale.

25. Composition selon l'une quelconque des revendications précédentes, **caractérisée par le fait qu'**elle est destinée à la teinture d'oxydation ou la teinture directe éclaircissante et qu'elle renferme alors au moins un agent oxydant.

26. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 25, pendant un temps suffisant pour développer la coloration désirée, après quoi on rince, on lave éventuellement au shampooing, on rince à nouveau et on sèche.

27. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 25, pendant un temps suffisant pour développer la coloration désirée, sans rinçage final.

28. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A1) comprenant, dans un milieu approprié pour la teinture, au moins un colorant direct cationique (i) tel que défini dans les revendications précédentes et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant le polymère épaississant (ii) tel que défini dans les revendications précédentes.

29. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A2) comprenant, dans un milieu approprié pour la teinture, au moins un colorant direct cationique (i) tel que défini dans les revendications précédentes et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant le polymère épaississant (ii) tel que défini dans les revendications précédentes.

30. Dispositif à plusieurs compartiments ou "kit" de teinture à plusieurs compartiments, **caractérisé par le fait qu'**un premier compartiment renferme la composition (A1) ou (A2) telle que définie à la revendication 28 ou 29 et un second compartiment renferme la composition (B1) ou (B2) telle que définie à la revendication 28 ou 29.

## Claims

1. Composition for dyeing keratin fibres, and in particular human keratin fibres such as the hair, containing, in a medium which is suitable for dyeing, (i) at least one compound chosen from those of formulae (I), (II), (III) and (III') below:
a) the compounds of formula (I) below: 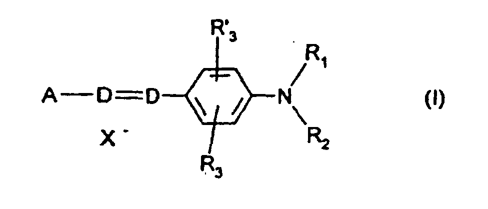 in which:
D represents a nitrogen atom or a -CH group,
R₁ and R₂, which may be identical or different, represent a hydrogen atom; a C₁-C₄ alkyl radical which can be substituted with a -CN, -OH or -NH₂ radical or form, with a carbon atom of the benzene ring, a heterocycle optionally containing oxygen or nitrogen, which can be substituted with one or more C₁-C₄ alkyl radicals; a 4'-aminophenyl radical,
R₃ and R'₃, which may be identical or different, represent a hydrogen atom, a halogen atom chosen from chlorine, bromine, iodine and fluorine, a cyano radical, or a C₁-C₄ alkyl, C₁-C₄ alkoxy or acetyloxy radical,
X⁻ represents an anion preferably chosen from chloride, methyl sulphate and acetate,
A represents a group chosen from the structures A1 to A18 below: 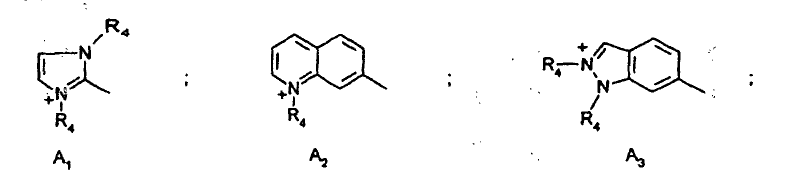 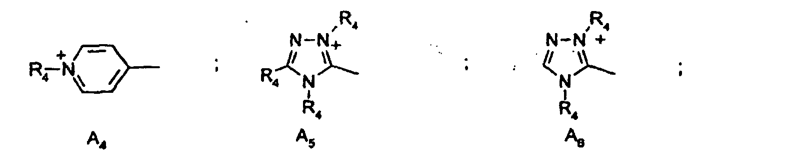 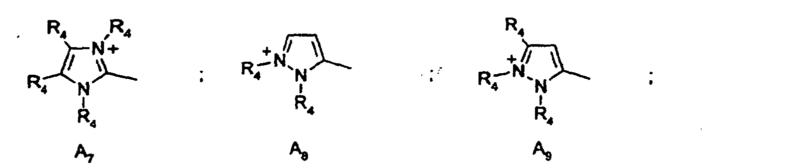 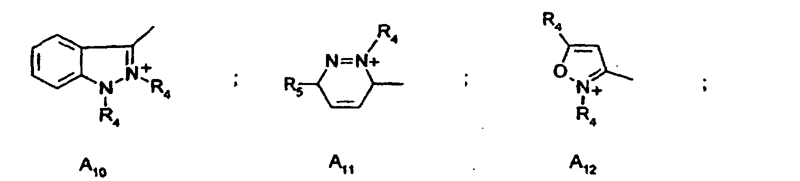 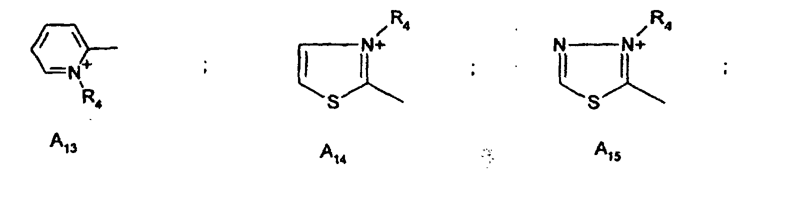 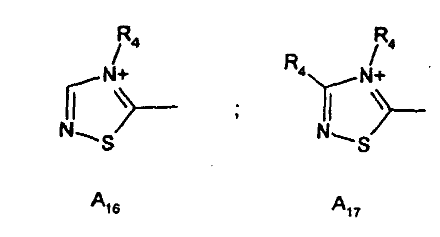 and 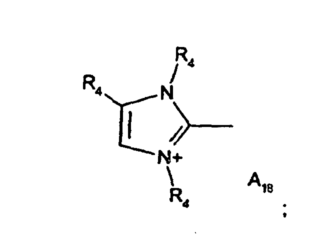 in which R₄ represents a C₁-C₄ alkyl radical which can be substituted with a hydroxyl radical and R₅ represents a C₁-C₄ alkoxy radical, with the proviso that when D represents -CH, when A represents A₄ or A₁₃ and when R₃ is other than an alkoxy radical, then R₁ and R₂ do not simultaneously denote a hydrogen atom;
**b) the compounds of formula (II) below:** 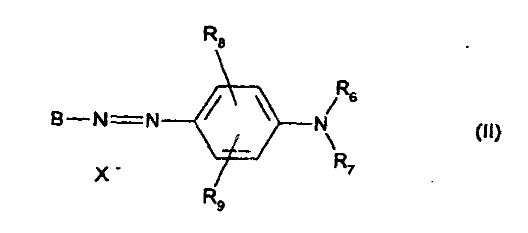 in which:
R₆ represents a hydrogen atom or a C₁-C₄ alkyl radical,
R₇ represents a hydrogen atom, an alkyl radical which can be substituted with a -CN radical or with an amino group, a 4'-aminophenyl radical or forms with R₆ a heterocycle optionally containing oxygen and/or nitrogen, which can be substituted with a C₁-C₄ alkyl radical,
R₈ and R₉, which may be identical or different, represent a hydrogen atom, a halogen atom such as bromine, chlorine, iodine or fluorine, a C₁-C₄ alkyl or C₁-C₄ alkoxy radical or a -CN radical,
X⁻ represents an anion preferably chosen from chloride, methyl sulphate and acetate,
B represents a group chosen from the structures B1 to B6 below 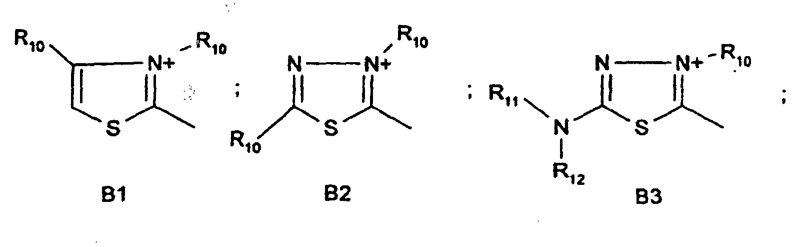 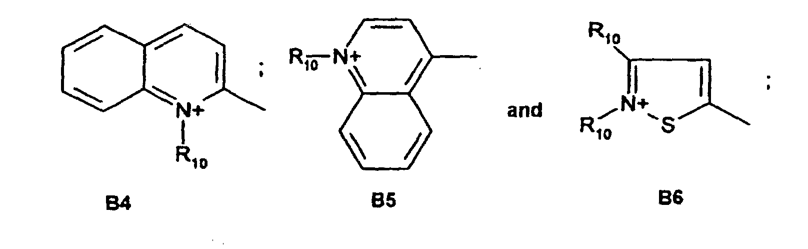 in which R₁₀ represents a C₁-C₄ alkyl radical, R₁₁ and R₁₂, which may be identical or different, represent a hydrogen atom or a C₁-C₄ alkyl radical;
**c) the compounds of formulae (III) and (III') below:** 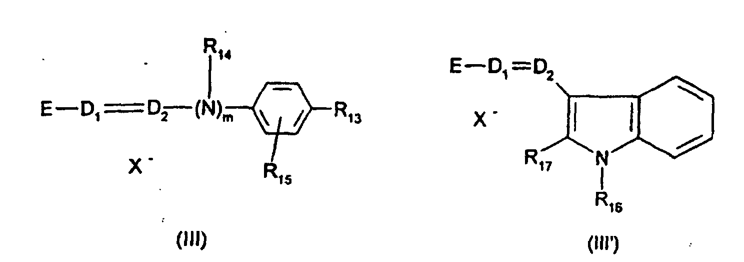 in which:
R₁₃ represents a hydrogen atom, a C₁-C₄ alkoxy radical, a halogen atom such as bromine, chlorine, iodine or fluorine, or an amino radical,
R₁₄ represents a hydrogen atom, a C₁-C₄ alkyl radical or forms, with a carbon atom of the benzene ring, a heterocycle optionally containing oxygen and/or substituted with one or more C₁-C₄ alkyl groups,
R₁₅ represents a hydrogen atom or a halogen atom such as bromine, chlorine, iodine or fluorine,
R₁₆ and R₁₇, which may be identical or different, represent a hydrogen atom or a C₁-C₄ alkyl radical,
D₁ and D₂, which may be identical or different, represent a nitrogen atom or a -CH group,
m = 0 or 1,
it being understood that when R₁₃ represents an unsubstituted amino group, then D₁ and D₂ simultaneously represent a -CH group and m = 0,
X⁻ represents an anion preferably chosen from chloride, methyl sulphate and acetate,
E represents a group chosen from the structures E1 to E8 below: 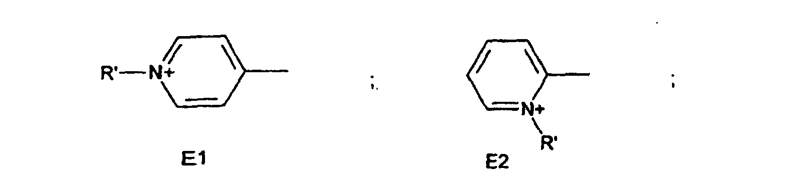 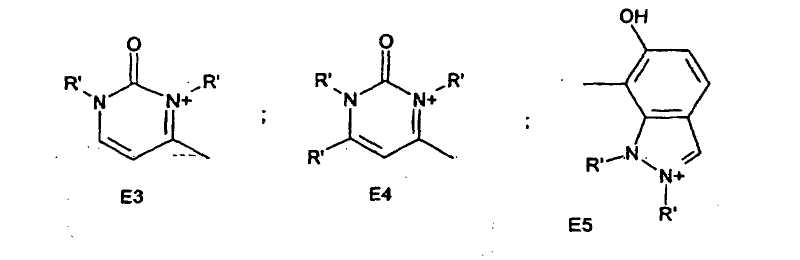 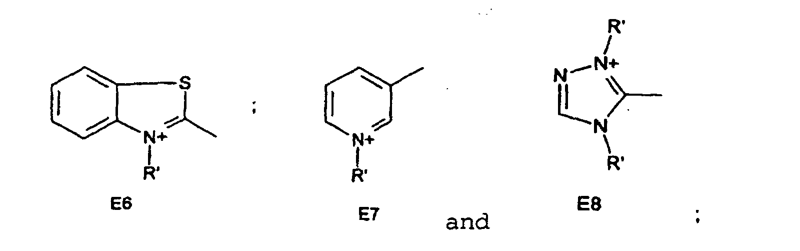 in which R' represents a C₁-C₄ alkyl radical;
when m = 0 and when D₁ represents a nitrogen atom, then E can also denote a group of structure E9 below: 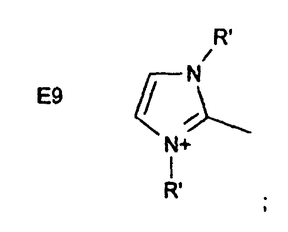 in which R' represents a C₁-C₄ alkyl radical;
the said composition being **characterized in that** it also contains
**(ii)** at least one thickening polymer comprising at least one sugar unit chosen from the group comprising:
**(ii)**_{**1**} - nonionic guar gums;
**(ii)**_{**2**} - biopolysaccharide gums of microbial origin, such as sclero glucan or xanthan gums;
**(ii)**_{**3**} - gums derived from plant exudates, such as gum arabic, ghatti gum, karaya gum, gum tragacanth, carrageenan gum, agar gum and carob gum;
**(ii)**_{**4**} - pectins;
**(ii)**_{**5**} - alginates;
**(ii)**_{**6**} - starches;
**(ii)**_{**7**} - hydroxyalkylcelluloses and
carboxyalkylcelluloses.

2. Composition according to Claim 1, **characterized in that** the cationic direct dyes of formula (I) are chosen from the compounds corresponding to the structures (I1) to (I54) below:

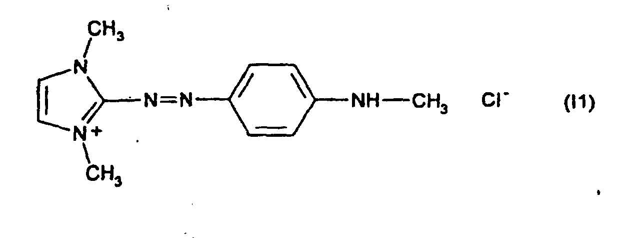

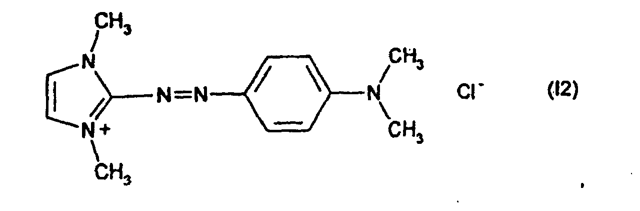

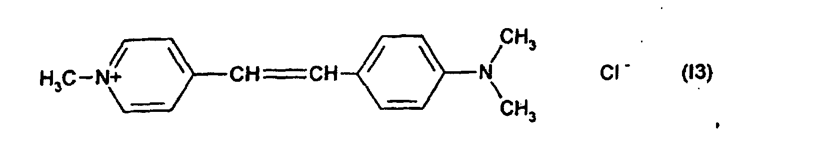

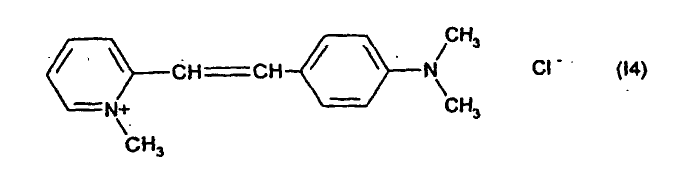

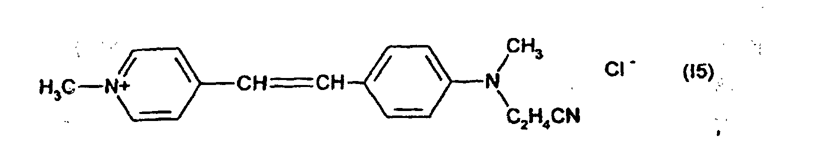

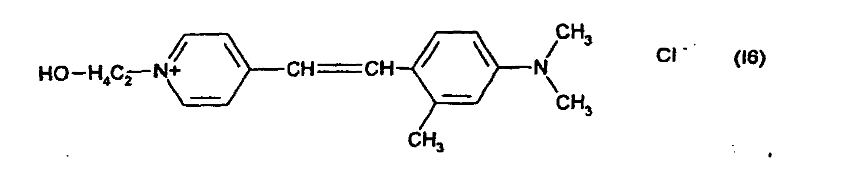

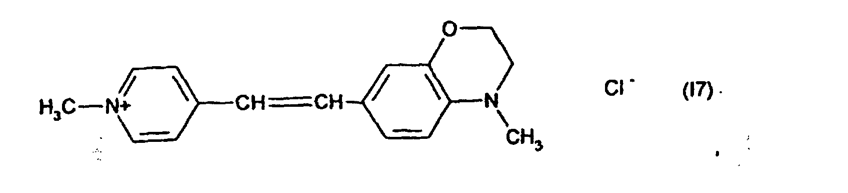

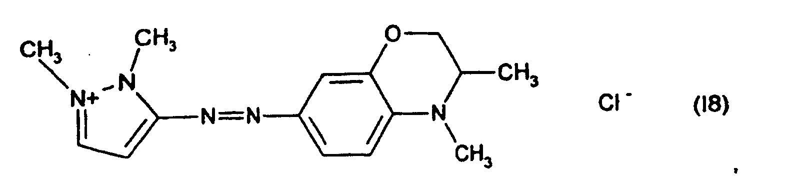

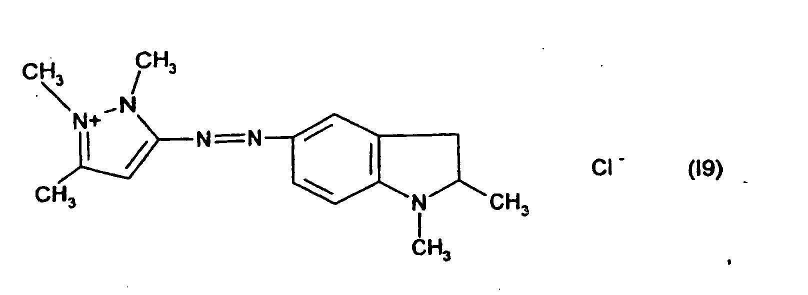

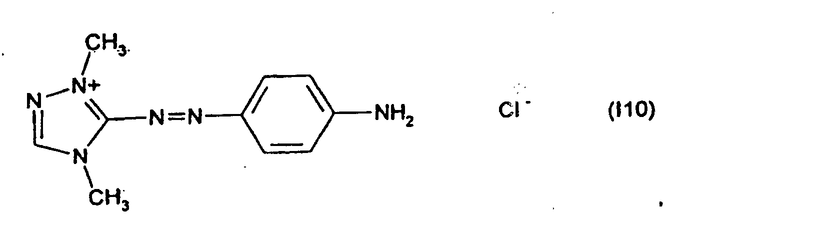

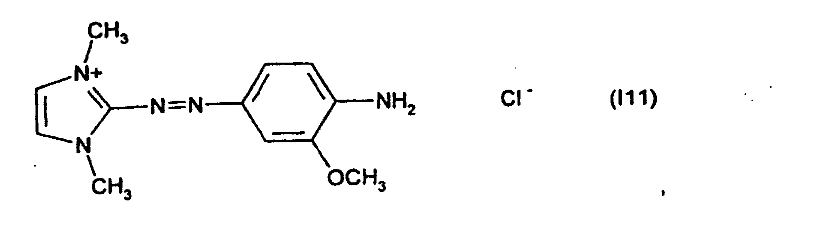

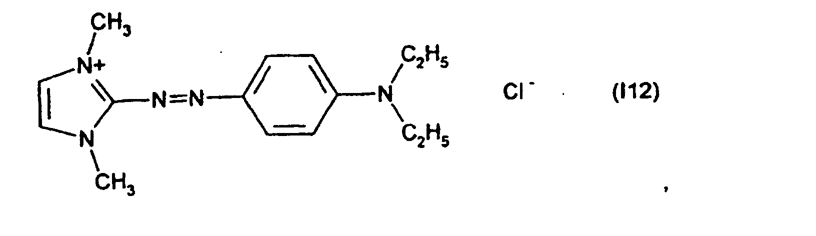

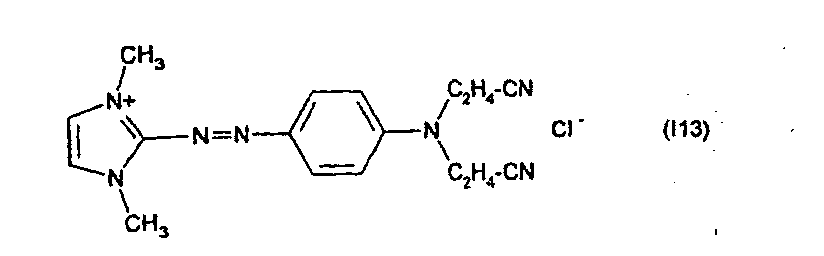

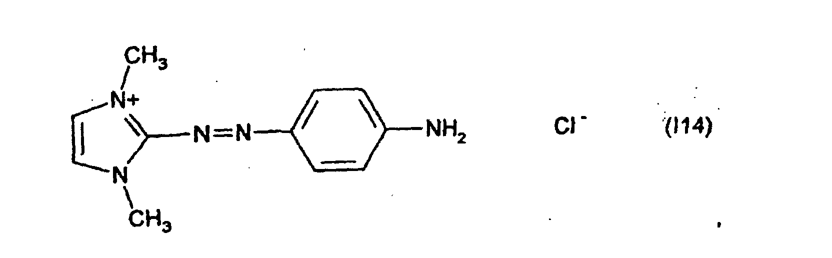

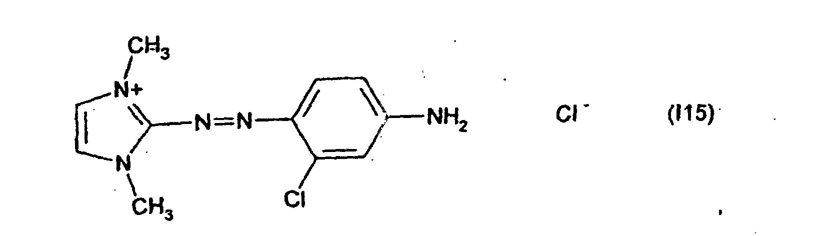

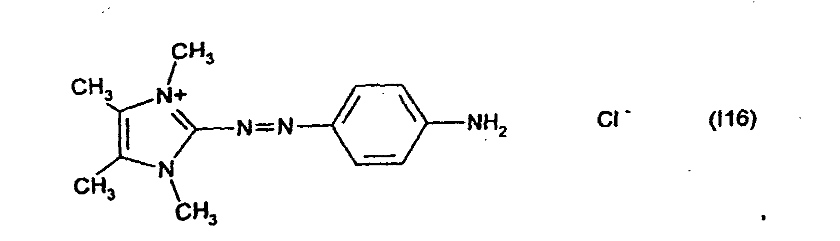

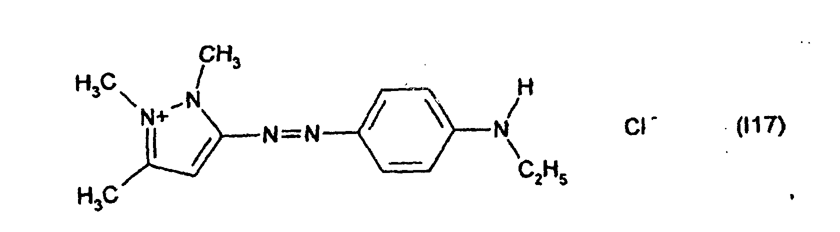

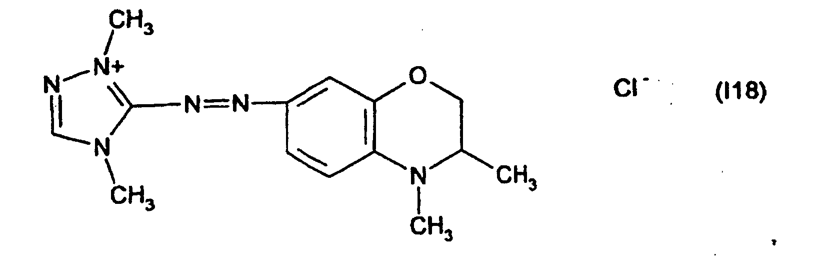

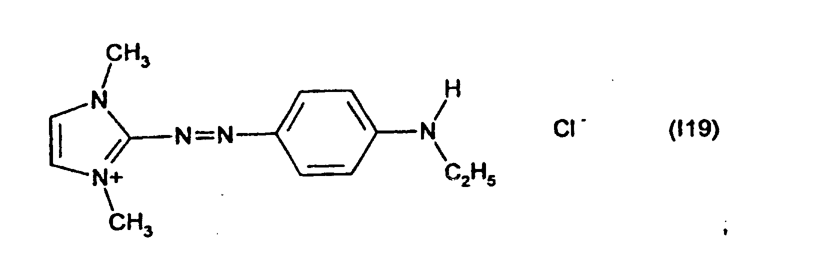

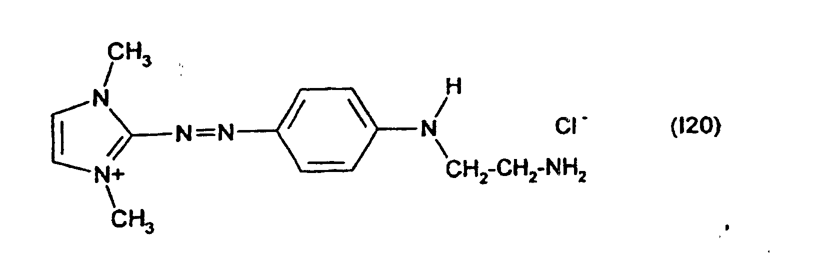

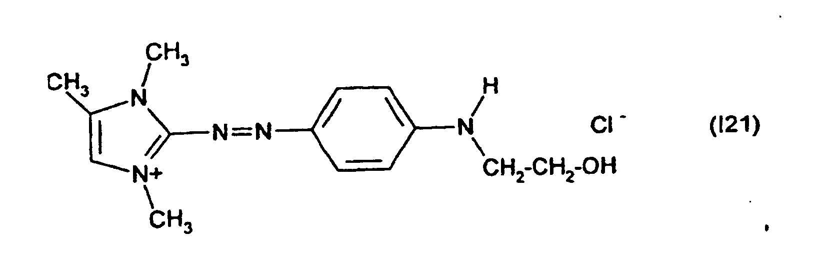

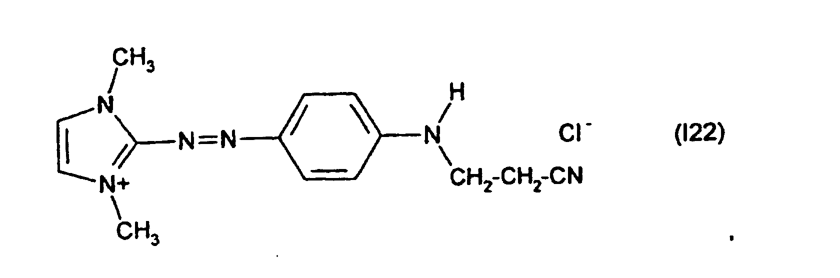

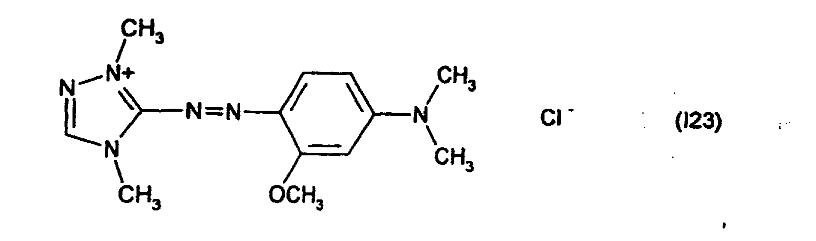

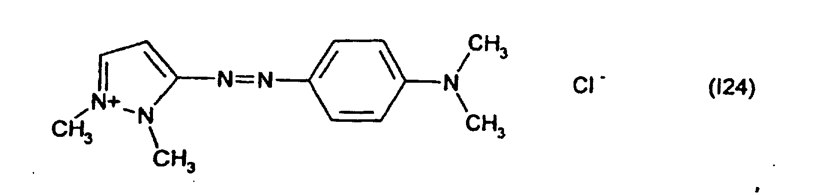

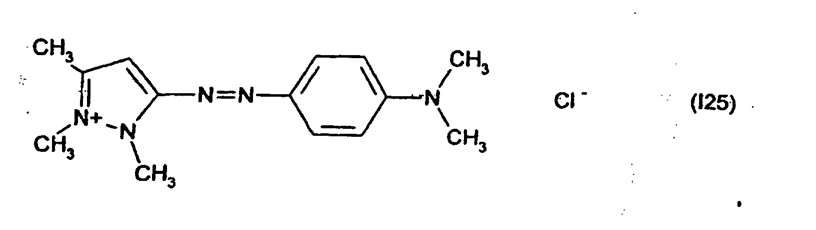

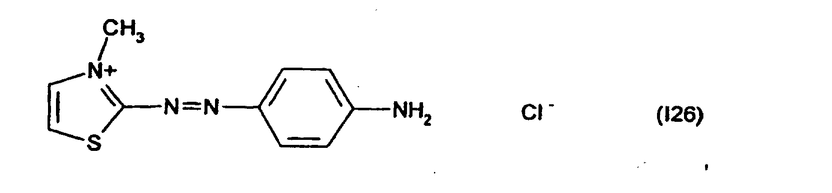

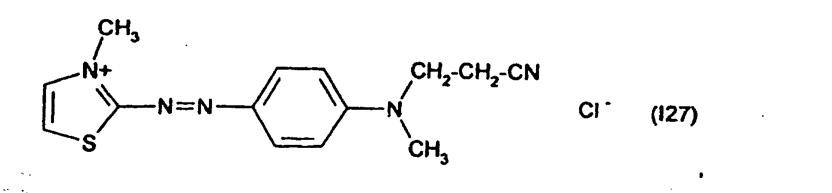

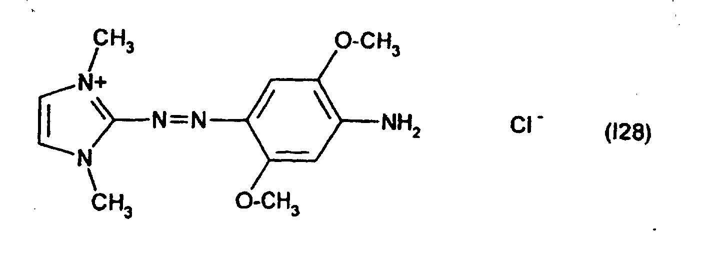

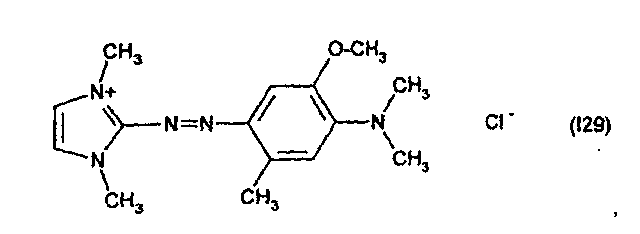

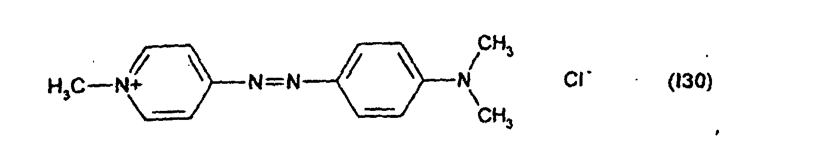

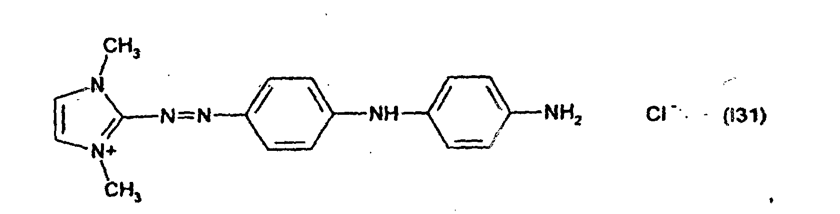

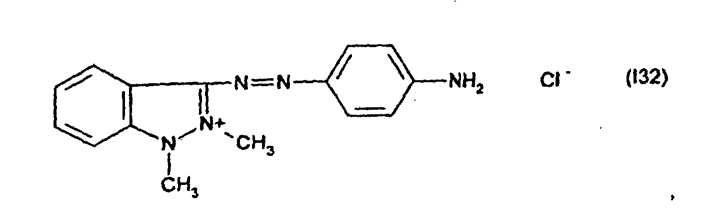

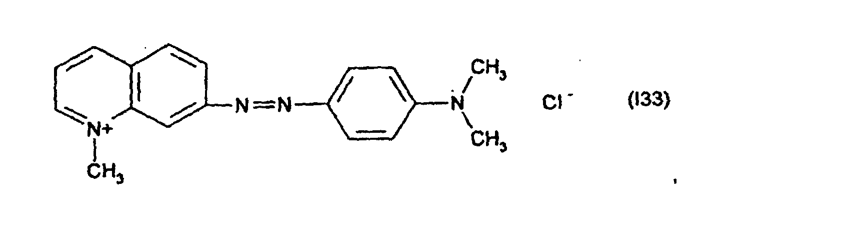

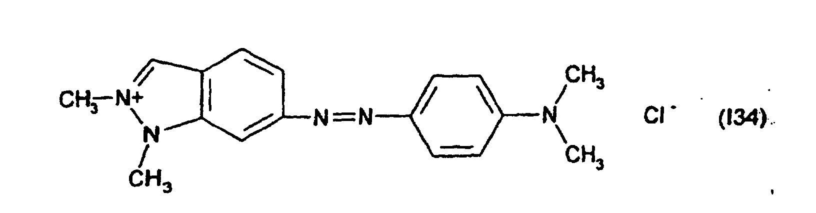

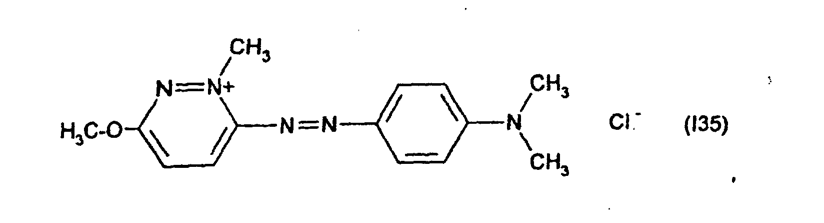

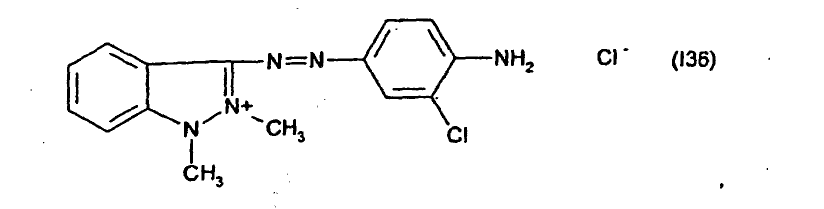

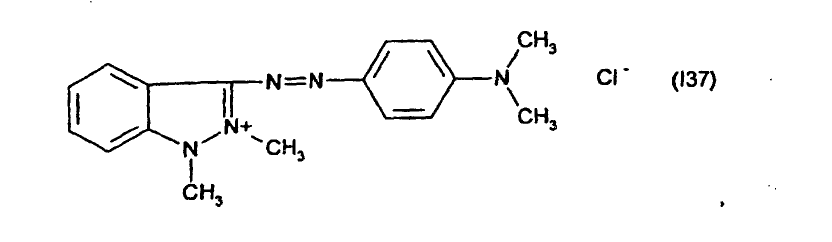

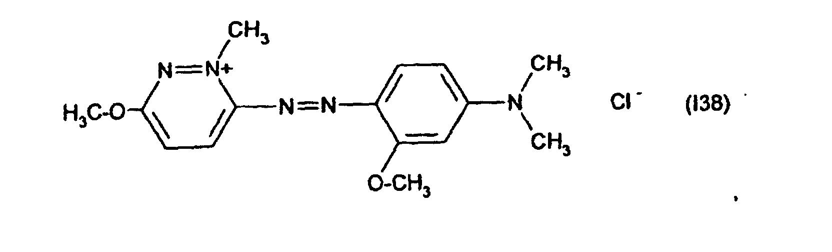

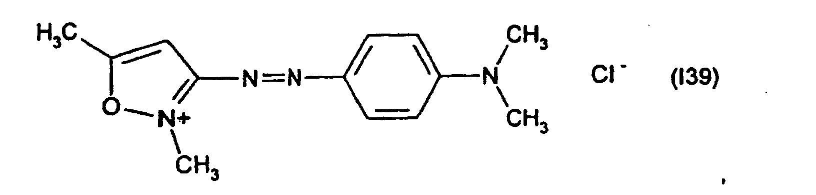

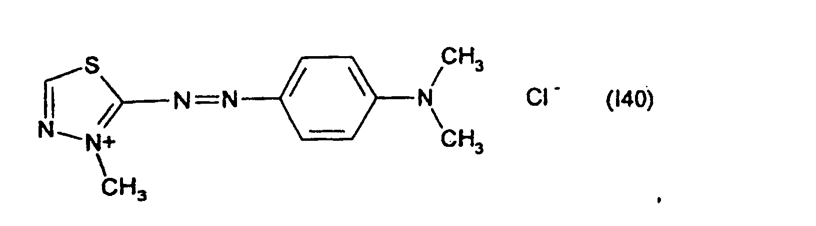

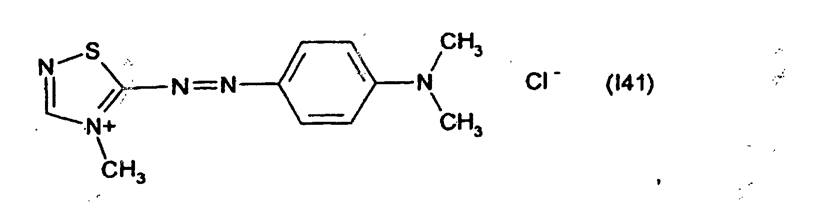

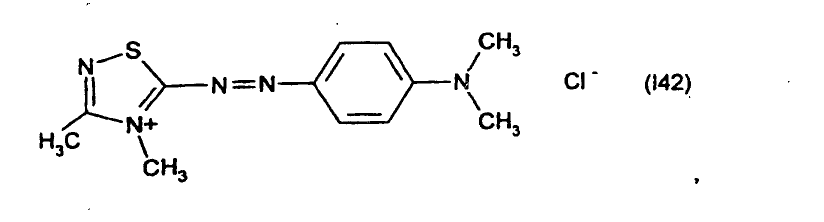

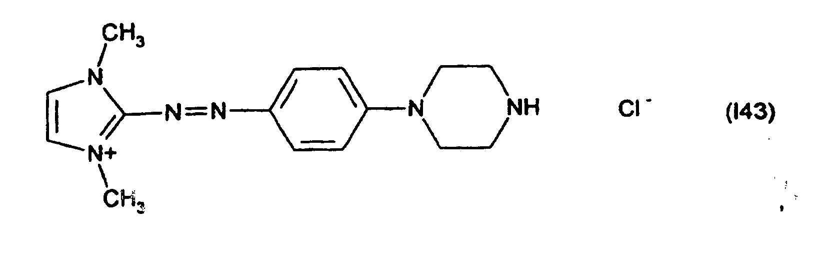

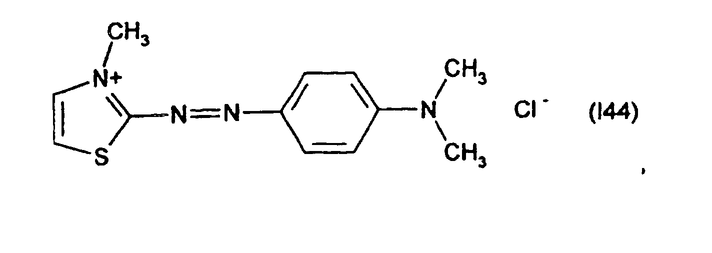

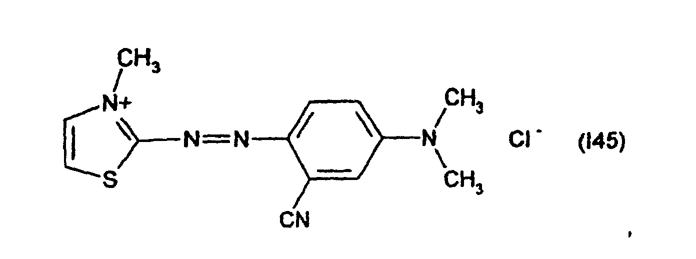

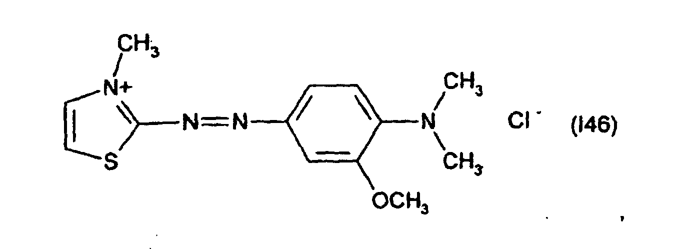

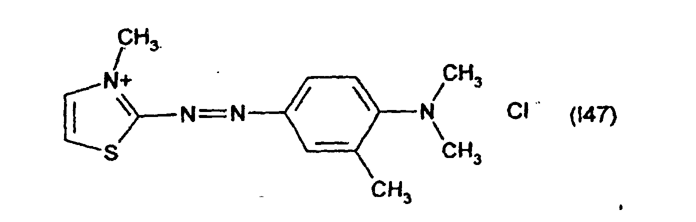

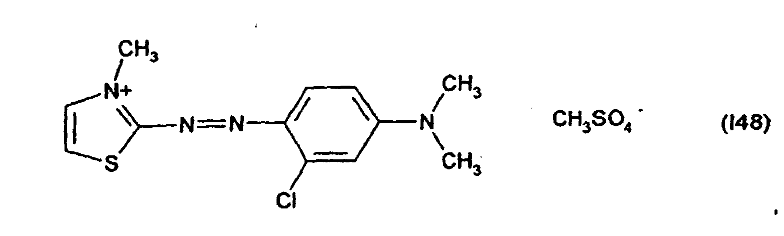

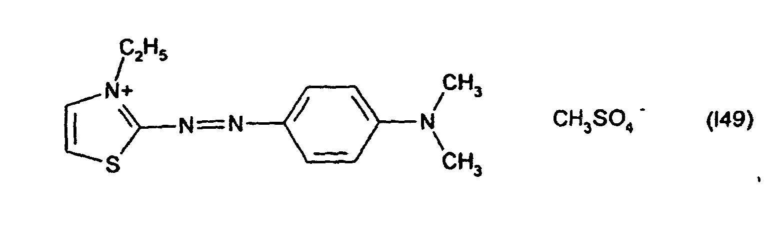

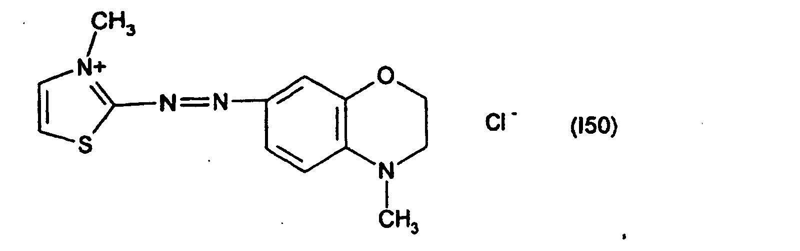

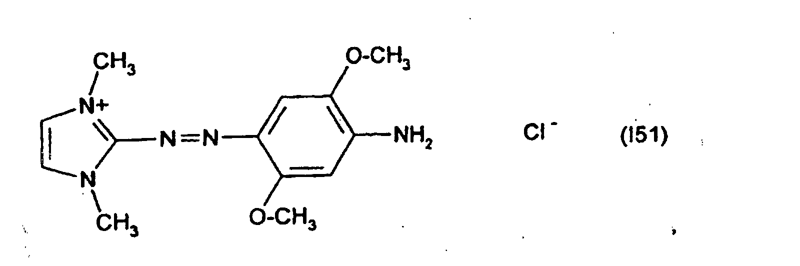

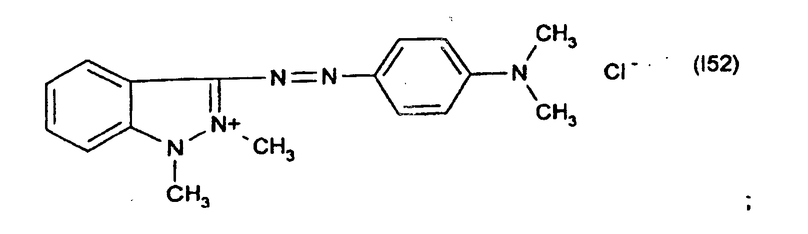

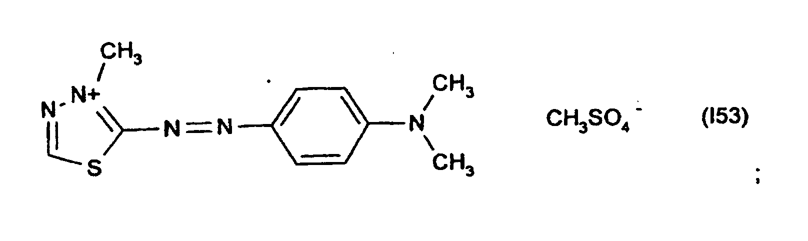

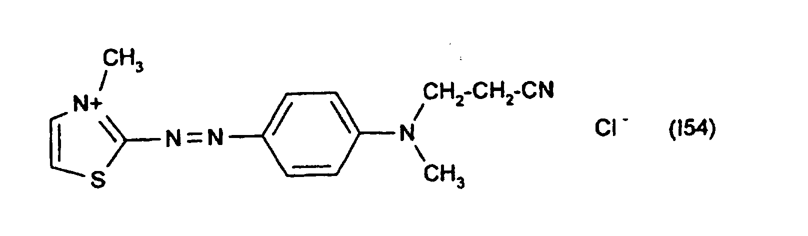

3. Composition according to Claim 2, **characterized in that** the cationic direct dyes correspond to the structures (I1), (I2), (I14) and (I31).

4. Composition according to Claim 1 or 2, **characterized in that** the cationic direct dyes of formula (II) are chosen from the compounds corresponding to the structures (II1) to (II9) below: 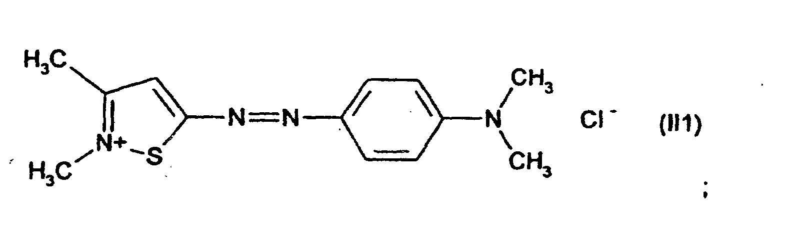 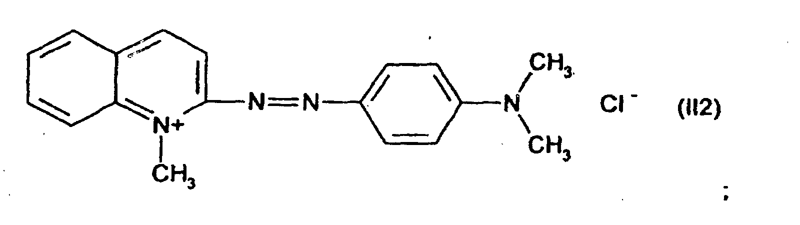 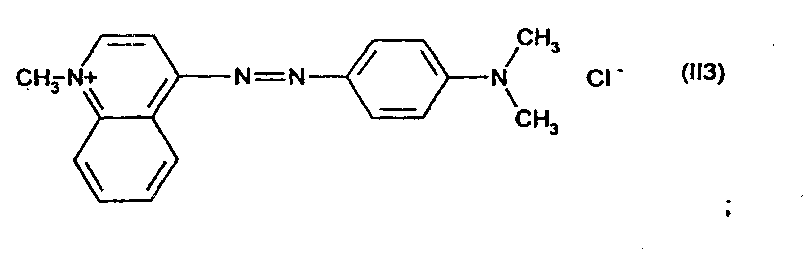 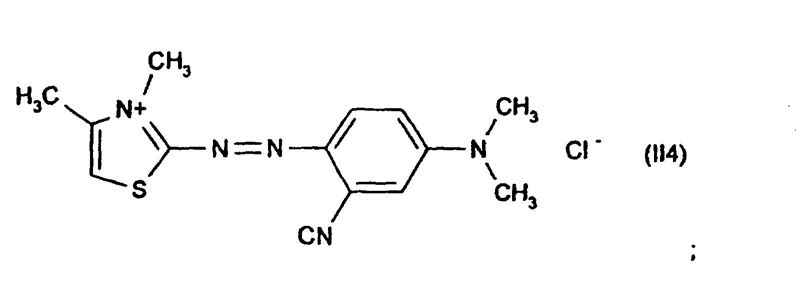 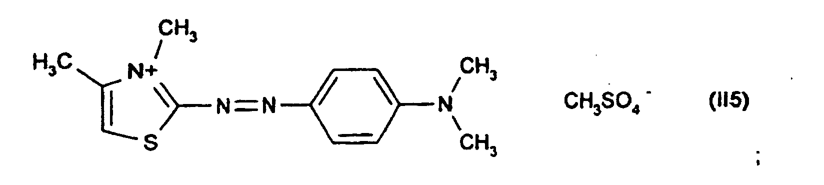 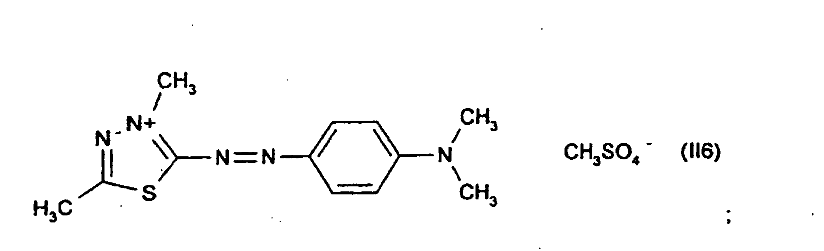 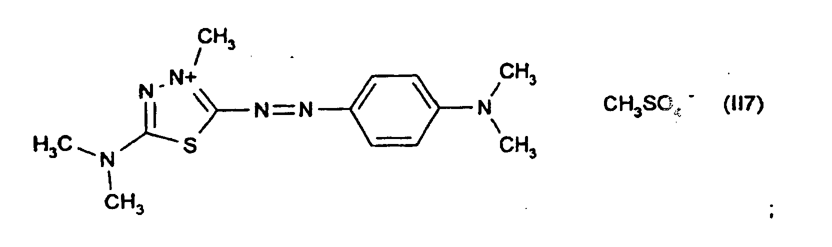 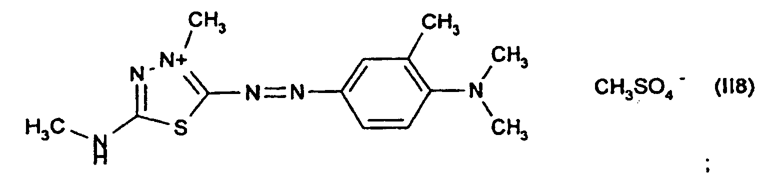 and

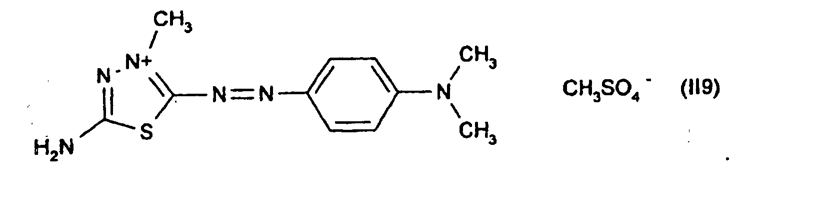

5. Composition according to Claim 1 or 2, **characterized in that** the cationic direct dyes of formula (III) are chosen from the compounds corresponding to the structures (III1) to (III18) below: 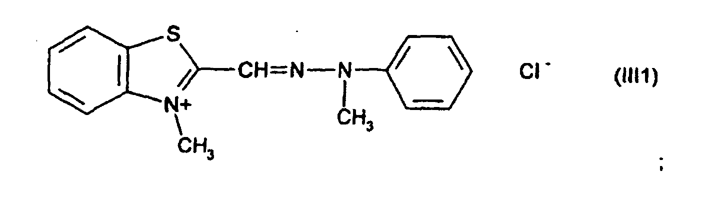 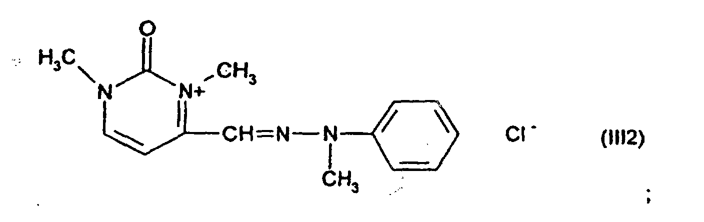 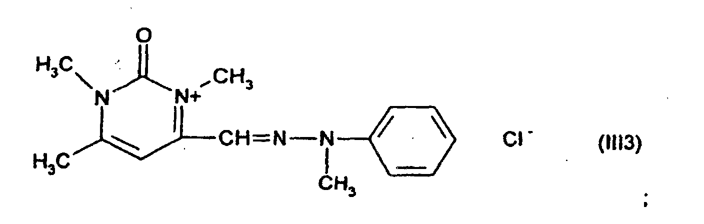 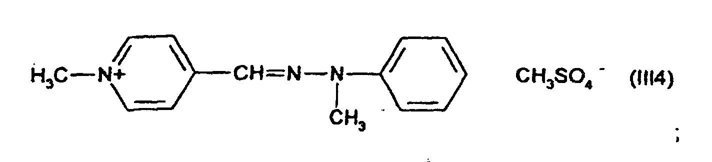 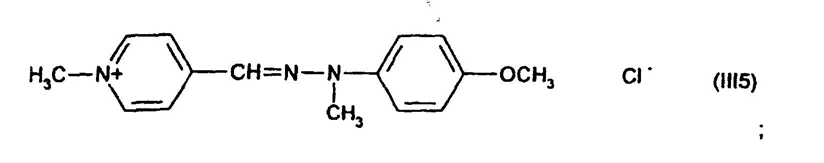 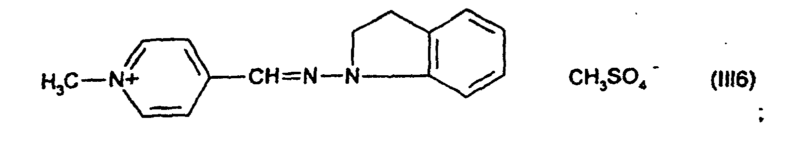 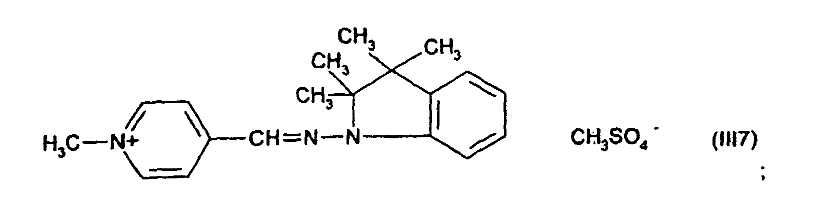 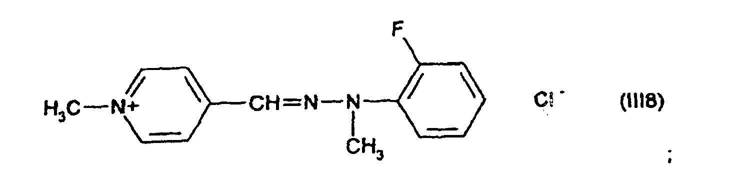 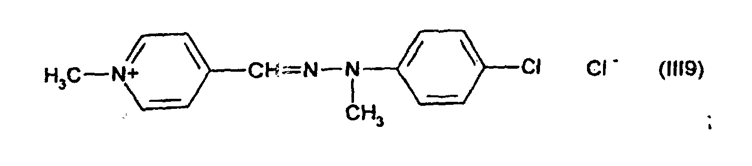 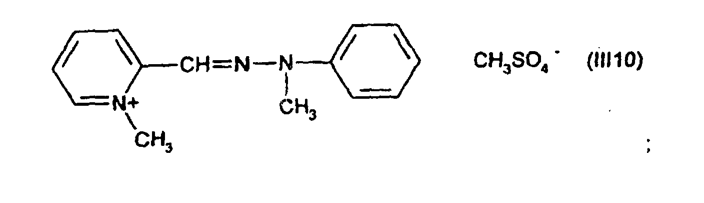 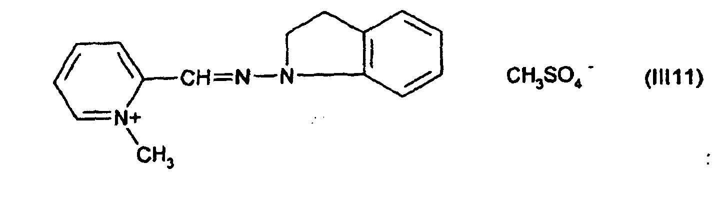 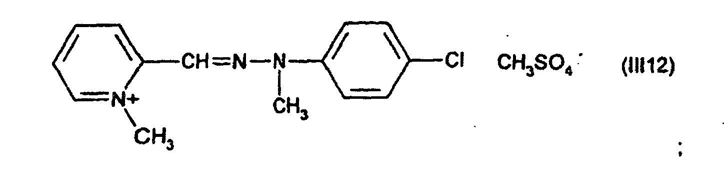 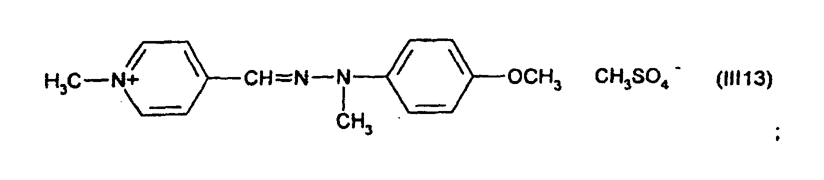 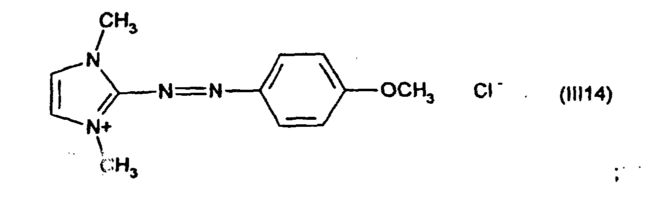 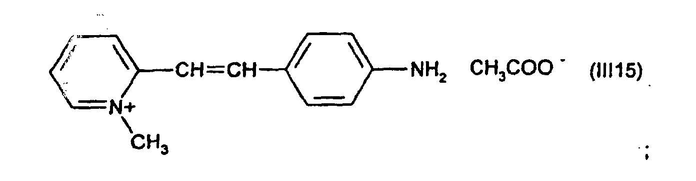 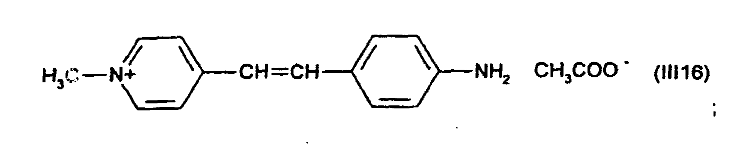 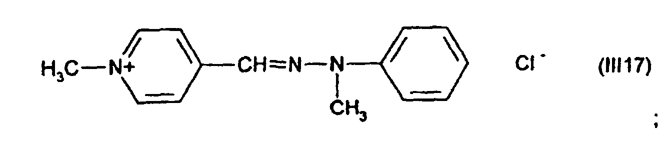 ; and

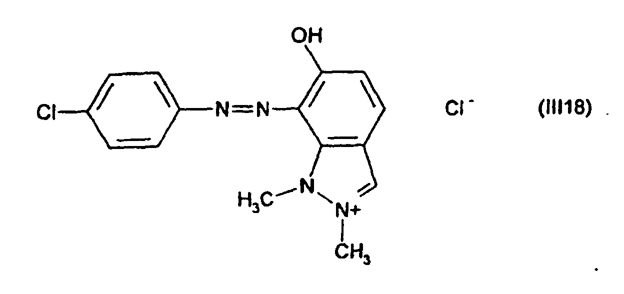

6. Composition according to Claim 5, **characterized in that** the cationic direct dyes of formula (III) are chosen from the compounds corresponding to the structures (III4), (III5) and (III13).

7. Composition according to Claim 1 or 2, **characterized in that** the cationic direct dyes of formula (III') are chosen from the compounds corresponding to the structures (III'1) to (III'3) below: 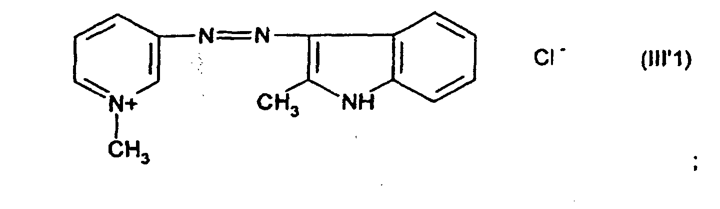 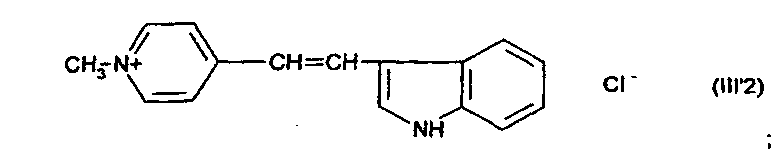 and

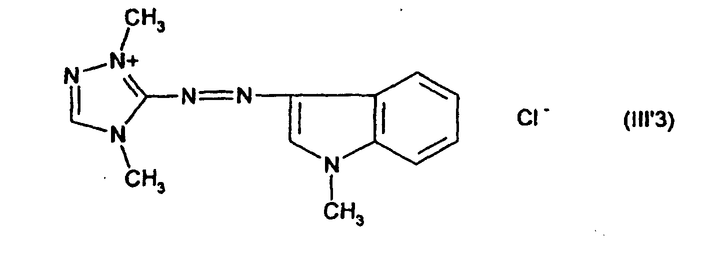

8. Composition according to any one of the preceding claims, **characterized in that** the cationic direct dye(s) of formula (I), (II), (III) or (III') represent(s) from 0.001 to 10% by weight relative to the total weight of the composition.

9. Composition according to Claim 8, **characterized in that** the cationic direct dye(s) of formula (I), (II), (III) or (III') represent(s) from 0.005 to 5% by weight relative to the total weight of the composition.

10. Composition according to any one of the preceding claims, **characterized in that** the thickening polymer of hydroxyalkylcellulose type is a hydroxyethylcellulose.

11. Composition according to any one of Claims 1 to 9, **characterized in that** the thickening polymer of hydroxyalkylcellulose type is a hydroxypropylcellulose.

12. Composition according to any one of Claims 1 to 9, **characterized in that** the thickening polymer is a nonionic guar gum modified with C₁-C₆ hydroxyalkyl groups.

13. Composition according to Claim 12, **characterized in that** the nonionic gum has a degree of hydroxyalkylation ranging between 0.4 and 1.2.

14. Composition according to any one of Claims 1 to 9, **characterized in that** the thickening polymer of carboxyalkylcellulose type is a carboxyethylcellulose.

15. Composition according to any one of the preceding claims, **characterized in that** the thickening polymer (ii) represents from 0.01 to 10% by weight relative to the total weight of the composition.

16. Composition according to Claim 15, **characterized in that** the thickening polymer (ii) represents from 0.1 to 5% by weight relative to the total weight of the composition.

17. Composition according to any one of the preceding claims, **characterized in that** the medium which is suitable for dyeing (or support) consists of water or of a mixture of water and at least one organic solvent.

18. Composition according to any one of the preceding claims, **characterized in that** it has a pH of between 2 and 11 and preferably between 5 and 10.

19. Composition according to any one of the preceding claims, **characterized in that** it is intended for oxidation dyeing and **in that** it contains one or more oxidation bases chosen from paraphenylenediamines, bis(phenyl)alkylenediamines, para-aminophenols, ortho-aminophenols and heterocyclic bases.

20. Composition according to Claim 19, **characterized in that** the oxidation base(s) represent(s) from 0.0005 to 12% by weight relative to the total weight of the dye composition.

21. Composition according to Claim 20, **characterized in that** the oxidation base(s) represent(s) from 0.005 to 6% by weight relative to the total weight of the dye composition.

22. Composition according to any one of Claims 19 to 21, **characterized in that** it contains one or more couplers chosen from meta-phenylenediamines, meta-aminophenols, meta-diphenols and heterocyclic couplers.

23. Composition according to Claim 22, **characterized in that** the coupler(s) represent(s) from 0.0001 to 10% by weight relative to the total weight of the dye composition.

24. Composition according to Claim 23, **characterized in that** the coupler(s) represent(s) from 0.005 to 5% by weight relative to the total weight of the dye composition.

25. Composition according to any one of the preceding claims, **characterized in that** it is intended for oxidation dyeing or lightening direct dyeing and **in that** it contains at least one oxidizing agent.

26. Process for dyeing keratin fibres, and in particular human keratin fibres such as the hair, **characterized in that** at least one dye composition as defined in any one of Claims 1 to 25 is applied to the fibres, for a period which is sufficient to develop the desired coloration, after which the fibres are rinsed, optionally washed with shampoo, rinsed again and dried.

27. Process for dyeing keratin fibres, and in particular human keratin fibres such as the hair, **characterized in that** at least one dye composition as defined in any one of Claims 1 to 25 is applied to the fibres, for a period which is sufficient to develop the desired coloration, without final rinsing.

28. Process for dyeing keratin fibres, and in particular human keratin fibres such as the hair, **characterized in that** it comprises a first step which consists in separately storing, on the one hand, a composition (A1) comprising, in a medium which is suitable for dyeing, at least one cationic direct dye (i) as defined in the preceding claims and at least one oxidation base, and, on the other hand, a composition (B1) comprising, in a medium which is suitable for dyeing, at least one oxidizing agent, and then in mixing them together at the time of use, after which this mixture is applied to the keratin fibres, the composition (A1) or the composition (B1) containing the thickening polymer (ii) as defined in the preceding claims.

29. Process for dyeing keratin fibres, and in particular human keratin fibres such as the hair, **characterized in that** it comprises a first step which consists in separately storing, on the one hand, a composition (A2) comprising, in a medium which is suitable for dyeing, at least one cationic direct dye (i) as defined in the preceding claims and, on the other hand, a composition (B2) comprising, in a medium which is suitable for dyeing, at least one oxidizing agent, and then in mixing them together at the time of use, after which this mixture is applied to the keratin fibres, the composition (A2) or the composition (B2) containing the thickening polymer (ii) as defined in the preceding claims.

30. Multi-compartment dyeing device or multicompartment dyeing "kit", **characterized in that** a first compartment comprises the composition (A1) or (A2) as defined in Claim 28 or 29 and a second compartment comprises the composition (B1) or (B2) as defined in Claim 28 or 29.

## Patentansprüche

1. Zusammensetzung zum Färben von Keratinfasern und insbesondere menschlichen Keratinfasern, wie dem Haar, die in einem zum Färben geeigneten Medium enthält: (i) mindestens eine Verbindung, die unter den Verbindungen der folgenden Formeln (I), (II), (III) und (III') ausgewählt ist:
(a) Verbindungen der folgenden Formel (I): 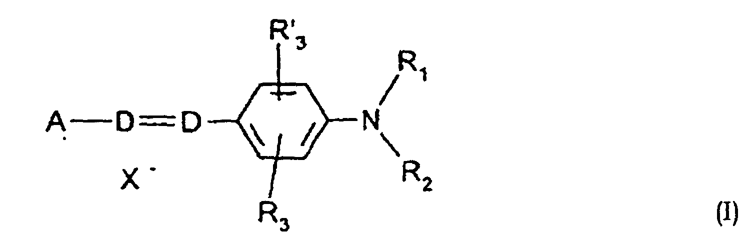 worin bedeuten:
D ein Stickstoffatom oder die Gruppe -CH,
R₁ und R₂, die gleich oder verschieden sind, ein Wasserstoffatom;
eine C₁₋₄-Alkylgruppe, die mit einer Gruppe -CN, -OH oder -NH₂ substituiert sein kann, oder sie bilden mit einem Kohlenstoffatom des Benzolrings einen gegebenenfalls sauerstoff- oder stickstoffhaltigen Heterocyclus, der mit einer oder mehreren C₁₋₄-Alkylgruppen substituiert sein kann; oder eine 4'-Aminophenylgruppe,
R₃ und R'₃, die identisch oder voneinander verschieden sind, ein Wasserstoffatom, ein Halogenatom, das unter Chlor, Brom, Iod und Fluor ausgewählt ist, Cyano, C₁₋₄-Alkyl, C₁₋₄-Alkoxy oder Acetyloxy,
X- ein Anion, das vorzugsweise unter Chlorid, Methylsulfat und Acetat ausgewählt ist,
A eine Gruppe, die unter den folgenden Strukturen A1 bis A18 ausgewählt ist: 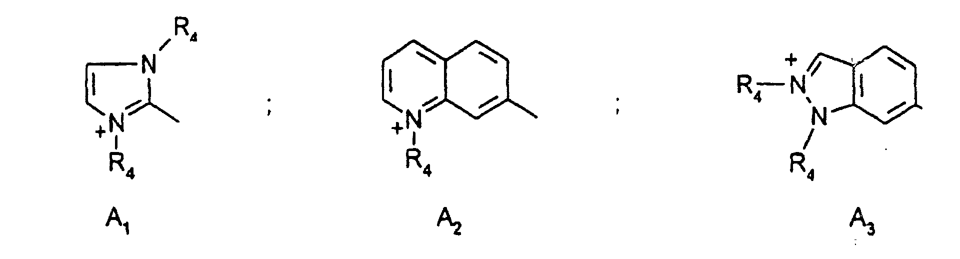 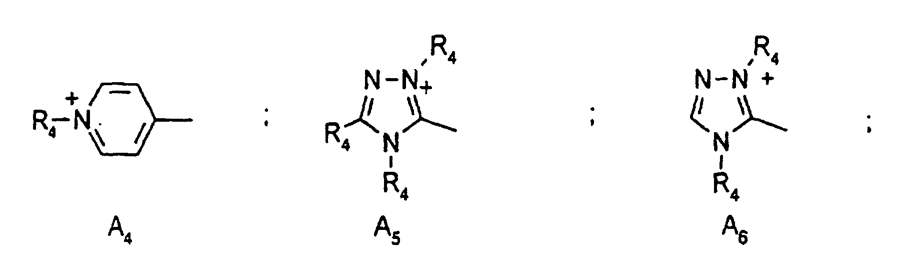 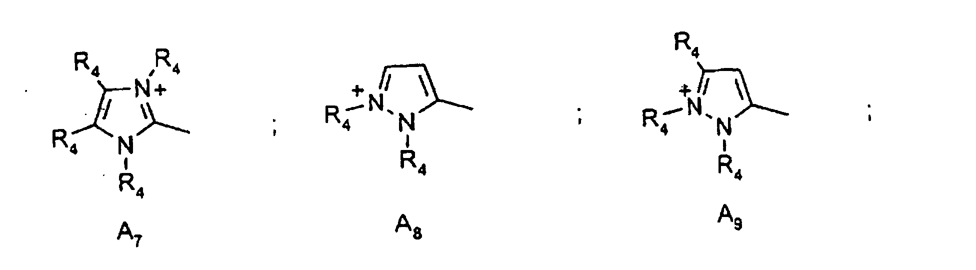 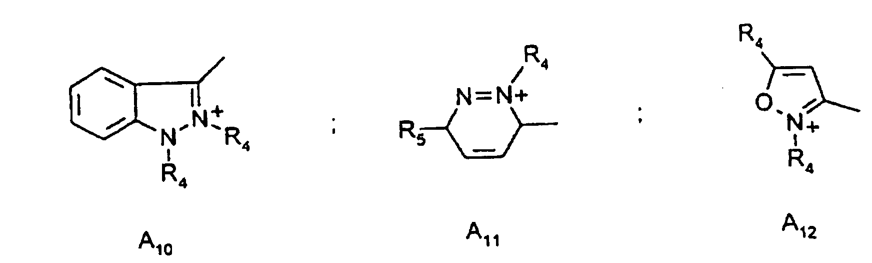 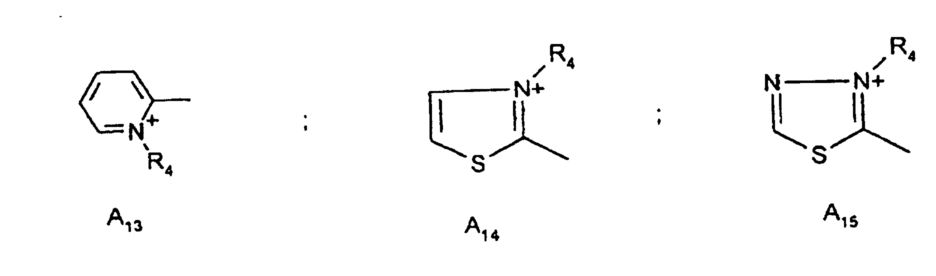 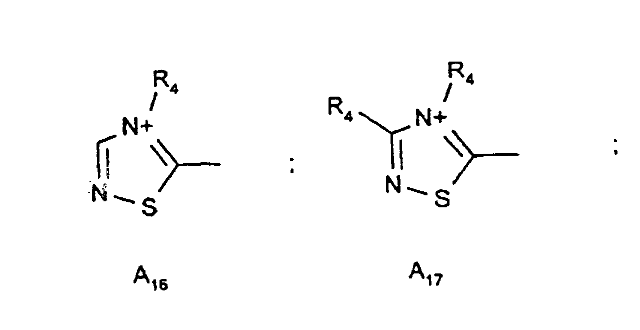 und 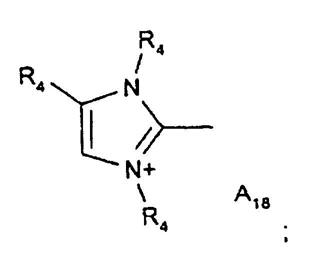 worin die Gruppe R₄ eine C₁₋₄-Alkylgruppe bedeutet, die mit einer Hydroxygruppe substituiert sein kann, und R₅ eine C₁₋₄-Alkoxygruppe ist, mit der Maßgabe, dass die Gruppen Ri und R₂ nicht gleichzeitig ein Wasserstoffatom bedeuten, wenn D die Gruppe -CH ist, die Gruppe A A₄ oder A₁₃ bedeutet und R₃ von Alkoxy verschieden ist;
(b) Verbindungen der folgenden Formel (II): 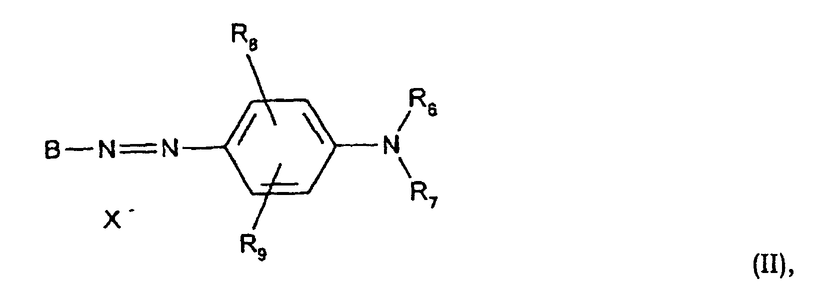 worin bedeuten:
R₆ ein Wasserstoffatom oder eine C₁₋₄-Alkylgruppe,
R₇ ein Wasserstoffatom, eine Alkylgruppe, die mit der Gruppe -CN oder einer Aminogruppe substituiert sein kann, oder 4'-Aminophenyl oder R₇ bildet mit R₆ einen gegebenenfalls sauerstoffhaltigen und/oder stickstoffhaltigen Heterocyclus, der mit einer C₁₋₄-Alkylgruppe substituiert sein kann,
R₈ und R₉, die gleich oder verschieden sind, ein Wasserstoffatom, ein Halogenatom, wie Brom, Chlor, Iod oder Fluor, eine C₁₋₄-Alkylgruppe, eine C₁₋₄-Alkoxygruppe oder die Gruppe -CN,
X- ein Anion, das vorzugsweise unter Chlorid, Methylsulfat und Acetat ausgewählt ist,
B eine Gruppe, die unter den folgenden Strukturen B 1 bis B6 ausgewählt ist: 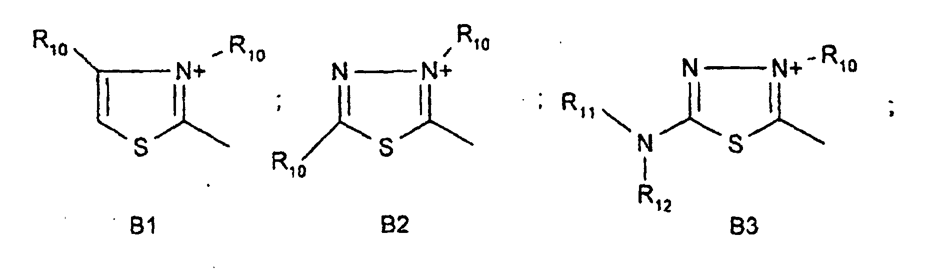 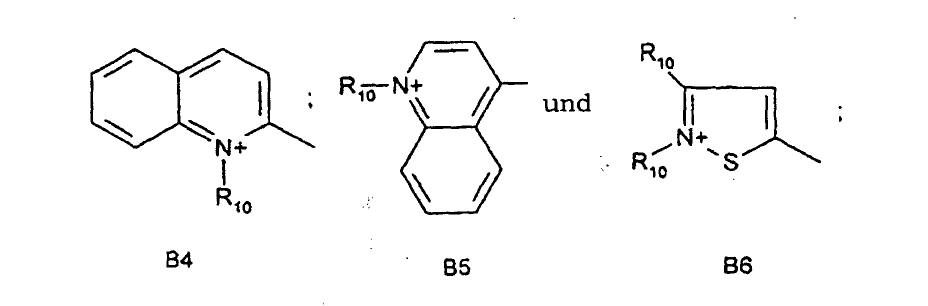
worin die Gruppe R₁₀ eine C₁₋₄-Alkylgruppe bedeutet und die Gruppen R₁₁ und R₁₂, die gleich oder verschieden sind, Wasserstoff oder C₁₋₄-Alkyl bedeuten;
(c) Verbindungen der folgenden Formel (III) und (III'): 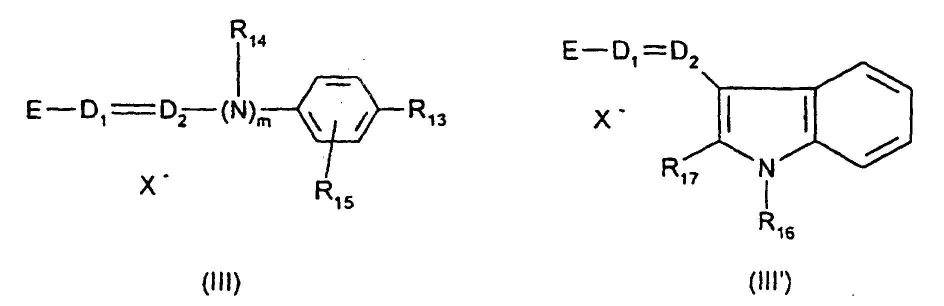 wobei in den Formeln (III) und (III') bedeuten:
R₁₃ ein Wasserstoffatom, eine C₁₋₄-Alkoxygruppe, ein Halogenatom, wie Brom, Chlor, Iod oder Fluor, oder eine Aminogruppe, R₁₄ ein Wasserstoffatom oder eine C₁₋₄-Alkylgruppe oder R₁₄ bildet mit einem Kohlenstoffatom des Benzolrings einen Heterocyclus, der gegebenenfalls sauerstoffhaltig und/oder mit einer oder mehreren C₁₋₄-Alkylgruppen substituiert ist;
R₁₅ ein Wasserstoffatom oder ein Halogenatom, wie Brom, Chlor, Iod oder Fluor,
R₁₆ und R₁₇, die identisch oder voneinander verschieden sind, Wasserstoff oder C₁₋₄-Alkyl,
D₁ und D₂, die gleich oder verschieden sind, ein Stickstoffatom oder die Gruppe -CH,
m = 0 oder 1,
mit der Maßgabe, dass die Gruppen D₁ und D₂ gleichzeitig -CH bedeuten und m = 0, wenn R₁₃ eine unsubstituierte Aminogruppe ist,
X- ein Anion, das vorzugsweise unter Chlorid, Methylsulfat und Acetat ausgewählt ist,
E eine Gruppe, die unter den folgenden Strukturen E 1 bis E8 ausgewählt ist: 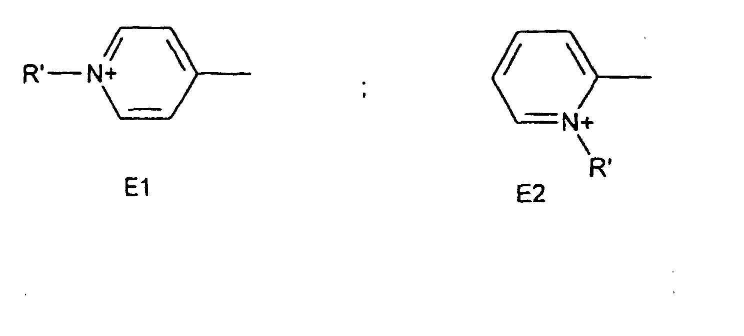 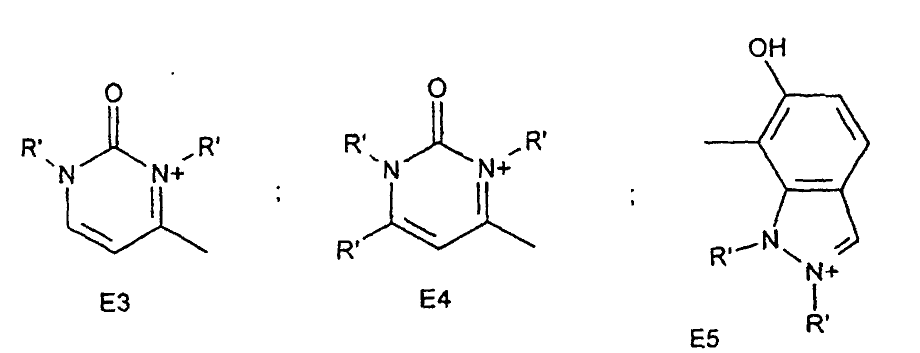 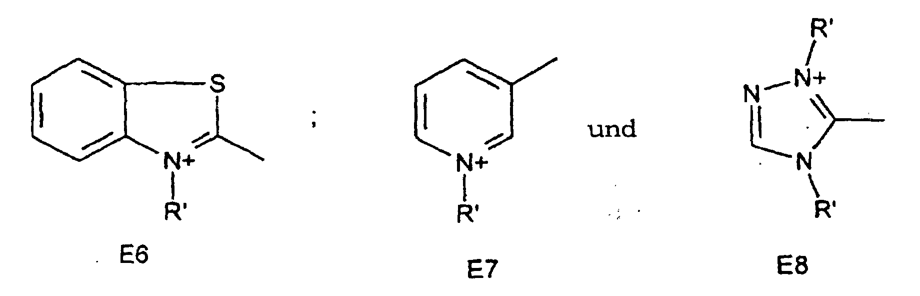
wobei R' eine C₁₋₄-Alkylgruppe bedeutet;
wenn m = 0 und D₁ ein Stickstoffatom bedeutet, kann die Gruppe E auch eine Gruppe der folgenden Struktur E9 sein: 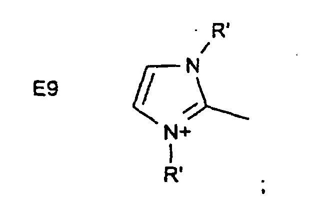 wobei R' eine C₁₋₄-Alkylgruppe bedeutet;
wobei die Zusammensetzung **dadurch gekennzeichnet ist, dass** sie ferner enthält:
(ii) mindestens ein verdickendes Polymer, das mindestens eine Zuckereinheit aufweist und unter den folgenden Polymeren ausgewählt ist:
(ii)₁ nichtionischen Guargummen;
(ii)₂ Gummen von Biopolysacchariden mikrobieller Herkunft, beispielsweise Scleroglucanen und Xanthangummi;
(ii)₃ Gummen aus pflanzlichen Exsudaten, wie Gummi arabicum, Ghatti Gummi, Karaya-Gummi, Tragant, Carrageenan, Agar und Johannisbrotkernmehl;
(ii)₄ Pektinen;
(ü)s Alginaten;
(ii)₆ Stärkeverbindungen; und
(ii)₇ Hydroxyalkylcellulosen und Carboxyalkylcellulosen.

2. Zusammensetzung nach Anspruch 1, **dadurch gekennzeichnet, dass** die kationischen Direktfarbstoffe der Formel (I) unter den Verbindungen der folgenden Strukturen (I1) bis (I54) ausgewählt sind:

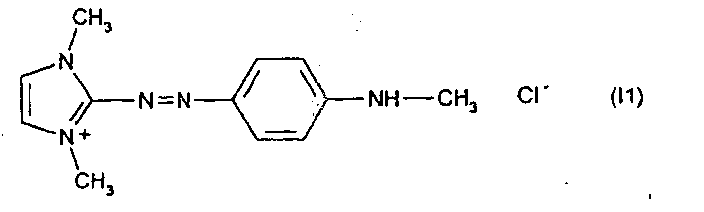

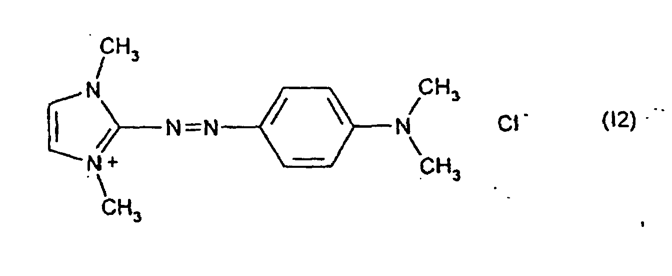

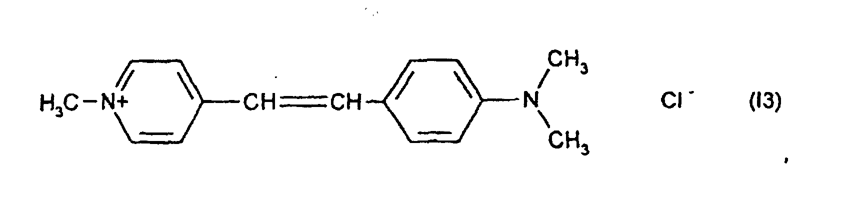

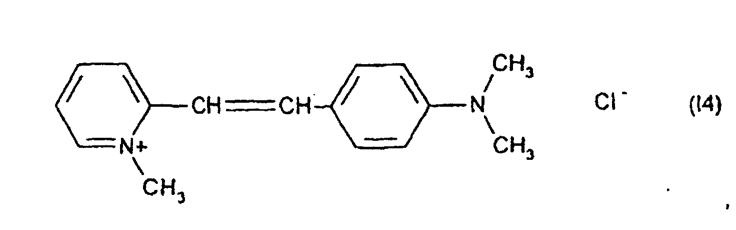

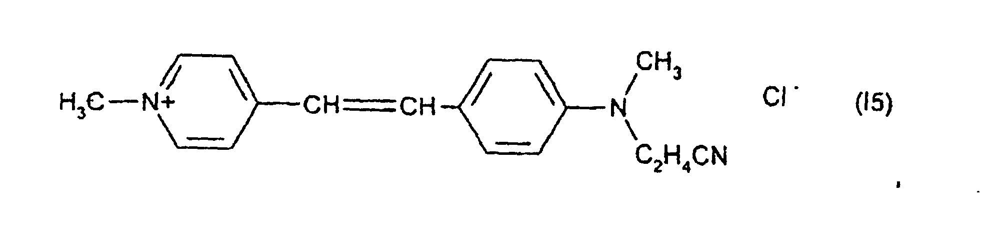

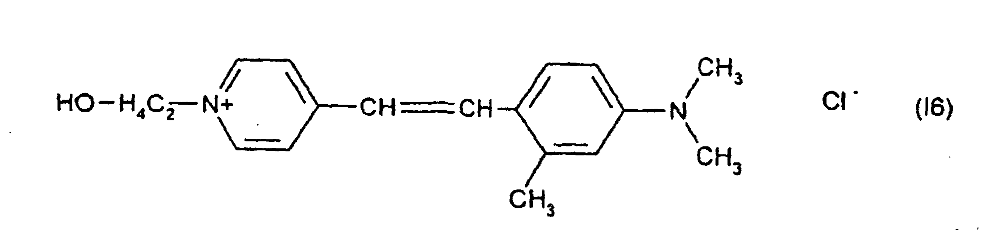

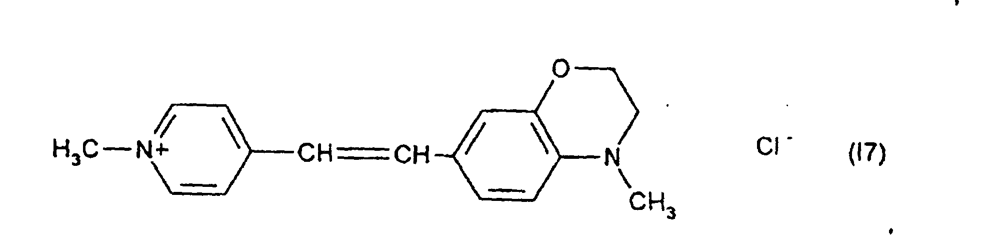

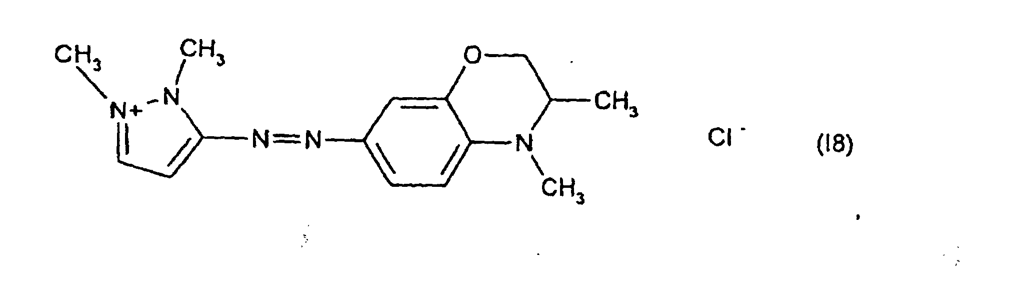

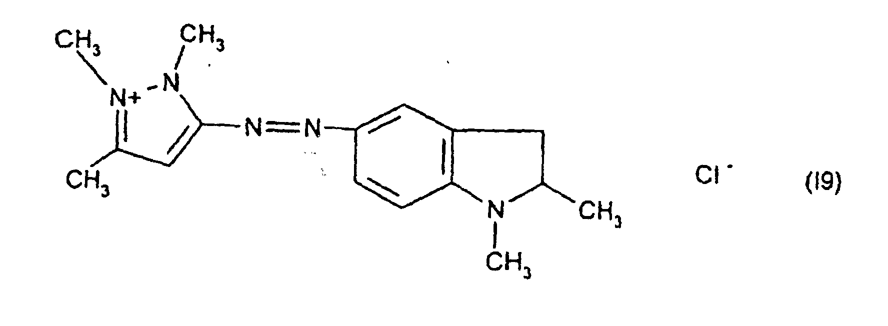

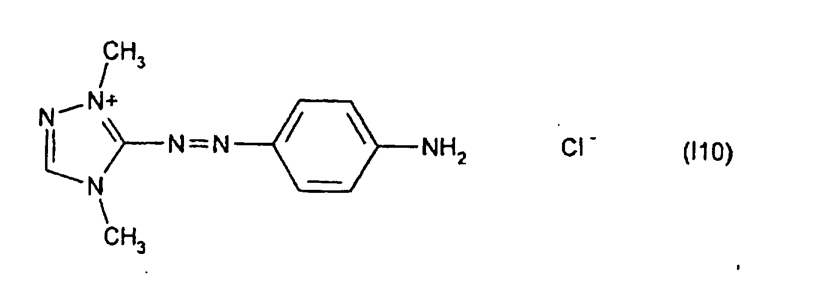

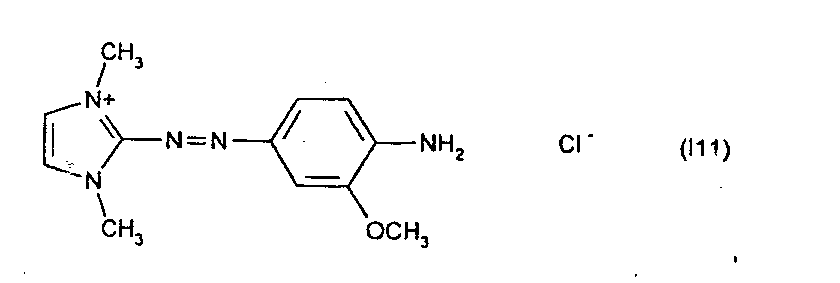

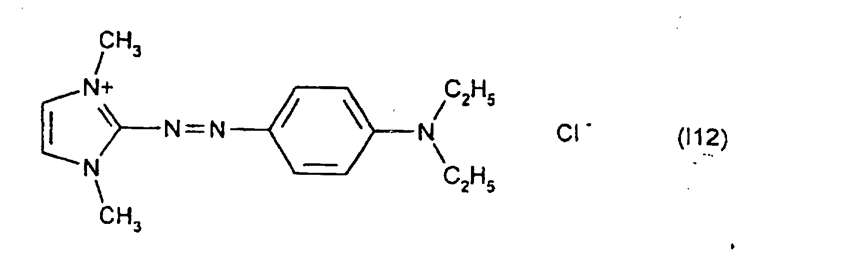

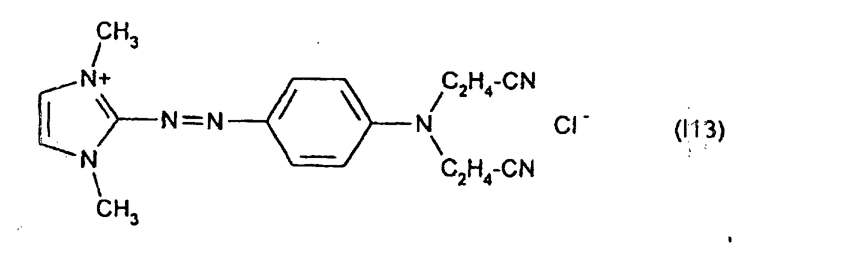

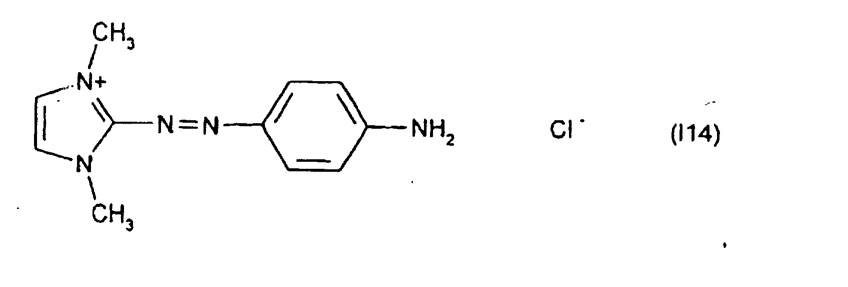

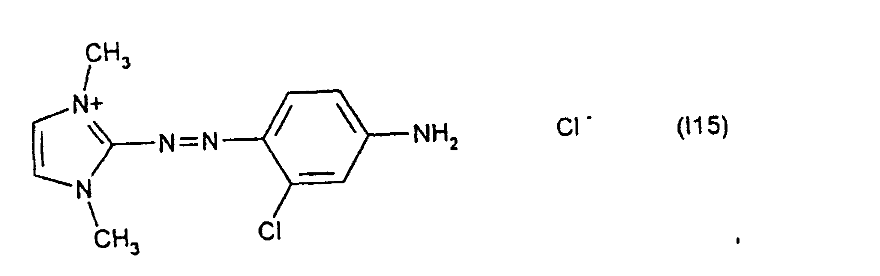

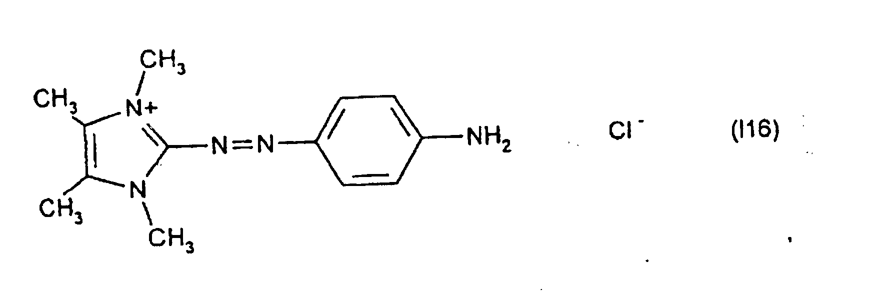

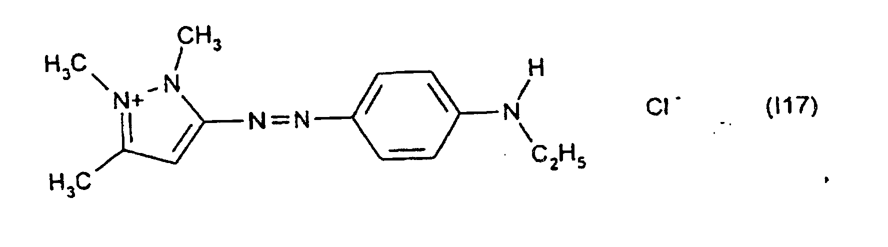

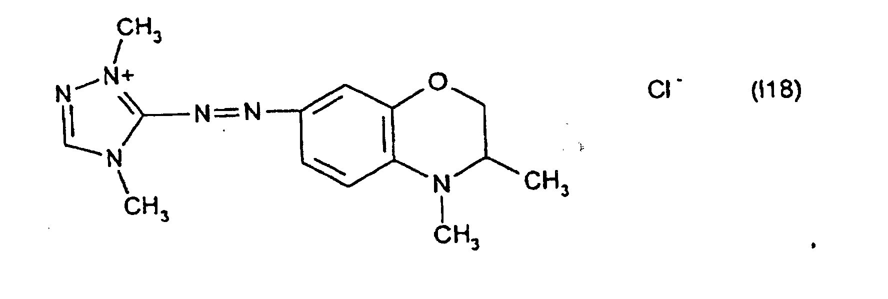

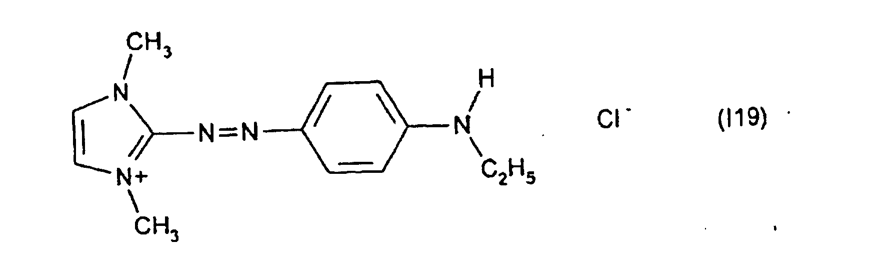

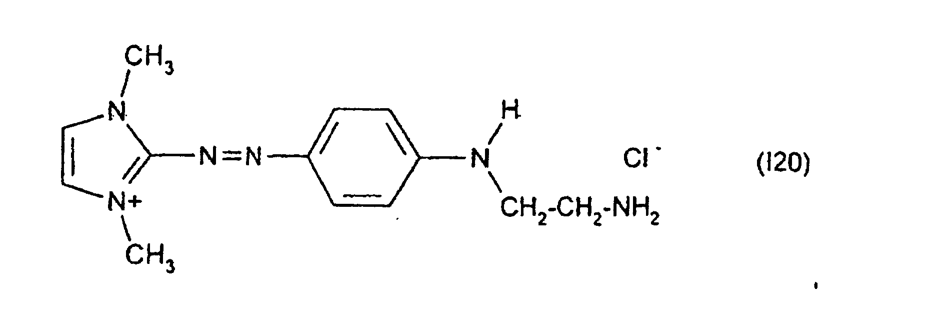

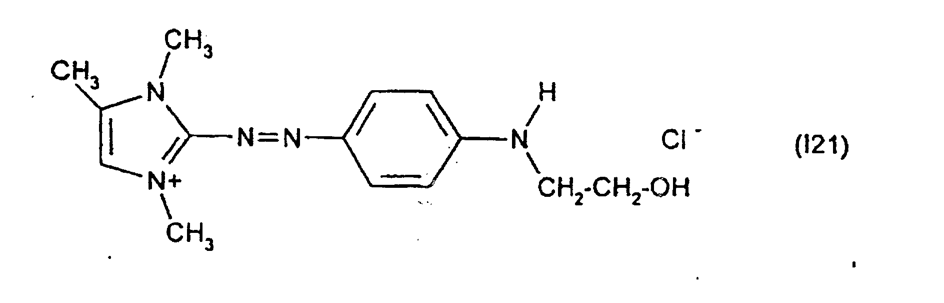

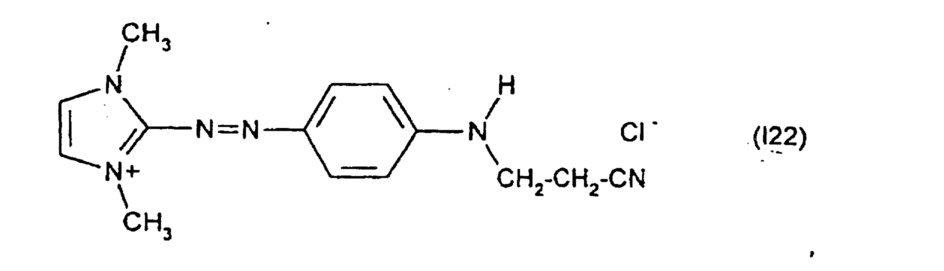

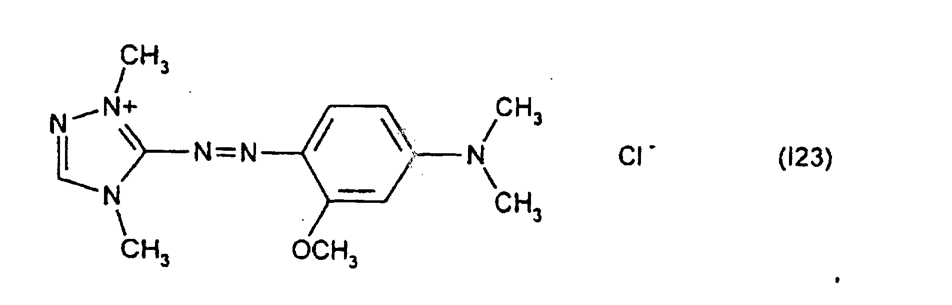

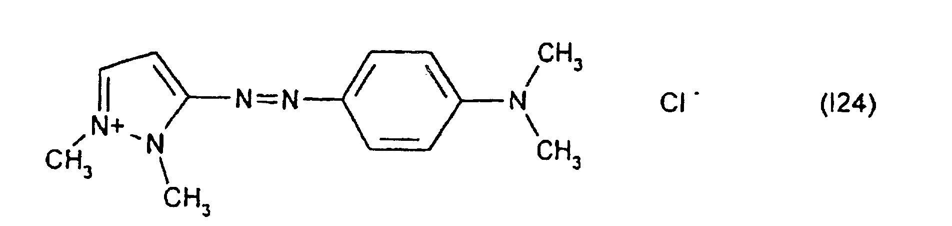

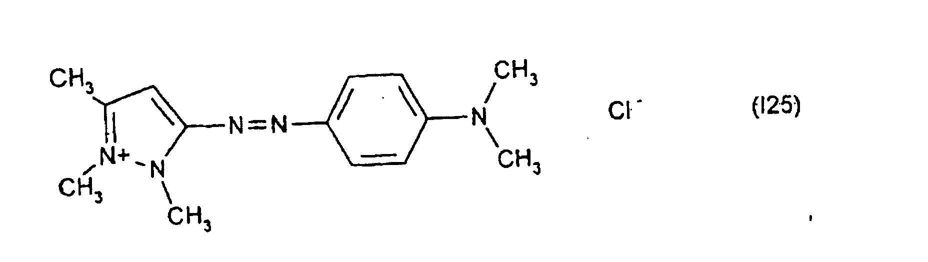

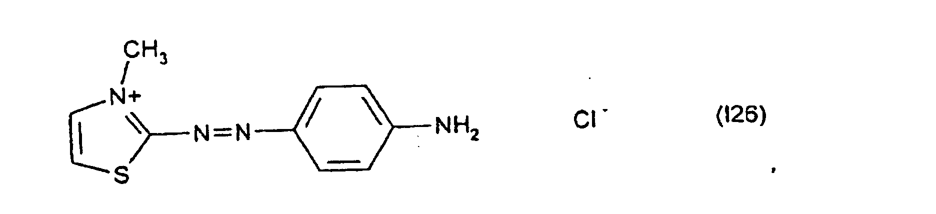

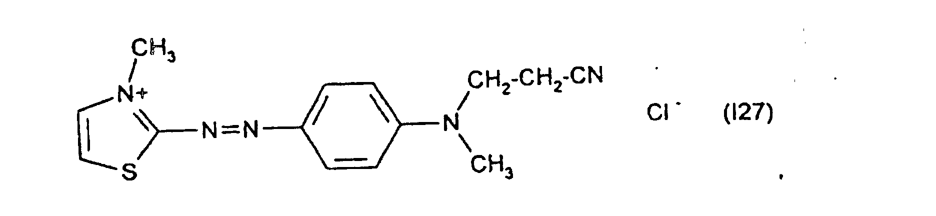

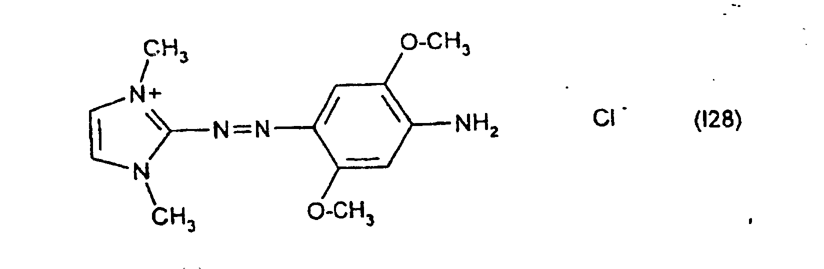

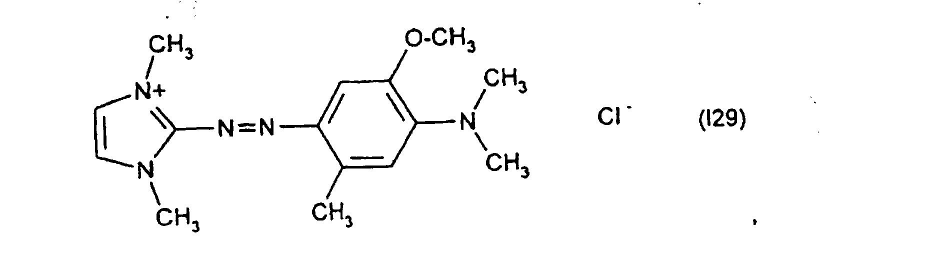

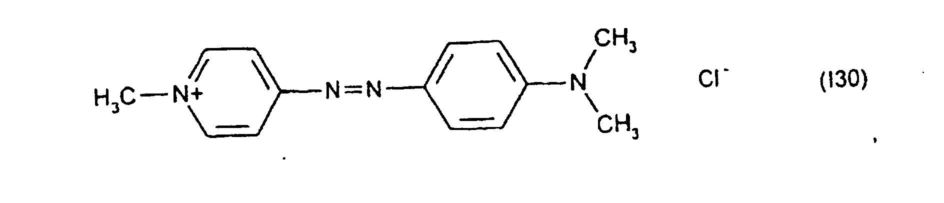

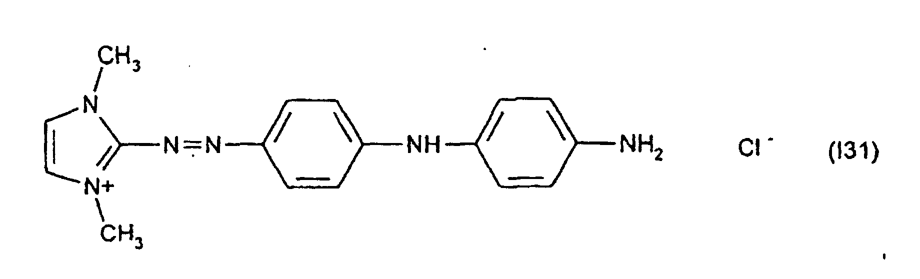

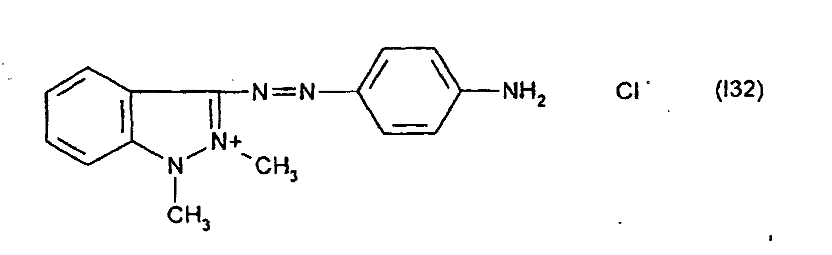

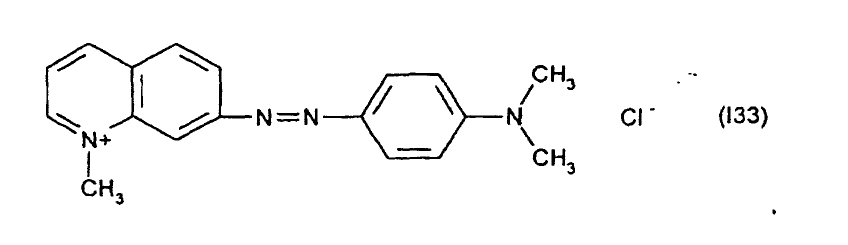

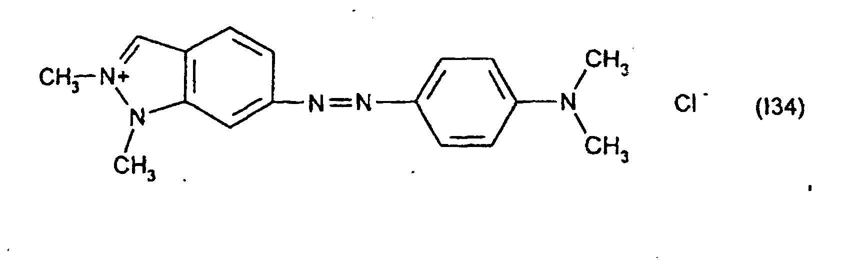

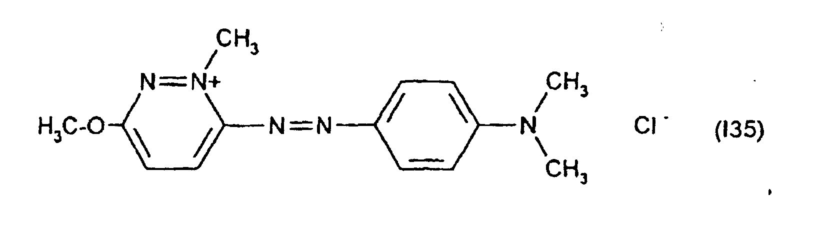

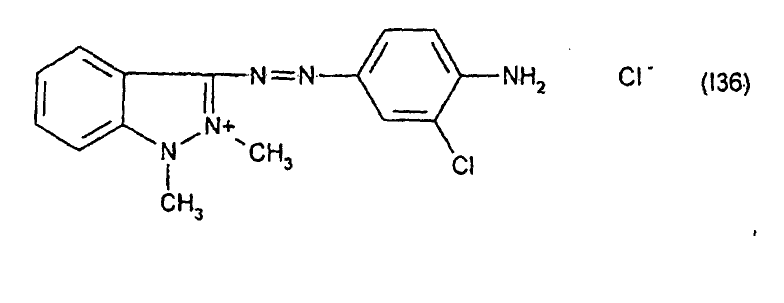

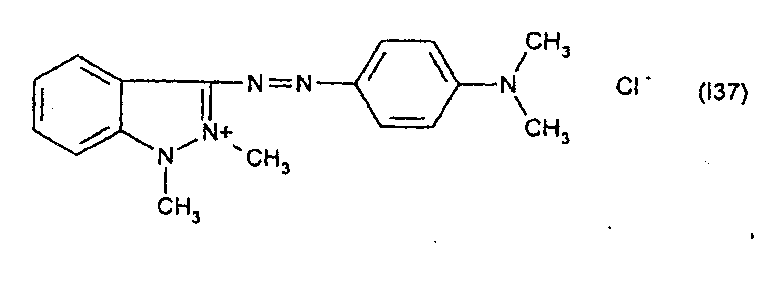

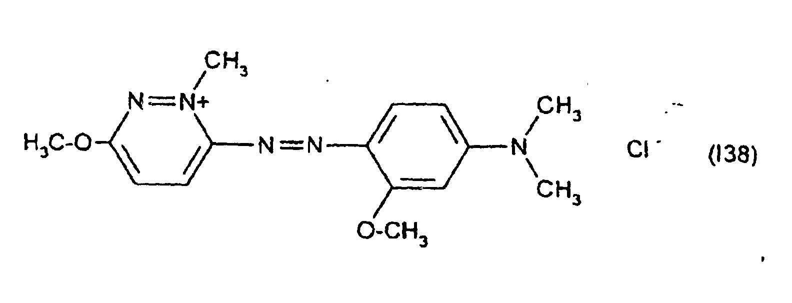

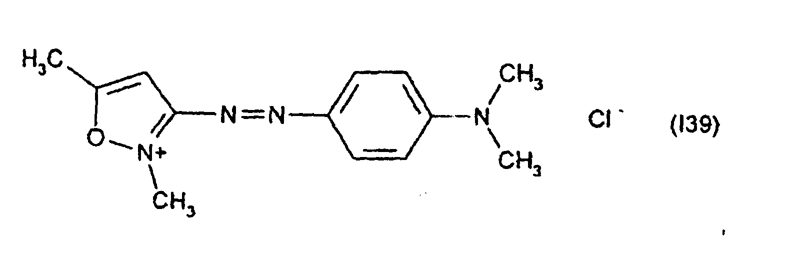

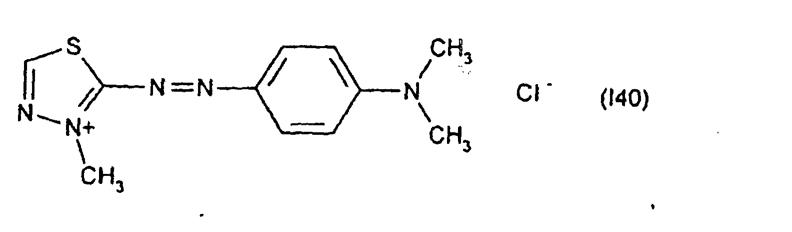

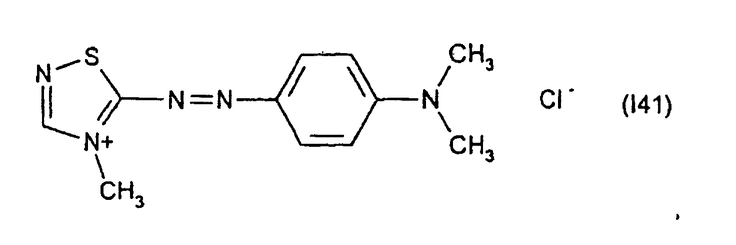

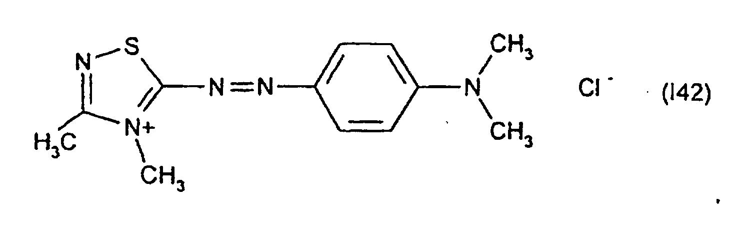

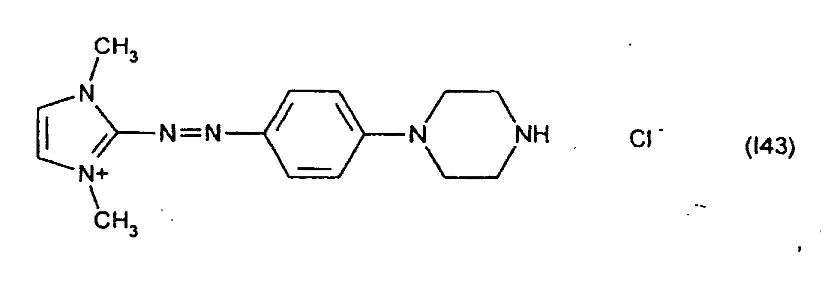

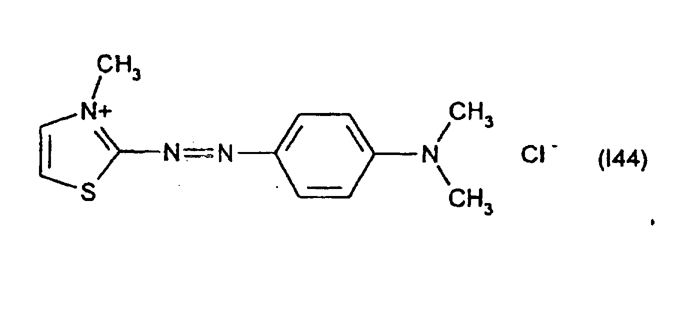

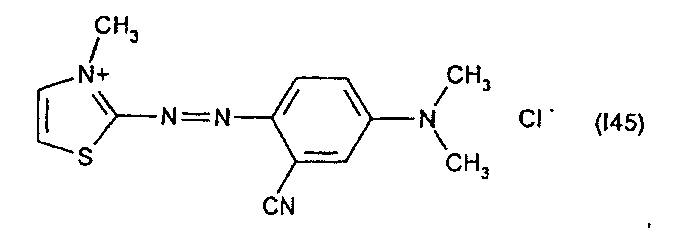

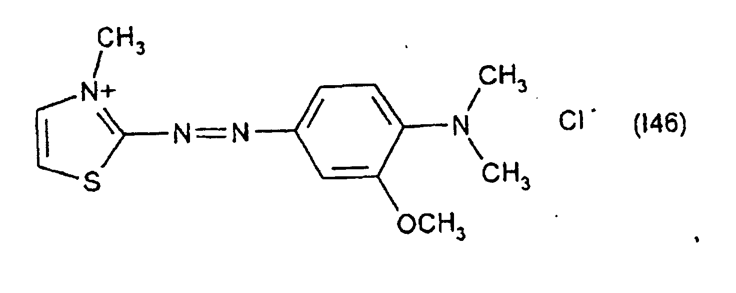

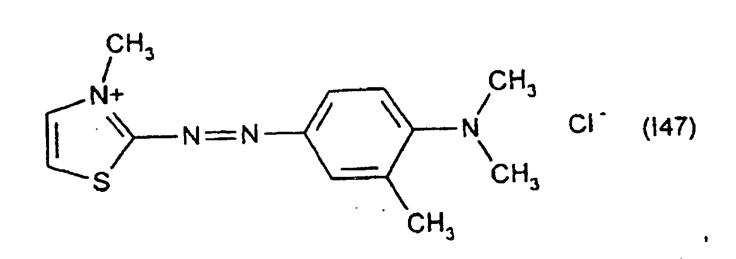

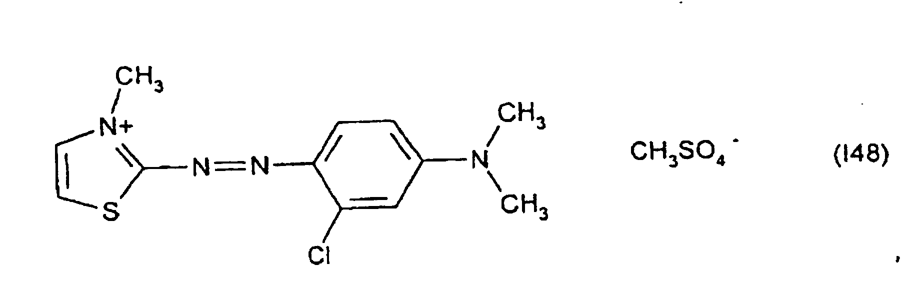

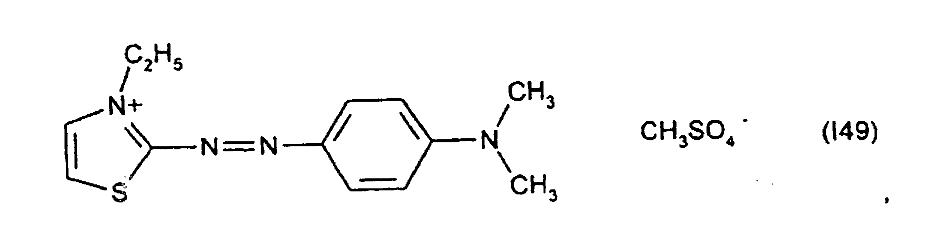

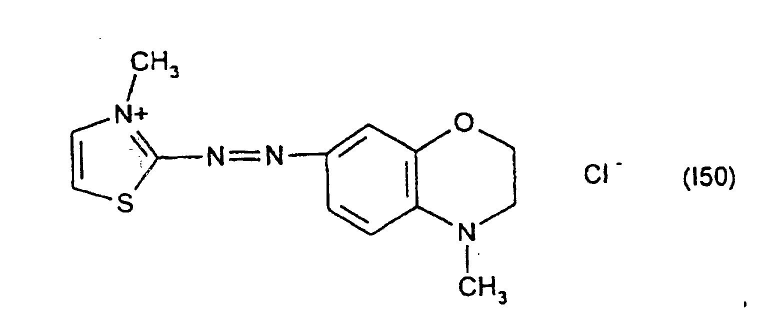

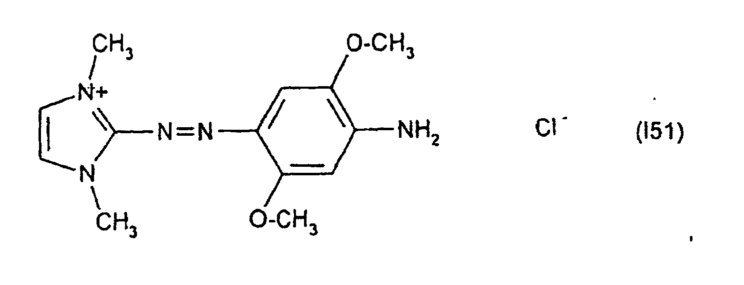

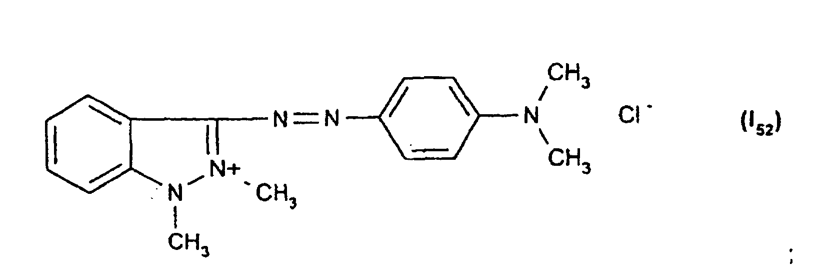

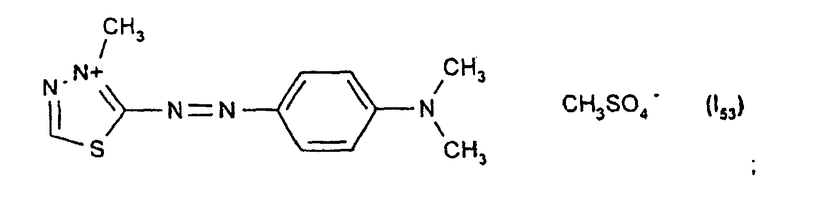

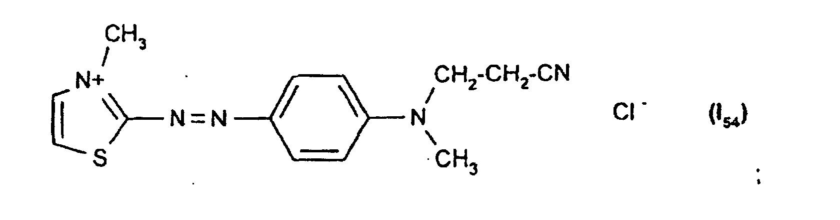

3. Zusammensetzung nach Anspruch 2, **dadurch gekennzeichnet, dass** die kationischen Direktfarbstoffe den Strukturen (I1), (I2), (I14) und (I31) entsprechen.

4. Zusammensetzung nach Anspruch 1 oder 2, **dadurch gekennzeichnet, dass** die kationischen Direktfarbstoffe der Formel (II) unter den Verbindungen der folgenden Strukturen (II1) bis (II9) ausgewählt sind: 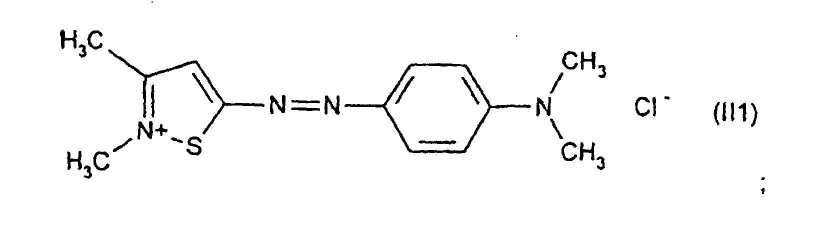 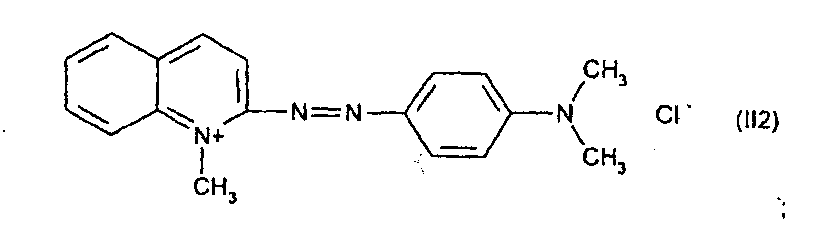 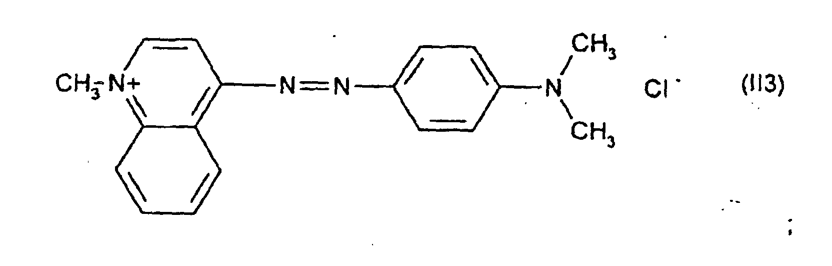 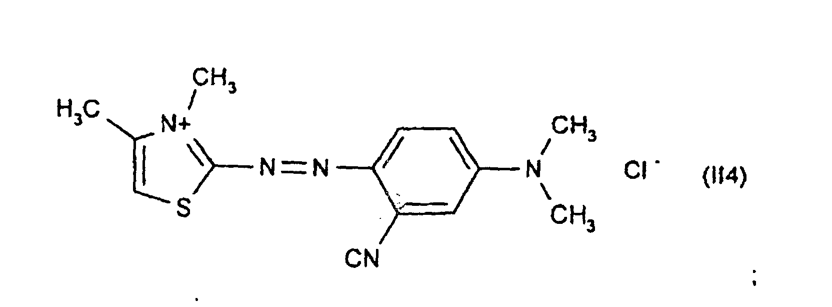 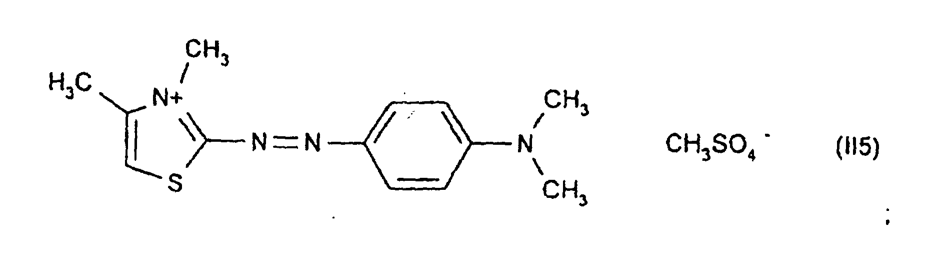 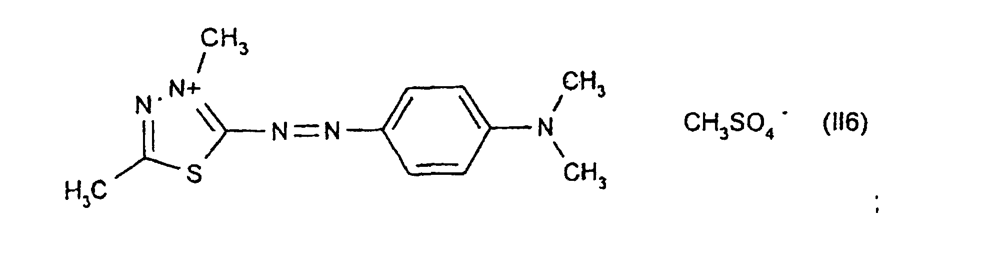 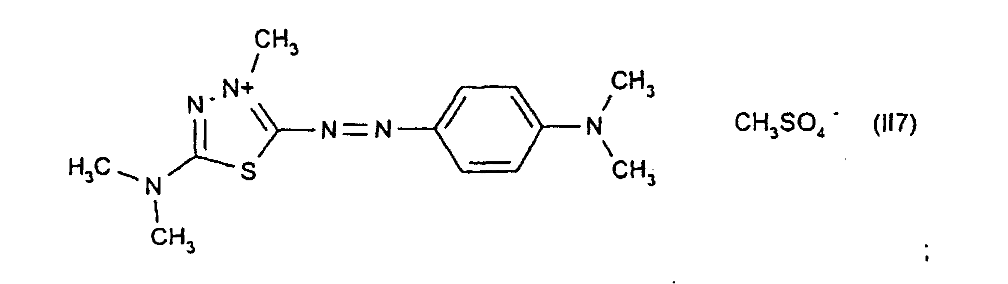 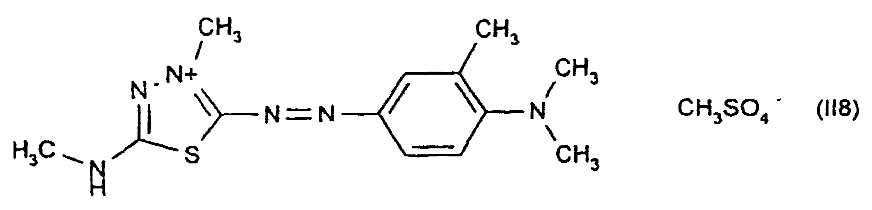 und

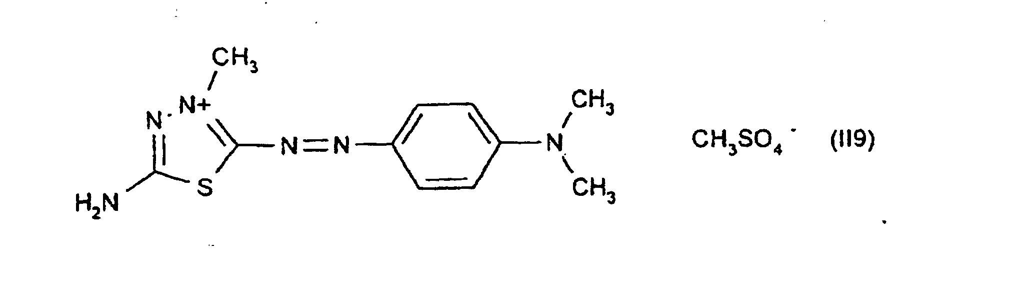

5. Zusammensetzung nach Anspruch 1 oder 2, **dadurch gekennzeichnet, dass** die kationischen Direktfarbstoffe der Formel (III) unter den Verbindungen der folgenden Strukturen (III1) bis (III18) ausgewählt sind: 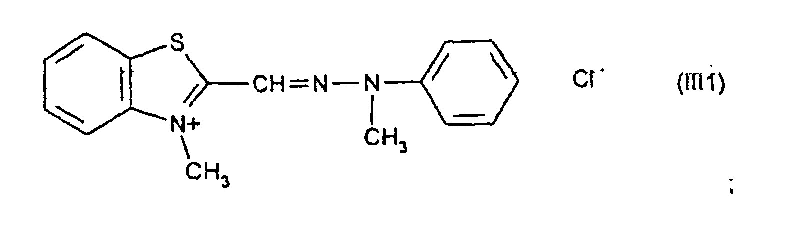 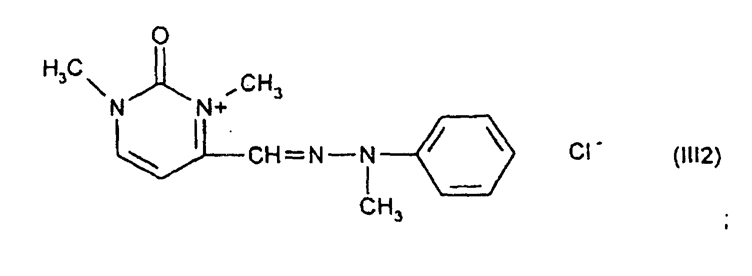 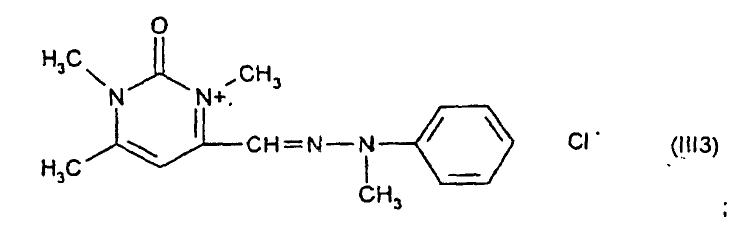 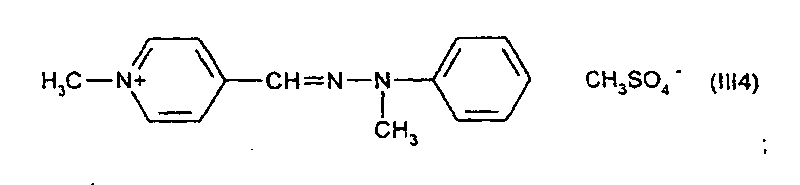 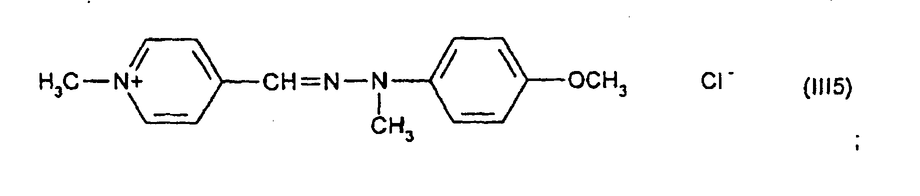 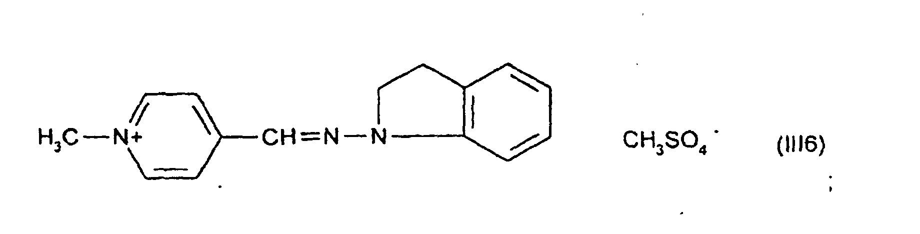 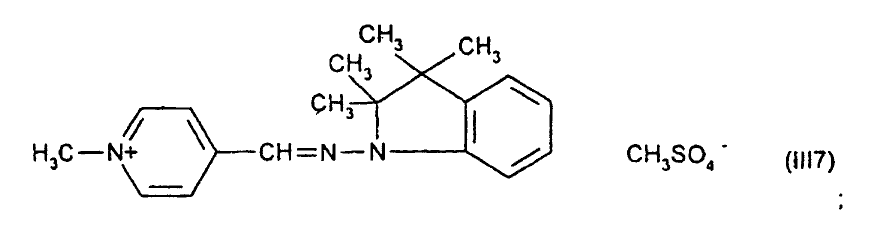 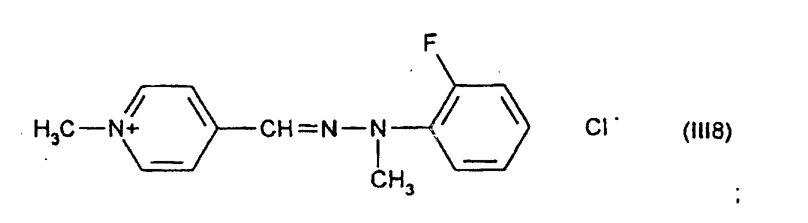 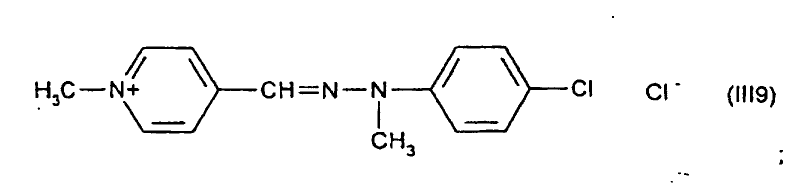 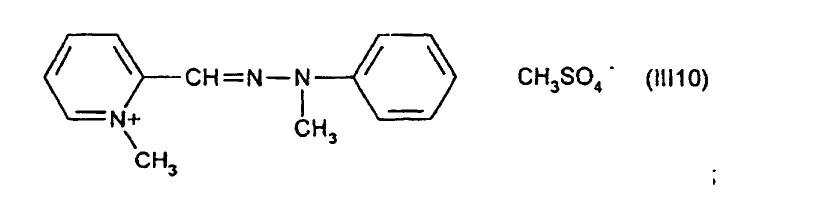 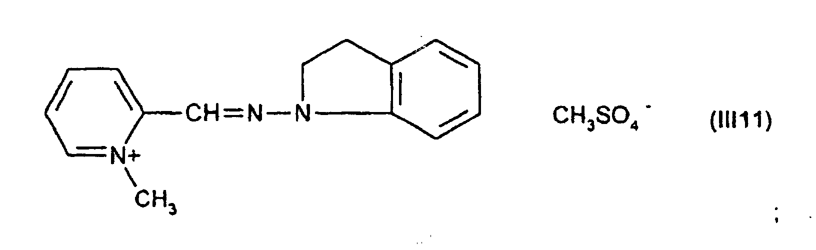 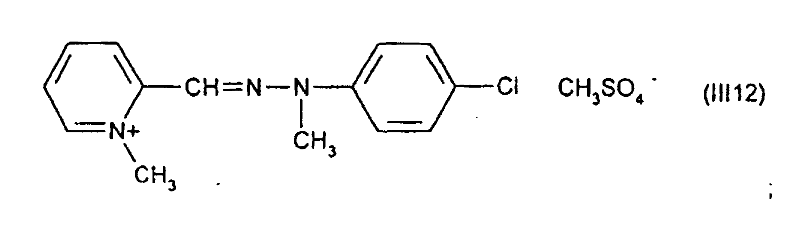 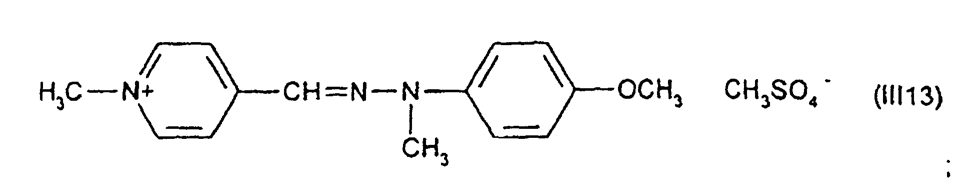 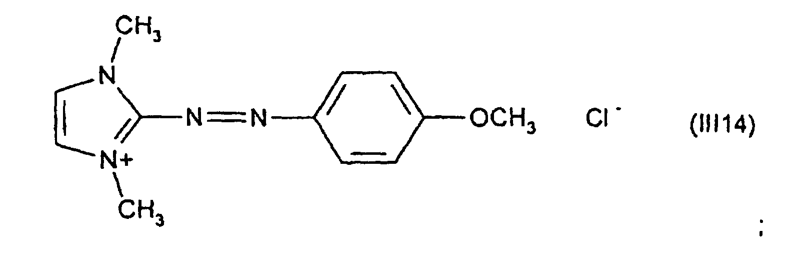 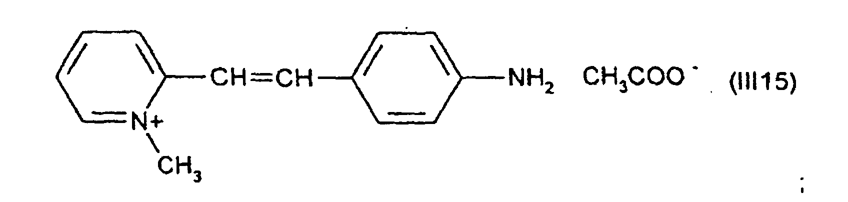 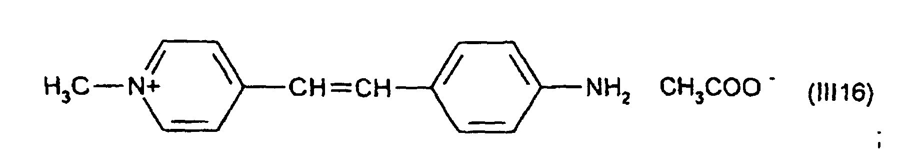 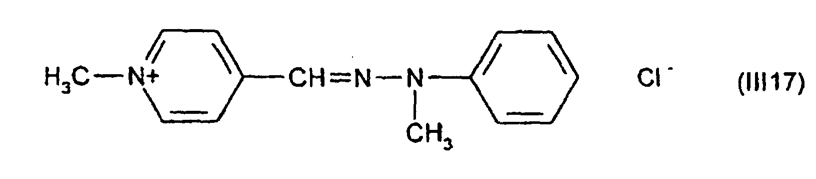 und

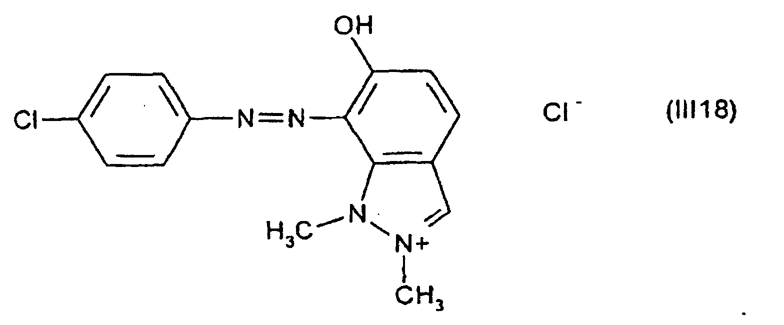

6. Zusammensetzung nach Anspruch 5, **dadurch gekennzeichnet, dass** die kationischen Direktfarbstoffe der Formel (III) den Strukturen (III4), (III5) und (III13) entsprechen.

7. Zusammensetzung nach Anspruch 1 oder 2, **dadurch gekennzeichnet, dass** die kationischen Direktfarbstoffe der Formel (III') unter den Verbindungen der folgenden Strukturen (III'1) bis (III'3) ausgewählt sind: 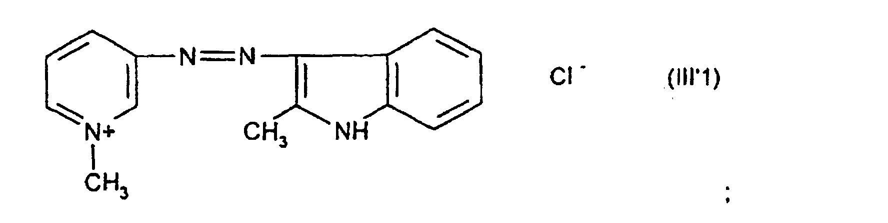 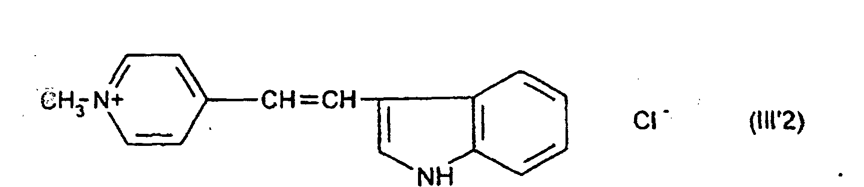 und

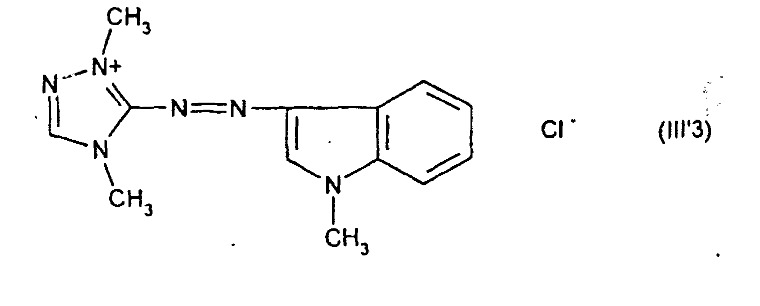

8. Zusammensetzung nach einem der vorhergehenden Ansprüche, **dadurch gekennzeichnet, dass** der oder die kationische(n) Direktfarbstoff(e) der Formeln (I), (II), (III) oder (III') 0,001 bis 10 Gew.-% des Gesamtgewichts der Zusammensetzung ausmachen.

9. Zusammensetzung nach Anspruch 8, **dadurch gekennzeichnet, dass** der oder die kationische(n) Direktfarbstoff(e) der Formeln (I), (II), (III) oder (III') 0,005 bis 5 Gew.-% des Gesamtgewichts der Zusammensetzung ausmachen.

10. Zusammensetzung nach einem der vorhergehenden Ansprüche, **dadurch gekennzeichnet, dass** das verdickende Polymer von Typ Hydroxyalkylcellulose eine Hydroxyethylcellulose ist.

11. Zusammensetzung nach einem der Ansprüche 1 bis 9, **dadurch gekennzeichnet, dass** das verdickende Polymer von Typ Hydroxyalkylcellulose eine Hydroxypropylcellulose ist.

12. Zusammensetzung nach einem der Ansprüche 1 bis 9, **dadurch gekennzeichnet, dass** das verdickende Polymer ein nichtionisches, mit C₁₋₆-Hydroxyalkylgruppen modifiziertes Guargummi ist.

13. Zusammensetzung nach Anspruch 12, **dadurch gekennzeichnet, dass** das nichtionische Guargummi einen Hydroxyalkylierungsgrad von 0,4 bis 1,2 aufweist.

14. Zusammensetzung nach einem der Ansprüche 1 bis 9, **dadurch gekennzeichnet, dass** das verdickende Polymer von Typ Carboxyalkylcellulose eine Carboxyethylcellulose ist.

15. Zusammensetzung nach einem der vorhergehenden Ansprüche, **dadurch gekennzeichnet, dass** das verdickende Polymer (ü) 0,01 bis 10 Gew.-% des Gesamtgewichts der Farbmittelzusammensetzung ausmacht.

16. Zusammensetzung nach Anspruch 15, **dadurch gekennzeichnet, dass** das verdickende Polymer (ii) 0,1 bis 5 Gew.-% des Gesamtgewichts der Farbmittelzusammensetzung ausmacht.

17. Zusammensetzung nach einem der vorhergehenden Ansprüche, **dadurch gekennzeichnet, dass** das zum Färben geeignete Medium (oder der Träger) aus Wasser oder einem Gemisch von Wasser und mindestens einem organischen Lösungsmittel besteht.

18. Zusammensetzung nach einem der vorhergehenden Ansprüche, **dadurch gekennzeichnet, dass** sie einen pH-Wert im Bereich von 2 bis 11 und vorzugsweise 5 bis 10 aufweist.

19. Zusammensetzung nach einem der vorhergehenden Ansprüche, **dadurch gekennzeichnet, dass** sie zum oxidativen Färben vorgesehen ist und eine oder mehrere Oxidationsbasen enthält, die unter den p-Phenylendiaminen, Bisphenylalkylendiaminen, p-Aminophenolen, o-Aminophenolen und heterocyclischen Basen ausgewählt sind.

20. Zusammensetzung nach Anspruch 19, **dadurch gekennzeichnet, dass** die Oxidationsbase(n) 0,0005 bis 12 Gew.-% des Gesamtgewichts der Farbmittelzusammensetzung ausmachen.

21. Zusammensetzung nach Anspruch 20, **dadurch gekennzeichnet, dass** die Oxidationsbase(n) 0,005 bis 6 Gew.-% des Gesamtgewichts der Farbmittelzusammensetzung ausmachen.

22. Zusammensetzung nach einem der Ansprüche 19 bis 21, **dadurch gekennzeichnet, dass** sie einen oder mehrere Kuppler enthält, die unter den m-Phenylendiaminen, m-Aminophenolen, m-Dihydroxybenzolen und heterocyclischen Kupplern ausgewählt sind.

23. Zusammensetzung nach Anspruch 22, **dadurch gekennzeichnet, dass** der oder die Kuppler 0,0001 bis 10 Gew.-% des Gesamtgewichts der Farbmittelzusammensetzung ausmachen.

24. Zusammensetzung nach Anspruch 23, **dadurch gekennzeichnet, dass** der oder die Kuppler 0,005 bis 5 Gew.-% des Gesamtgewichts der Farbmittelzusammensetzung ausmachen.

25. Zusammensetzung nach einem der vorhergehenden Ansprüche, **dadurch gekennzeichnet, dass** sie zum oxidativen Färben oder für die aufhellende Direktfärbung vorgesehen ist und mindestens ein Oxidationsmittel enthält.

26. Verfahren zum Färben von Keratinfasern und insbesondere menschlichen Keratinfasern, beispielsweise zum Färben der Haare, **dadurch gekennzeichnet, dass** auf die Fasern mindestens eine Farbmittelzusammensetzung nach einem der Ansprüche 1 bis 25 während einer Zeitspanne, die zur Bildung der gewünschten Färbung ausreichend ist, aufgebracht wird und dann gespült, gegebenenfalls mit Haarwaschmittel gewaschen, nochmals gespült und getrocknet wird.

27. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 25 während einer Zeitspanne, die zur Bildung der gewünschten Färbung ausreichend ist, aufgebracht wird, ohne dass nochmals gespült wird.

28. Verfahren zum Färben von Keratinfasern und insbesondere menschlichen Keratinfasern, wie Haaren, **dadurch gekennzeichnet, dass** es einen vorbereitenden Schritt umfasst, der darin besteht, einerseits eine Zusammensetzung (A1), die in einem zum Färben geeigneten Medium mindestens einen in den vorhergehenden Ansprüchen definierten Direktfarbstoff (i) und mindestens eine Oxidationsbase enthält, und andererseits eine Zusammensetzung (B 1) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A1) oder die Zusammensetzung (B1) das in den vorhergehenden Ansprüchen definierte verdickende Polymer (ii) enthält.

29. Verfahren zum Färben von Keratinfasern und insbesondere menschlichen Keratinfasern, wie Haaren, **dadurch gekennzeichnet, dass** es einen vorbereitenden Schritt umfasst, der darin besteht, einerseits eine Zusammensetzung (A2), die in einem zum Färben geeigneten Medium mindestens einen in den vorhergehenden Ansprüchen definierten kationischen Direktfarbstoff (i) enthält, und andererseits eine Zusammensetzung (B2) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A2) oder die Zusammensetzung (B2) das in den vorhergehenden Ansprüchen definierte verdickende Polymer (ii) enthält.

30. Vorrichtung mit mehreren Abteilungen oder "Kit" zum Färben mit mehreren Abteilungen, **dadurch gekennzeichnet, dass** eine erste Abteilung die Zusammensetzung (A1) oder (A2) nach Anspruch 28 oder 29 und eine andere Abteilung die Zusammensetzung (B1) oder (B2) nach Anspruch 28 oder 29 enthält.
